# EUROPÄISCHE PATENTSCHRIFT

(11) **EP 1 147 162 B1**
(45) Veröffentlichungstag und Bekanntmachung des Hinweises auf die Patenterteilung: **09.06.2004**
(21) Anmeldenummer: 99966957.5
(22) Anmeldetag: 13.12.1999
(51) Int. Cl.: C09K 19/02, C09K 19/34, C07D 213/63, C07D 239/26, C07D 239/34, C07D 239/74, C07D 285/12, C07D 333/38, C07D 409/12

(54) **FERROELEKTRISCHE AKTIVMATRIX-DISPLAYS MIT WEITEM ARBEITSTEMPERATURBEREICH**
FERROELECTRIC ACTIVE MATRIX DISPLAYS WITH WIDE OPERATING TEMPERATURE RANGE
AFFICHEUR FERROELECTRIQUE A MATRICE ACTIVE, POSSEDANT UNE PLAGE DE TEMPERATURE DE FONCTIONNEMENT LARGE

(30) Priorität: 11.12.1998 DE 19857352
(43) Veröffentlichungstag der Anmeldung: 24.10.2001
(73) Patentinhaber: Clariant GmbH, 65929 Frankfurt am Main (DE)
(72) Erfinder: NONAKA, Toshiaki, Kakegawa-shi, Shizuoka Pref. 436-0027 (JP); DÜBAL, Hans-Rolf, D-65343 Eltville (DE); WINGEN, Rainer, D-65795 Hattersheim (DE)
(74) Vertreter: Isenbruck, Günter, Dr.
(86) Internationale Anmeldenummer: PCT/EP1999/009863
(87) Internationale Veröffentlichungsnummer: WO 2000/036054

(56) Entgegenhaltungen:
- EP-A- 0 308 794
- EP-A- 0 459 406
- WO-A-92/11241
- WO-A-97/04039
- DE-A- 19 825 484
- CHEMICAL ABSTRACTS, vol. 124, no. 24, 10. Juni 1996 (1996-06-10) Columbus, Ohio, US; abstract no. 328597, IWAKI, TAKASHI ET AL: "Tetrahydroquinazoline compound and liquid-crystal composition, liquid-crystal element, and display device containing same" XP002137247 & JP 08 059629 A (CANON KK, JAPAN) 5. März 1996 (1996-03-05) in der Anmeldung erwähnt

## Beschreibung

Der Ersatz der Kathodenstrahlröhre (Bildröhre) durch einen flachen Bildschirm erfordert eine Displaytechnologie, die gleichzeitig eine hohe Auflösung, d.h. mehr als 1000 Zeilen, eine hohe Helligkeit (>200 Cd/m²), einen hohen Kontrast (>100:1), eine hohe Bildfrequenz (>60 Hz), eine ausreichende Farbdarstellung (>16 Mio), ein großes Bildformat (>40 cm), eine geringe Leistungsaufnahme und einen weiten Betrachtungswinkel ermöglicht, verbunden mit niedrigen Herstellkosten. Zur Zeit existiert keine Technologie, die alle diese Merkmale gleichzeitig in vollem Umfang erfüllt.

Viele Hersteller haben auf der Basis nematischer Flüssigkristalle Bildschirme entwickelt, die seit einigen Jahren im Bereich von Notebook PC, Personal Digital Assistants, Desktop Monitore usw. im Einsatz sind. Dabei werden die Technologien STN (Supertwisted Nematics), AM-TN (Active Matrix - Twisted Nematics), AM-IPS (Active Matrix - In Plane Switching), AM-MVA (Active Matrix - Multidomain Vertically Aligned) verwendet, die in der einschlägigen Literatur ausführlich beschrieben werden (siehe z.B. T. Tsukuda, TFT/LCD: Liquid Crystal Displays Addressed by Thin-Film Transistors, Gordon and Breach 1996, ISBN 2-919875-01-9 und darin zitierte Literatur; SID Symposium 1997, ISSN-0097-966X und darin zitierte Literatur). Darüber hinaus wird auf die Technologien PDP (Plasma Display Panel), PALC (Plasma Addressed Liquid Crystal), ELD (Electro Luminescent Display), FED (Field Emission Display) usw. hingewiesen, die ebenfalls im oben zitierten SID-Bericht erläutert sind.

Clark und Lagerwall (US-Patent 4,367,924) konnten zeigen, daß der Einsatz ferroelektrischer Flüssigkristalle (FLC) in sehr dünnen Zellen zu optoelektrischen Schalt- oder Anzeigeelementen führt, die im Vergleich zu den herkömmlichen TN ("twisted nematic")-Zellen um bis zu einem Faktor 1000 kürzere Schaltzeiten haben (siehe z. B. EP-A 0 032 362). Aufgrund dieser und anderer günstiger Eigenschaften, z. B. der bistabilen Schaltmöglichkeit und des nahezu blickwinkelunabhängigen Kontrasts, sind FLCs grundsätzlich für Anwendungsgebiete wie Computerdisplays und Fernsehgeräte geeignet, wie ein seit Mai 1995 in Japan von Canon vermarkteter Monitor zeigt.

Für die Verwendung von FLCs in elektrooptischen oder vollständig optischen Bauelementen benötigt man entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen ferroelektrische smektische Phasen induzieren. Die gewünschte Phase soll dabei über einen möglichst großen Temperaturbereich stabil sein, um einen breiten Arbeitsbereich des Displays sicherzustellen.

Die einzelnen Bildelemente (Pixel) eines LC-Displays sind üblicherweise in einer x,y Matrix angeordnet, die durch die Anordnung je einer Serie von Elektroden (Leiterbahnen) entlang der Reihen und der Spalten an der Unter- bzw. Oberseiteseite des Displays gebildet wird. Die Kreuzungspunkte der horizontalen (Reihen-) und vertikalen (Spalten-) Elektroden bilden adressierbare Pixel.

Diese Anordnung der Bildpunkte bezeichnet man üblicherweise als eine passive Matrix. Zur Adressierung wurden verschiedene Multiplex-Schemata entwickelt, wie sie beispielsweise in Displays 1993, Vol. 14, Nr. 2, S. 86-93 und Kontakte 1993 (2), S. 3-14 beschrieben sind. Die passive Matrixadressierung hat den Vorteil einer einfacheren Herstellung des Displays und damit verbundenen geringen Herstellkosten, jedoch den Nachteil, daß die passive Adressierung immer nur zeilenweise erfolgen kann, was dazu führt, daß die Adressierungszeit des gesamten Bildschirms bei N Zeilen das N-fache der Zeilenadressierungszeit beträgt. Bei üblichen Zeilenadressierungszeiten von ca. 50 Mikrosekunden bedeutet das eine Bildschirmadressierungszeit von ca. 60 Millisekunden bei z.B. HDTV Norm (High Definition TV, 1152 Zeilen), d.h. eine maximale Bildfrequenz von ca. 16 Hz, die für bewegte Bilder zu gering ist. Zudem ist die Darstellung von Graustufen schwierig. Mizutani et.al. haben auf der FLC-Konferenz in Brest, Frankreich (20.-24 Juli 1997, siehe Abstract Book 6^{th} International Conference on Ferroelectric Liquid Crystals, Brest / France) ein passives FLC-Display mit digitalen Graustufen vorgestellt, bei dem jeder der RGB-Bildpunkte (RGB= red, green, blue) in Unterpunkte unterteilt wurde, wodurch vermittels partiellem Schalten die Darstellung von Grauwerten in digitaler Form ermöglicht wird. Bei N Grauwerten unter Verwendung dreier Grundfarben (rot, grün, blau) ergeben sich 3^{N} Farben. Der Nachteil dieser Methode ist eine starke Erhöhung der Anzahl benötigter Bildschirmtreiber und damit der Kosten (im Falle des in Brest gezeigten Bildschirm wurden dreimal soviele Treiber benötigt, wie bei einem normalen FLC Display ohne digitale Graustufen).

Bei der sogenannten Aktivmatrix-Technologie (AMLCD) wird üblicherweise ein nicht-strukturiertes Substrat mit einem Aktivmatrix-Substrat kombiniert. An jedem Pixel des Aktivmatrixsubstrates ist ein elektrisch nichtlineares Element, beispielsweise ein Dünnschichttransistor, integriert. Bei dem nichtlinearen Element kann es sich auch um Dioden, Metall-Insulator-Metall u.ä. Elemente handeln, die vorteilhaft mit Dünnschichtverfahren hergestellt werden und in der einschlägigen Literatur beschrieben sind (s. z.B. T. Tsukuda, TFT/LCD: Liquid Crystal Displays Addressed by Thin-Film Transistors, Gordon and Breach 1996, ISBN 2-919875-01-9 und darin zitierte Literatur).

Aktivmatrix-LCD werden üblicherweise mit nematischen Flüssigkristallen im TN-(twisted nematics), ECB- (electrically controlled birefringence), VA- (vertically aligned) oder IPS- (in plane switching) Modus betrieben. In jedem Fall wird durch die aktive Matrix an jedem Bildpunkt ein elektrisches Feld individueller Stärke erzeugt, das eine Orientierungsänderung und damit eine Änderung der Doppelbrechung erzeugt, die wiederum im polarisierten Licht optisch sichtbar ist. Ein schwerwiegender Nachteil dieser Verfahren ist die mangelnde Videofähigkeit, d.h. die zu langen Schaltzeiten nematischer Flüssigkristalle.

Unter anderem aus diesem Grunde wurden Flüssigkristallanzeigen, die auf der Kombination aus ferroelektrischen Flüssigkristallmaterialien und aktiven MatrixElementen beruhen, z.B. in WO 97/12355 oder in Ferroelectrics 1996, 179, 141-152 oder bei W.J.A.M. Hartmann (IEEE Trans. Electron. Devices 1989, 36,(9;Pt. 1), 1895-9, sowie Dissertation Eindhoven, Niederlande 1990) vorgeschlagen.

Hartmann nutzte eine Kombination aus der sogenannten 'Quasi-bookshelf Geometrie' (QBG) von FLC und einer TFT (Thin-Film-Transistor) Aktivmatrix und erhielt gleichzeitig eine hohe Schaltgeschwindigkeit, Graustufen und hohe Transmission. Allerdings ist die QBG nicht über einen weiten Temperaturbereich stabil, da durch die Temperaturabhängigkeit der smektischen Schichtdicke die feldinduzierte Lagenstruktur aufbricht oder sich dreht. Darüber hinaus nutzt Hartmann ein FLC-Material mit mehr als 20 nC/cm², was bei Bildpunkten von realistischer Dimension von z.B. 0,01 mm² zu großen elektrischen Ladungen führt (bei Sättigung gilt Q = 2 A P, A= Bildpunktfläche, P= spontane Polarisation), die z.B. mit kostengünstig herstellbaren amorphen Silizium - TFT während der Öffnungszeit des TFT nicht auf den Bildpunkt gelangen können. Aus diesen Gründen wurde diese Technologie bisher nicht weiterverfolgt.

Während Hartmann die ladungskontrollierte Bistabilität zur Darstellung einer nahezu kontinuierlichen Grauskala ausnutzt, haben Nito et. al. eine monostabile FLC Geometrie vorgeschlagen (Journal of the SID, 1 / 2, 1993, Seiten 163-169), bei der das FLC Material mit Hilfe verhältnismäßig hoher Spannungen derart orientiert wird, daß nur eine stabile Lage entsteht, aus der dann bei Anlegen eines elektrischen Feldes über einen Dünnschichttransistor eine Reihe von Zwischenzuständen erzeugt werden, die bei angepaßter Zellgeometrie zwischen gekreuzten Polarisatoren einer Reihe von verschiedenen Helligkeitsgraden (Grauwerte) entsprechen.

Der Nachteil bei der Arbeit von Nito et.al. ist nun das Auftreten einer Streifentextur, die den Kontrast und die Helligkeit dieser Zelle begrenzt (siehe Abb. 8 des o.a. Zitates). Die nachteilige Streifentextur läßt sich durch eine Behandlung mit einem hohen elektrischen Feld (20-50 V) in der nematischen bzw. cholesterischen Phase (s. S. 168 des o.a. Zitates) zwar korrigieren; jedoch ist eine solche Feldbehandlung nicht für die Massenfertigung von Bildschirmen geeignet und führt in der Regel auch nicht zu temperaturstabilen Texturen. Darüber hinaus ergibt diese Methode lediglich ein Schalten in einem Winkelbereich von bis zu maximal dem einfachen Tiltwinkel, der bei dem von Nito et. al. verwendeten Material bei ca. 22 ° liegt (s.S. 165 Abb. 6) und damit nur eine Transmission von maximal 50 % der Transmission zweier paralleler Polarisatoren ergibt.

Aufgabe der Erfindung ist die Bereitstellung einer vorzugsweise chiral-smektischen Aktiv-Matrix-Flüssigkristallanzeige, enthaltend eine vorzugsweise chiral-smektische Flüssigkristallmischung, wobei die Flüssigkristallmischung eine sehr hohe Maximaltransmission sowie einen sehr hohen Kontrast sowie eine konstante Schwellspannung über einen weiten Temperaturbereich ermöglicht.

Insbesondere soll eine ferroelektrische Aktiv-Matrix-Flüssigkristallanzeige, enthaltend eine ferroelektrische Flüssigkristallmischung bereitgestellt werden, wobei die Flüssigkristallmischung eine monostabile Lage einnimmt, jedoch keinerlei Streifentextur bildet, temperaturstabil ist und eine sehr hohe Maximaltransmission sowie einen sehr hohen Kontrast sowie eine konstante Schwellspannung über einen weiten Temperaturbereich ermöglicht.

Die Aufgabe wird erfindungsgemäß gelöst durch nachstehendes chiral-smektisches Aktivmatrix-Display, enthaltend eine Flüssigkristallschicht mit einem über einen weiten Temperaturbereich nahezu konstanten Tiltwinkel, sowie einem nahezu konstanten Lagenkippwinkel (Layer Leaning Angle), wobei die Flüssigkristallschicht mindestens 1 Verbindung der nachstehenden Formel (I) enthält.

Ausdrücklich einbezogen ist die vorteilhafte Verwendung der erfindungsgemäßen Materialien und Mischungen für Aktivmatrix-Displays, antiferroelektrische Displays sowie twisted smektische Displays.

Das Aktivmatrix-Display ist ein monostabiles ferroelektrisches Aktivmatrix-Display, enthaltend eine Flüssigkristallschicht in Form einer Monodomäne mit einer eindeutig definierten Richtung der Schichtennormalen z der SmC-*Phase, wobei die Schichtennormale z und die Vorzugsrichtung n in nematischen bzw. cholesterischen Phase (N*-Phase) einen Winkel von mehr als 5° ausbilden und die Flüssigkristallschicht aus einer ferroelektrischen (chiralsmektischen) Flüssigkristallmischung besteht, die mindestens eine Verbindung der Formel (I) enthält

R¹-(A¹-M¹)ₐ-(A²-M²)_{b}-A³-X-B¹-(B²)_{c}-R² (I)

worin die Symbole die folgenden Bedeutungen haben:
- R¹, R²: unabhängig voneinander gleich oder verschieden
a) Wasserstoff, Fluor, oder CN
   ein geradkettiger oder verzweigter (mit oder ohne asymmetrische C-Atome) Alkenyl-, Alkenyloxy-, Alkyl- oder Alkyloxy-Rest mit 2 - 16 C-Atomen, worin
   b1) eine oder zwei nicht terminale -CH₂-Gruppen ersetzt sein können durch -O- , -OC(=O)- , -(C=O), -C(=O)O-, -Si(CH₃)₂-, -CH(CL)- und / oder eine oder zwei -CH₂-Gruppen ersetzt sein können durch -CH=CH- oder -C≡C und worin auch ein oder mehrere H-Atome durch F ersetzt sein können und/ oder
   b2) eine oder mehrere -CH₂-Gruppen ersetzt sein können durch Phenylen-1,4-diyl (gegebenenfalls 1-oder 2-fach durch F substituiert), Phenylen-1,3-diyl (gegebenenfalls 1- oder 2-fach durch F substituiert), Cyclohexan-1,4-diyl (gegebenenfalls 1-fach durch F oder CN substituiert) oder Cylopropan-1,2-diyl
   und worin auch ein oder mehrere H-Atome durch F ersetzt sein können
mit den Maßgaben, daß nur einer der Reste R¹, R² Wasserstoff, F oder CN sein kann und zwei benachbarte -CH₂-Gruppen nicht durch -O- ersetzt sein können
- M¹, M²: unabhängig voneinander gleich oder verschieden
-C(=O)O-, -OC(=O)-, -CH₂O-, -OCH₂-, -CF₂O-, -OCF₂-, -CH₂CH₂-, -CF₂CF₂-, -CH=CH-, -CH=CF-, -CF=CF-, -C≡C-, -CH₂CH₂C(=O)O-, -OC(=O)CH₂CH₂-, -(CH₂)₄-, -OCH₂CH₂CH₂-, -CH₂CH₂CH₂O-, - OCH₂CF₂CH₂-, -CH₂CF₂CH₂O- oder eine Einfachbindung
- A¹, A², A³: unabhängig voneinander gleich oder verschieden Cyclohexan-1,4-diyl (gegebenenfalls 1-fach substituiert durch F, CH₃, CN), Cyclohex-1-en-1,4-diyl, Cyclohex-2-en-1,4-diyl, 2-Oxocyclohexan-1,4-diyl, 2-Cyclohexen-1-on-3,6-diyl, 1-Alkyl-1-sila-cyclohexan-1,4-diyl, Bicyclo[2.2.2]octan-1,4-diyl, Spiro[4.5]decan-2,8-diyl, Spiro[5.5]undecan-3,9-diyl, Phenylen-1,4-diyl (gegebenenfalls 1- oder 2-fach substituiert durch CN, CH₃, CF₃, OCF₃ , gegebenenfalls 1-, 2-, 3- oder 4-fach substituiert durch F), Phenylen-1,3-diyl (gegebenenfalls 1- oder 2-fach substituiert durch CN, CH₃, CF₃, OCF₃, gegebenenfalls 1-, 2- , 3- oder 4-fach substituiert durch F), Thiophen-2,5-diyl, Thiophen-2,4-diyl, (1,3,4)-Oxadiazol-2,5-diyl, (1,3,4)-Thiadiazol-2,5-diyl, 1,3-Thiazol-2,5-diyl, 1,3-Thiazol-2,4-diyl, (1,3)-Oxazol-2,5-diyl, Isoxazol-2,5-diyl, Indan-2,6-diyl, Naphthalin-2,6-diyl (gegebenenfalls 1- oder 2-fach substituiert durch F oder CN), 1,2,3,4-Tetrahydronaphthalin-2,6-diyl, Decalin-2,6-diyl, Pyrimidin-2,5-diyl (gegebenenfalls 1-fach substituiert durch F), Pyridin-2,5-diyl (gegebenenfalls 1- oder 2-fach substituiert durch F), Pyrazin-2,5-diyl (gegebenenfalls 1-fach substituiert durch F), Pyridazin-3,6-diyl, Chinolin-2,6-diyl, Chinolin-3,7-diyl, Isochinolin-3,7-diyl, Chinazolin-2,6-diyl, 5,6,7,8-Tetrahydrochinazolin-2,6-diyl, Chinoxalin-2,6-diyl, 1,3-Dioxan-2,5-diyl (gegebenenfalls 1-fach substituiert durch CN), Benzothiazol-2,6-diyl, Piperidin-1,4-diyl, Piperazin-1,4-diyl
- B¹: Cyclohexan-1,4-diyl (gegebenenfalls 1- oder 2-fach substituiert durch F, CH₃, CN), Perfluorcyclohexan-1,4-diyl, Cyclohex-1-en-1,4-diyl, Cyclohex-2-en-1,4-diyl, 1-Alkyl-1-sila-cyclohexan-1,4-diyl, Bicyclo[2.2.2]octan-1,4-diyl, Cyclopentan-1,3-diyl, Cycloheptan-1,4-diyl, Tetrahydrofuran-2,5-diyl, Tetrahydrofuran-2,4-diyl, Phenylen-1,4-diyl (gegebenenfalls 1- oder 2-fach substituiert durch CN, CH₃, CF₃, OCF₃, gegebenenfalls 1-, 2-, 3- oder 4-fach substituiert durch F), Phenylen-1,3-diyl (gegebenenfalls 1- oder 2-fach substituiert durch CN, CH₃, CF₃, OCF₃, gegebenenfalls 1- oder 2- oder 3-fach substituiert durch F), Thiophen-2,5-diyl (gegebenenfalls 1-fach substituiert durch F), Thiophen-2,4-diyl (gegebenenfalls 1-fach substituiert durch F), 1,3-Thiazol-2,5-diyl (gegebenenfalls 1-fach substituiert durch F), 1,3-Thiazol-2,4-diyl (gegebenenfalls 1-fach substituiert durch F), (1,3,4)-Thiadiazol-2,5-diyl, 1,3-Dioxan-2,5-diyl (gegebenenfalls 1-fach substituiert durch CN), Tetrahydropyran-2,5-diyl, 6,6-Difluortetrahydropyran-2,5-diyl, 6,6-Difluor-2,3-dihydro-6H-pyran-2,5-diyl, 6-Fluor-3,4-dihydro-2H-pyran-2,5-diyl, Piperidin-1,4-diyl, Piperazin-1,4-diyl, Pyrimidin-2,5-diyl (gegebenenfalls 1-fach substituiert durch F), Pyridin-2,5-diyl (gegebenenfalls 1-fach substituiert durch F), 1,2,3,4-Tetrahydronaphthalin-2,6-diyl, Decalin-2,6-diyl
- B²: Cyclohexan-1,4-diyl (gegebenenfalls 1- oder 2-fach substituiert durch F, CH₃, CN), Cyclohex-1-en-1,4-diyl (gegebenenfalls 1-fach substituiert durch F), Cyclohex-2-en-1,4-diyl, 1-Alkyl-1-silacyclohexan-1,4-diyl, Bicyclo[2.2.2]octan-1,4-diyl, Phenylen-1,4-diyl (gegebenenfalls 1- oder 2-fach substituiert durch CN, CH₃, CF₃, OCF₃, gegebenenfalls 1-, 2-, 3- oder 4-fach substituiert durch F), Phenylen-1,3-diyl (gegebenenfalls 1- oder 2-fach substituiert durch CN, CH₃, CF₃, OCF₃, gegebenenfalls 1- oder 2- oder 3-fach substituiert durch F), Thiophen-2,5-diyl, Thiophen-2,4-diyl, 1,3-Thiazol-2,5-diyl, 1,3-Thiazol-2,4-diyl, (1,3,4)-Thiadiazol-2,5-diyl, 1,3-Dioxan-2,5-diyl (gegebenenfalls 1-fach substituiert durch CN), Tetrahydrofuran-2,5-diyl, Tetrahydropyran-2,5-diyl, 6,6-Difluortetrahydropyran-2,5-diyl, 6,6-Difluor-2,3-dihydro-6H-pyran-2,5-diyl, 6-Fluor-3,4-dihydro-2H-pyran-2,5-diyl, Pyrimidin-2,5-diyl (gegebenenfalls 1-fach substituiert durch F), Pyridin-2,5-diyl (gegebenenfalls 1-fach substituiert durch F), Indan-2,6-diyl, Piperidin-1,4-diyl, Piperazin-1,4-diyl, Pyrimidin-2,5-diyl (gegebenenfalls 1-fach substituiert durch F)
- X: -(CH₂)ₙ-, wobei
a) eine oder zwei -CH₂-Gruppen durch -O- oder -C(=O)- ersetzt sein können und/oder
b) eine -CH₂CH₂-Gruppe durch -CH=CH- ersetzt sein kann und ein oder mehrere H der -CH₂-Gruppen durch F ersetzt sein können
mit den Maßgaben, daß
1) n 2, 3 oder 4 bedeutet
2) zwei benachbarte -CH₂-Gruppen nicht durch -O- ersetzt sein können
- a, b, c: Null, 1 oder 2 mit den Maßgaben, daß
1) a 1 sein muß, wenn R¹ Wasserstoff, F oder CN bedeutet
2) die Summe a+b+c mindestens 1 ist
3) die in der Klammer stehenden Reste A bzw. M unterschiedliche oder gleiche Bedeutung haben können, wenn der entsprechende Index 2 ist,
mit dem hier und im folgenden geltenden Verständnis, daß die Bezeichnung von bivalenten Resten im "freien Zustand" erfolgte und maßgeblich für die Charakterisierung der Verbindungen ist, obwohl die Bezeichnungen der bivalenten Reste als Teil der gesamten Markush-Formel - worunter sowohl bildlicher als auch spiegelbildlicher Einbau verstanden werden - streng nach IUPAC jedoch anders lauten können.

Gemäß einer Ausführungsform sind R¹, R² keine Alkenyl- oder Alkenyloxyreste.

Vorzugsweise weist die Flüssigkristallmischung eine spontane Polarisation von <200 nC/cm², besonders bevorzugt <25 nC/cm², insbesondere <15 nC/cm² auf, wobei der nachstehend definierte Wert DT (15,1) >20 ist.

Die Herstellungsverfahren der für die erfindungsgemäßen Mischungen geeigneten Materialien sind im Prinzip bekannt, ebenso wie die Herstellung von Flüssigkristallmischungen aus den Einzelkomponenten.

So sind z.B. beschrieben
Thiadiazol-Derivate: EP-A-0 309 514; EP-A-0 335 348; US 5,076,961; US 5,200,109
Thiazol-Derivate: EP-A-0 309 514; EP-A-0 439 170
Pyrimidin-Derivate: EP-A-0 220 296; 220 297; 227 717; 224 579; 293 910; US 4,891,151; EP-B 0 308 794; US 5,200,521; US 5,370,823; DE-A 43 00 435
4-Fluorpyrimidin-Derivate: US 5,344,585; EP-A-0 158 137
Pyridin-Derivate: WO 86 / 06401; EP-A-0 206 228; FP-A-0 239 403; US 4,795,587; JP-A 07309858; JP-A 62207257; JP-A 05331143; JP-A 05213875; JP-A 04356464; JP-A 01031765; JP-A 08062560; DE-A 40 26 223
fluorierte Pyridin-Derivate: JP-B 2079059; US 5,389,291; US 5,630,962; US 5,445,763; DE-A 44 27 199 ; US 5,445,763;
2-Fluorpyrazin-Derivate: US 5,562,859
1,2,3,4-Tetrahydrochinazolin-Derivate: US 4,402,849; JP-A 08062559; JP-A 08059629; JP-A 07207267
Chinolin-Derivate: DE-A 195 38 404
Dioxan-Derivate: Flüssige Kristalle in Tabellen II, pp. 349-352; DD 249 277; DD 249 278; DD 249 279
Isoxazol-Derivate: Mol. Cryst.Liq.Cryst. 1993, 225, 175-182
Pyran-Derivate: JP-A 10168076; JP-A 10176168
Naphthalin-Derivate: Flüssige Kristalle in Tabellen II, pp. 313-322; DE-A 195 17 056; DE-A 195 17 038; DE-A 195 70 60; DE-A 195 22 167; DE-A 196 52 247; WO 92 / 16500; EP-A-0 302 875
Indan-Derivate: EP-A-0 546 338
Fluorphenyl-Derivate: EP-A-0 210 215; GB-A 2,198,743
Difluorphenyl-Derivate: EP-A-0 210 215; EP-A-0 332 024, 332 025,
Trifluorphenyl-Derivate: EP-A-0 602 596
Tetrafluorphenyl-Derivate: EP-A-0 110 002; EP-A-0 113 293; EP-A-0 422 996; JP 58188840; JP 59010553; JP 02180869; Mol. Cryst.Liq.Cryst. 127, 413 (1985)
Biphenyl- und Terphenyl-Derivate: Flüssige Kristalle in Tabellen II, pp. 269-304; EP-A-0 213 841; EP-A-0 263 843; GB-B 2,198,743; GB-B 2,200,912; EP-B-0 395 666; EP-B-0 332 006; EP-A-0 360 042
Bicyclo[2.2.2]octan-Derivate: Flüssige Kristalle in Tabellen II, pp. 85-95
Cyclohexan-Derivate: Flüssige Kristalle in Tabellen II, pp. 32-72; Landolt-Börnstein Bd. IV / 7a, pp. 160-176; DE-A 23 44 732; 24 50 088; 24 29 093; 26 36 684; 27 01 591; 27 52 975; DE-A 32 31 707; EP-A 0 233 267; EP-A 0 238 576
Cyclohexen-Derivate: Flüssige Kristalle in Tabellen II, pp. 79 - 82; US 5,271,864; DE-A 39 30 119;
1-Alkyl-silacyclohexan-Derivate: EP-A-0 761 674; 742 222; 732 335; 727 428
meta-substituierte Mesogene: US 5,447,656
Thiophen-Derivate:Flüssige Kristalle in Tabellen II, pp. 353-356; EP-A-0 458 347; EP-A-0 364 923; EP-A-0 392 510; EP-A-0 459 406
Benzothiazol-Derivate: JP-A 09059266
Phenanthren-Derivate: US 5,648,021; EP-B 0 743 971; DE-A 195 24 230; DE-A 197 48 819; DE-A 196 53 010; DE-A 196 53 009; DE-A 196 53 008
Fluoren-Derivate: Landolt-Börnstein Bd. IV / 7a, pp. 36 - 41; DE-A 197 20 289
Ethin-Derivate: US 5,626,792; 5,178,791; 5,457,235; JP 10195025; WO 98 23637; JP 10130188; JP 10120600; EP-A-0 799 878
Ethan-Derivate: WO 98 23583; WO 98 23563; JP 10147544; JP 09235550; JP 09124660; JP 09087210; JP 06056703; DE-A 42 38 377; JP 06025030; DE-A 32 01 721
sowie Verbindungen mit den Strukturelementen
silylalkyl- EP-B-0 366 561
cyclopropylalkyl- EP-B-0 318 423 / 398 155;
perfluoralkyl- Ferroelectrics 1988, 85, 375-384 bzw. US 4,886,619, 5,082,587, 5,254,747 , 5,262,082 , 5,437,812 oder 5,482,650;
perfluorcyclohexyl DE-A 197 48 818
α-fluorcarbonyloxy, Liquid Crystals 1997, vol. 23, no.5, pp. 659-666
2,3-Difluoralkyloxy- US 5,051,506
2-Fluoralkyloxy- US 4,798,680
α-chlorcarbonyloxy- US 4,855,429
Methyl-verzweigte Alkylketten EP-B-0 201 578, 211 030 ; DE-A 196 27 899
mit nur einer Flügelgruppe *:* EP-A-0 541 081; EP-A-0 606 090
propionyloxy-: DD 284 894; EP-A-0 552 658; GB-B 2,235,192
tetrahydrofuranoyloxy: EP-A-0 355 561
cyanoalkyl: EP-A-0 310 620; EP-A-0 333 760; WO 89/05792
mit einer Oxiran-Gruppe*:* EP-B-0 263 437; EP-B-0 292 954; EP-B-0 365 820; DE-A 4133710; JP-B 2089393; JP-B 3-512741
mit einer 1,3-Dioxolan-Gruppe: EP-B-0 288 813; EP-B-0 361 272; EP-B-0 462 156; EP-B-0 351 746

Es wurde erfindungsgemäß gefunden, daß durch den Einsatz der Verbindungen der Formel (I) Aktivmatrix-Displays zugänglich sind, in denen die ferroelektrische smektische Phase über einen großen Temperaturbereich stabil ist. Zudem ist der Tiltwinkel über einen weiten Temperaturbereich sehr stabil, d.h. er unterliegt nur sehr geringen Änderungen. Gleiches gilt für den Lagenkippwinkel.

Vorzugsweise bedeutet in der Formel (I) X -OC(=)O-, -OCH₂- oder -OC(=O)CH₂CH₂-, besonders bevorzugt -O(C=O).

Vorzugsweise bedeutet B1 Cyclohexan-1,4-diyl, Cyclohex-1-en-1,4-diyl, Phenylen-1,4-diyl, gegebenenfalls 1-fach oder 2-fach durch F substituiert, oder Thiophen-2,5-diyl, besonders bevorzugt Cyclohexan-1,4-diyl oder Thiophen-2,5-diyl.

Vorzugsweise bedeutet A1 Pyrimidin-2,5-diyl (gegebenenfalls 1-fach durch F substituiert), Pyridin-2,5-diyl (gegebenenfalls 1-fach durch F substituiert), Phenylen-1,4-diyl (gegebenenfalls 1- oder 2-fach durch F substituiert) oder (1,3,4)-Thiadiazol-2,5-diyl.

Bevorzugte Verbindungen der Formel (I) entsprechen den Formeln 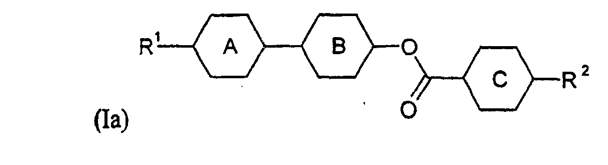 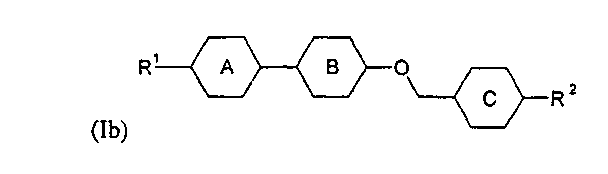 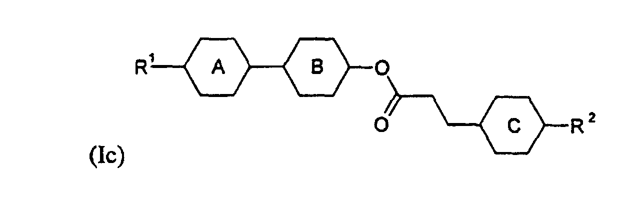 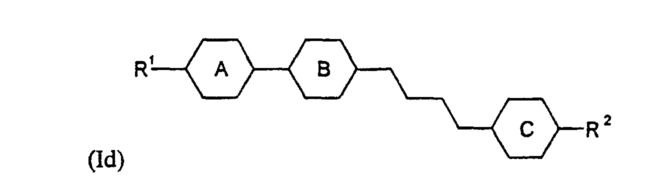 worin R¹, R² die weiter oben angebenen Bedeutungen haben und 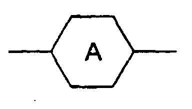 einen bivalenten Rest, ausgewählt aus der Gruppe Phenylen-1,4-diyl, gegebenenfalls einfach oder zweifach durch F substituiert, Pyrimidin-2,5-diyl, gegebenenfalls einfach durch F substituiert, Pyridin-2,5-diyl, gegebenenfalls einfach durch F substituiert, (1,3,4)-Thiadiazol-2,5-diyl, Indan-2,5-diyl, Cyclohexan-1,4-diyl, gegebenenfalls einfach durch F oder CN substituiert, Cylohex-1-en-1,4-diyl, 1,2,3,4-Tetrahydrochinazolin-2,6-diyl bedeutet 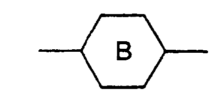 einen bivalenten Rest, ausgewählt aus der Gruppe Phenylen-1,4-diyl, gegebenenfalls einfach oder zweifach durch F substituiert, Pyrimidin-2,5-diyl, gegebenenfalls einfach durch F substituiert, Pyridin-2,5-diyl, gegebenenfalls einfach durch F substituiert, Indan-2,5-diyl bedeutet 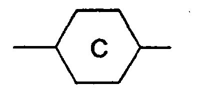 einen bivalenten Rest, ausgewählt aus der Gruppe Cyclohexan-1,4-diyl, gegebenenfalls einfach durch F oder CN substituiert, Cyclohex-1-en-1,4-diyl, (1,3)-Dioxan-2,5-diyl, gegebenenfalls einfach durch CN substituiert, Thiophen-2,5-diyl, Thiophen-2,4-diyl, Phenylen-1,4-diyl, gegebenenfalls einfach oder zweifach durch F substituiert, Phenylen-1,3-diyl, gegebenenfalls einfach oder zweifach durch F substituiert, bedeutet.

Besonders bevorzugte Verbindungen der Formel (I) entsprechen den Formeln 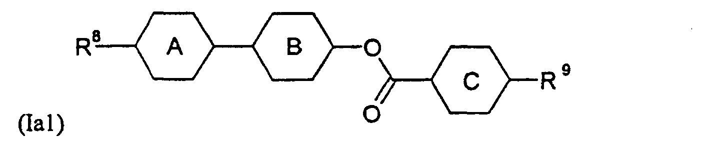 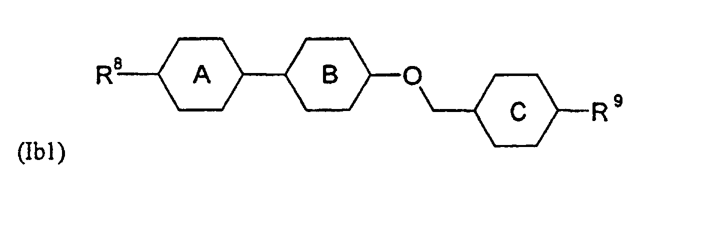 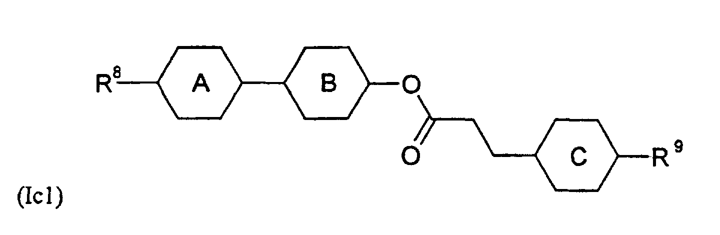 worin bedeuten: 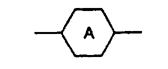 einen bivalenten Rest, ausgewählt aus der Gruppe Phenylen-1,4-diyl, gegebenenfalls einfach oder zweifach durch F substituiert, (1,3,4)-Thiadiazol-2,5-diyl, Pyrimidin-2,5-diyl, gegebenenfalls durch F substituiert, Pyridin-2,5-diyl, gegebenenfalls ortho zum Stickstoffatom durch F substituiert, 1,2,3,4-Tetrahydrochinazolin-2,6-diyl 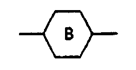 einen bivalenten Rest, ausgewählt aus der Gruppe Phenylen-1,4-diyl, gegebenenfalls einfach oder zweifach substituiert durch F, Pyrimidin-2,5-diyl, gegebenenfalls einfach substituiert durch F, Pyridin-2,5-diyl, gegebenenfalls einfach substituiert durch F 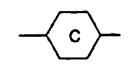 einen bivalenten Rest, ausgewählt aus der Gruppe Cyclohexan-1,4-diyl, Thiophen-2,5-diyl, Phenylen-1,4-diyl
und R⁸, R⁹ unabhängig voneinander Wasserstoff oder einen geradkettigen oder verzweigten Alkyl- oder Alkyloxy-Rest mit 1 bis 16 C-Atomen, worin auch eine oder zwei nicht terminale -CH₂-Gruppen ersetzt sein können durch -O- oder -C(=O)- oder -CH=CH- mit den Maßgaben, daß R⁸, R⁹ nicht beide Wasserstoff sein können und zwei benachbarte -CH₂-Gruppen nicht durch -O- ersetzt sein können.

Ganz besonders bevorzugte Verbindungen entsprechen den Formeln

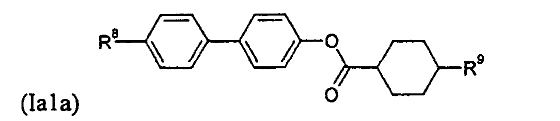

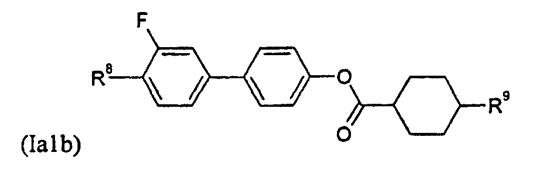

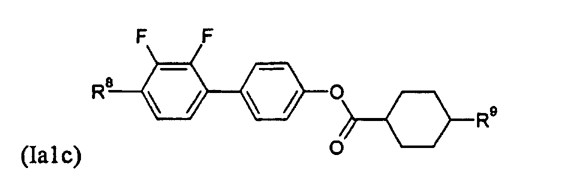

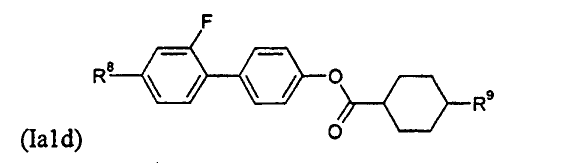

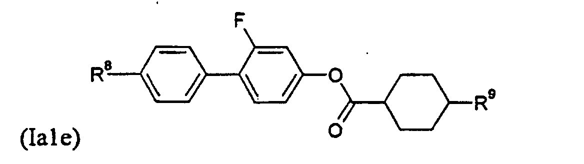

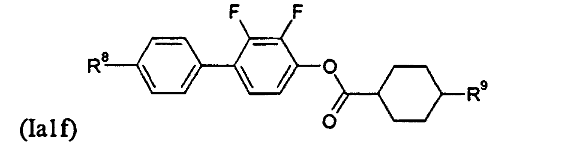

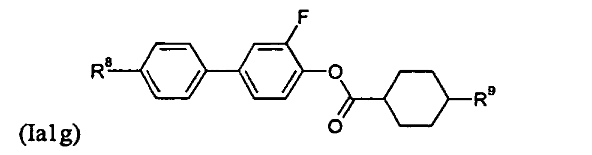

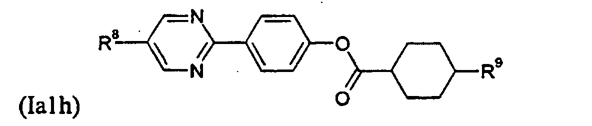

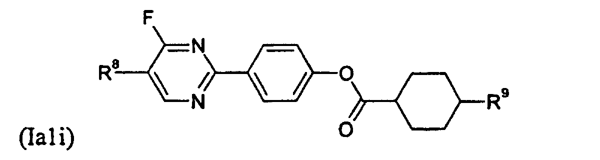

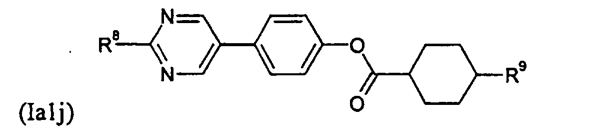

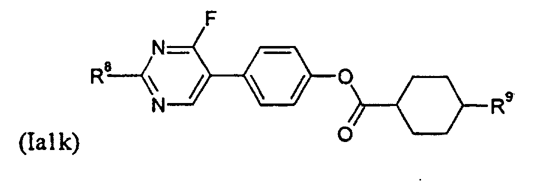

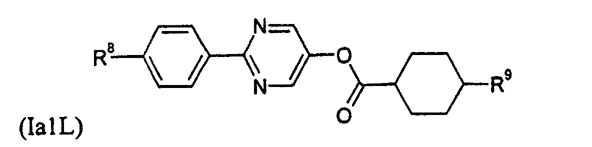

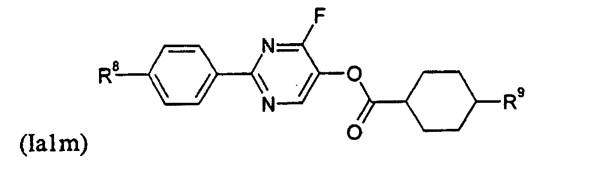

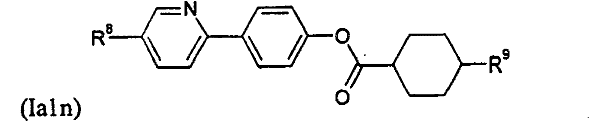

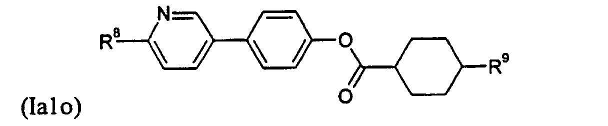

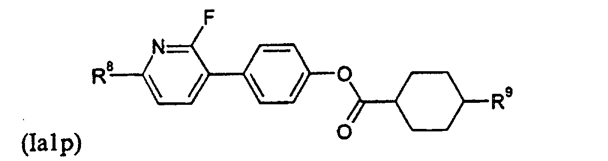

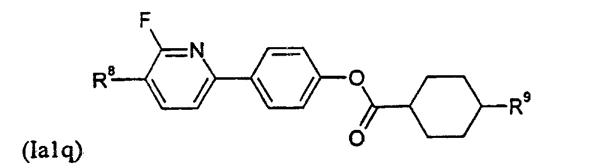

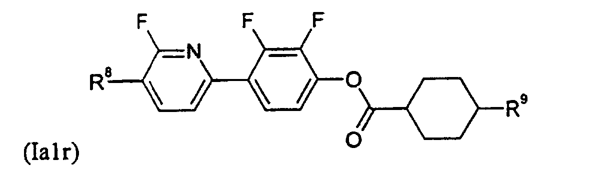

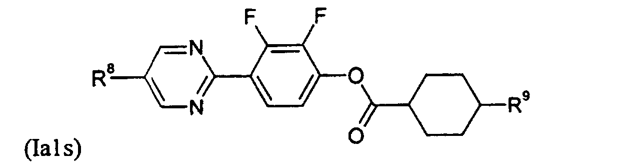

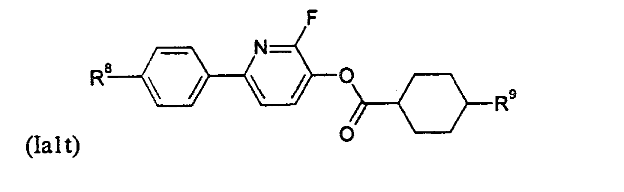

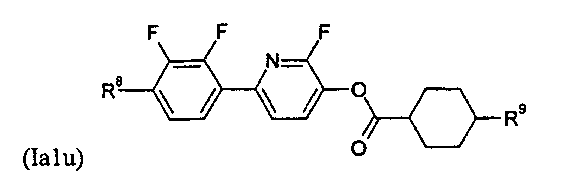

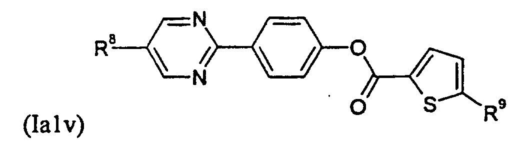

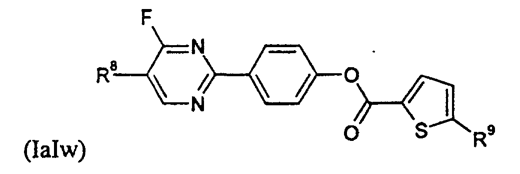

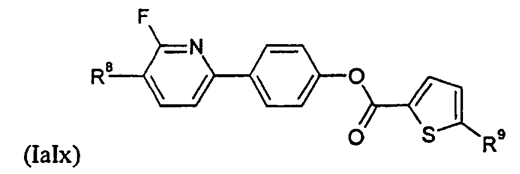

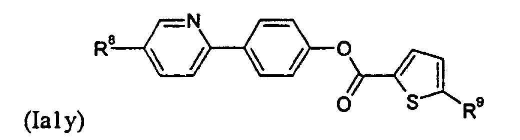

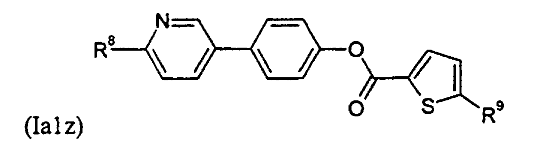

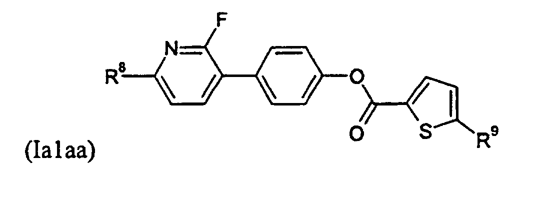

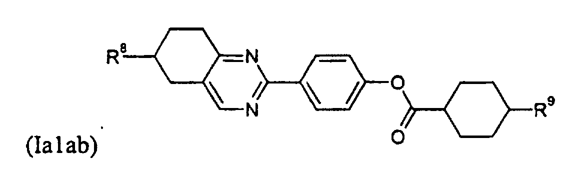

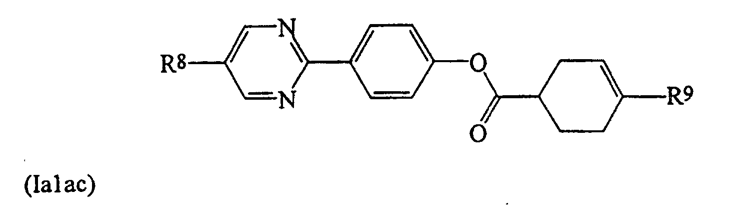

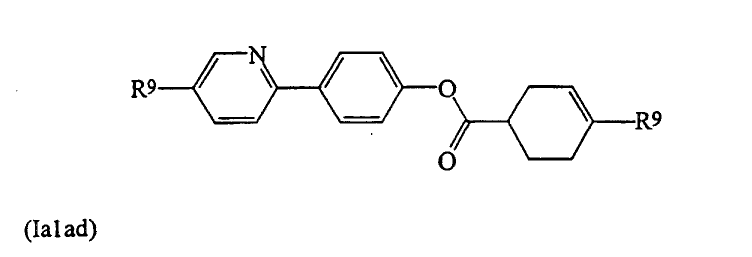

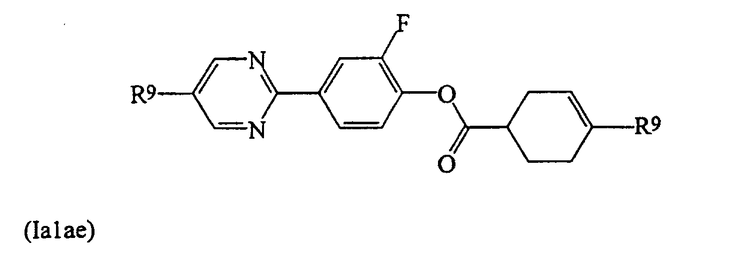

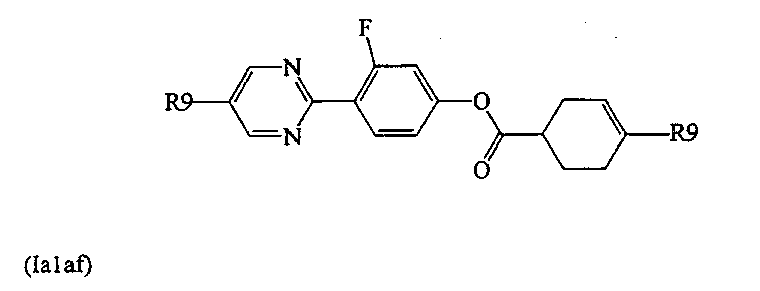

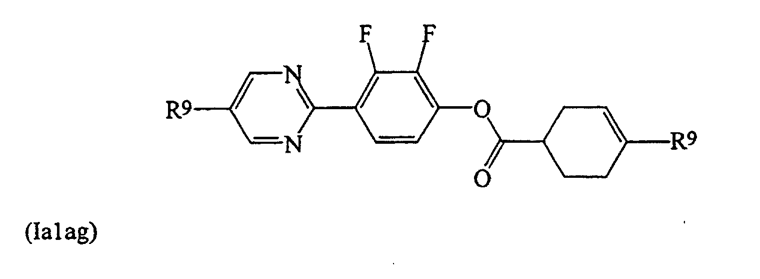

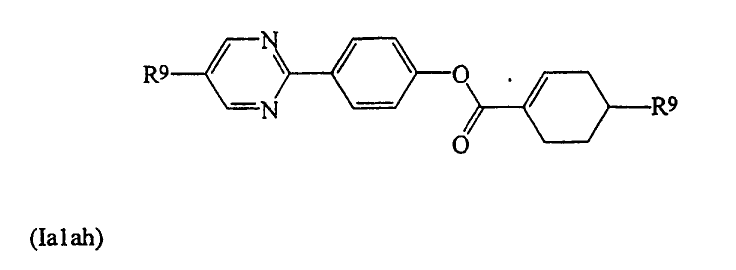

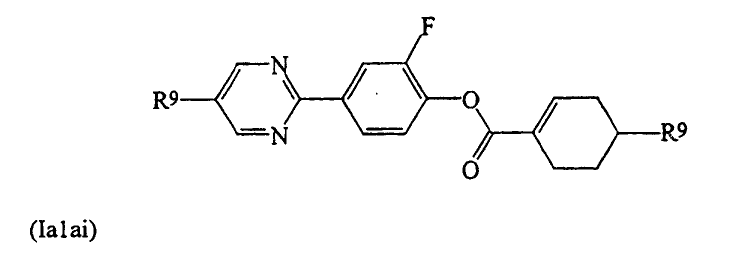

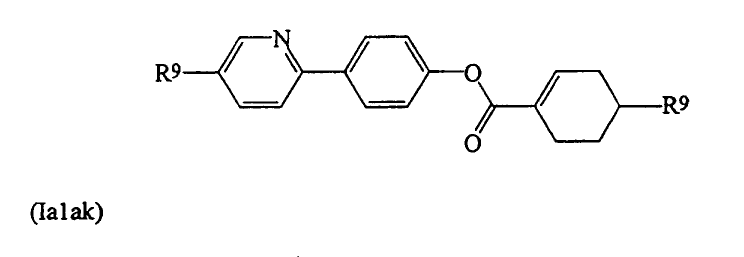

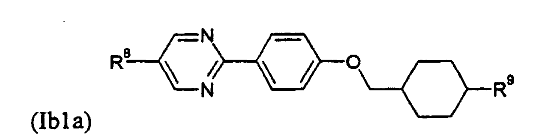

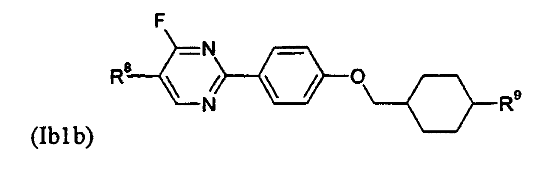

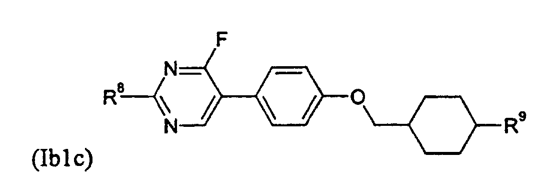

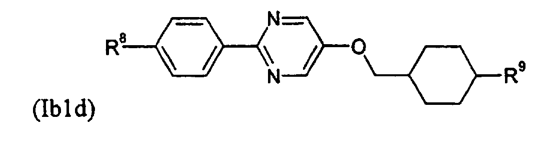

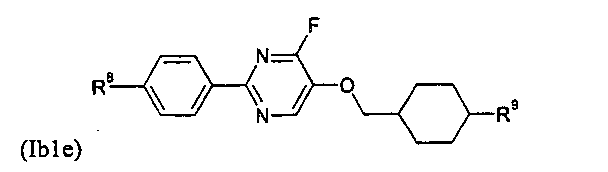

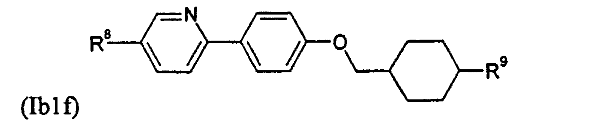

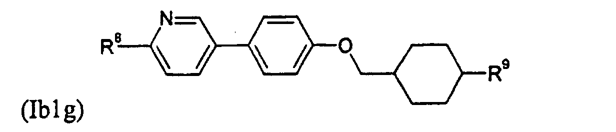

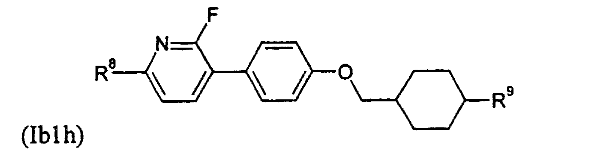

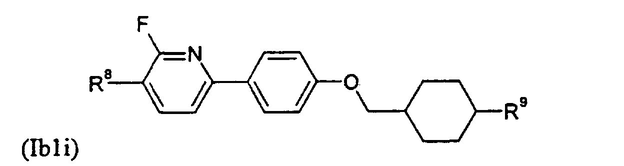

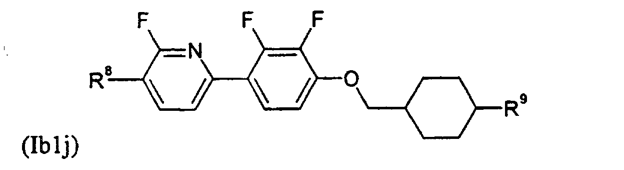

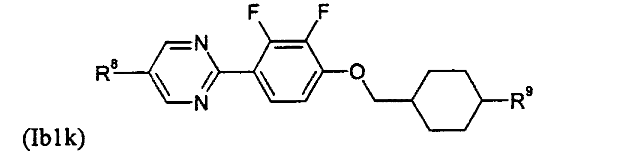

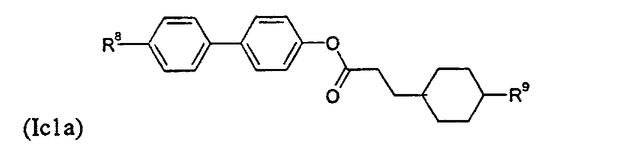

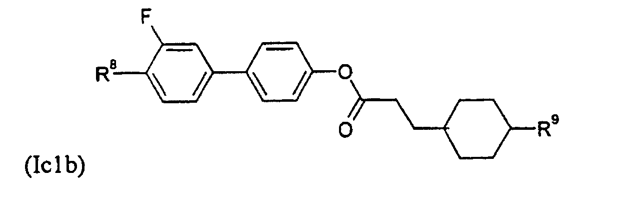

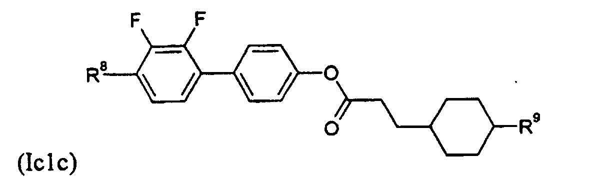

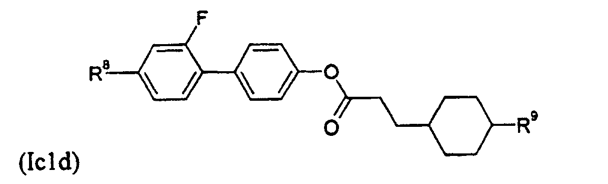

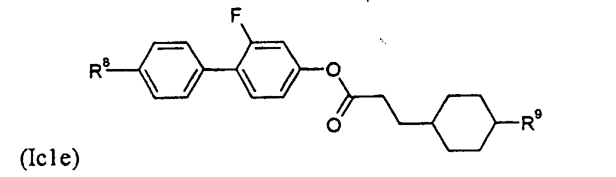

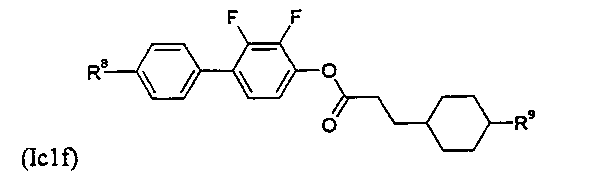

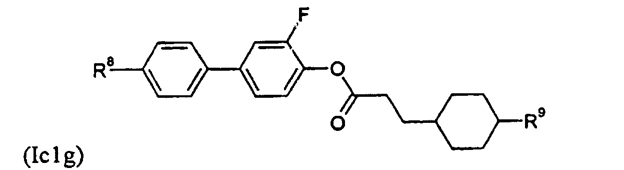

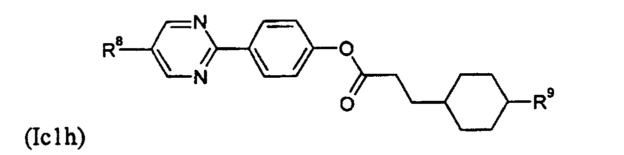

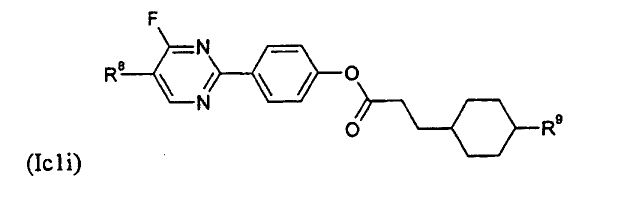

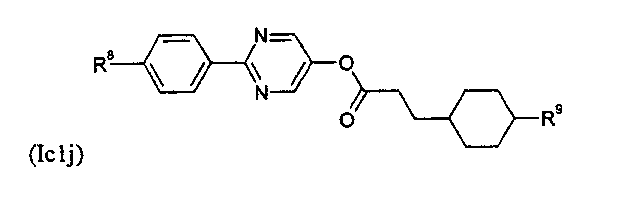

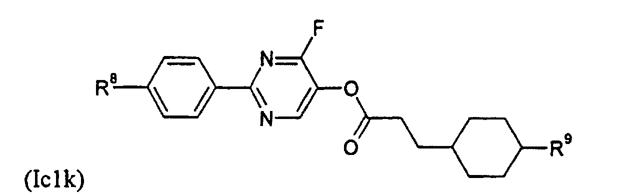

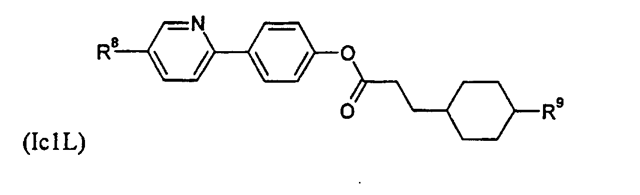

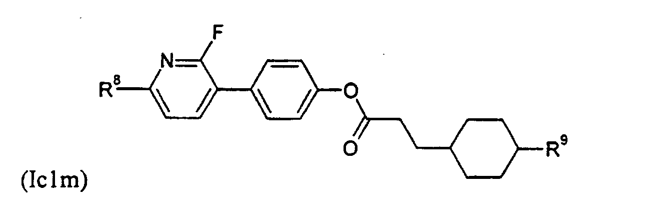

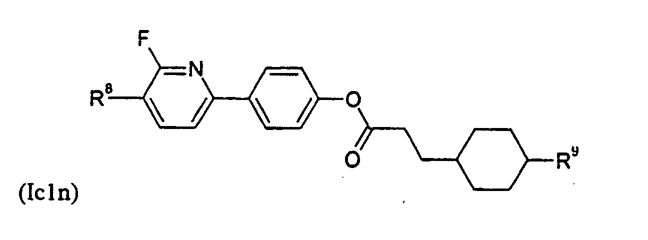

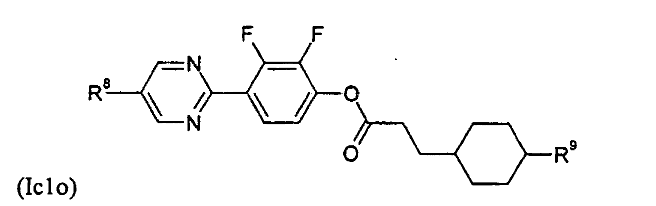

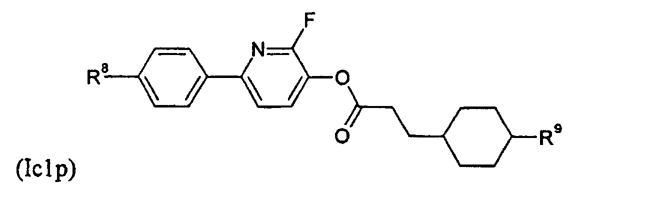

Dabei können die vorstehenden Formeln (Ialac) bis (Ialak) gegebenenfalls auch ausgenommen sein.

Die Flüssigkristallmischung des erfindungsgemäßen Displays enthält vorzugsweise neben einer oder mehrerer Verbindungen der Formel (I) noch 2 bis 30 weitere Verbindungen , die ausgewählt werden als ein oder mehrere Vertreter der Substanzklassen aus den Gruppen (II) bis (XVII) 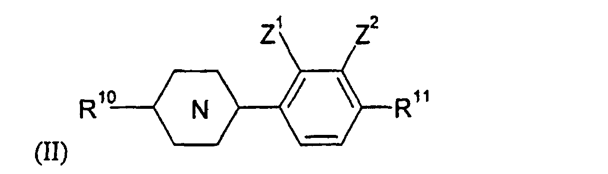 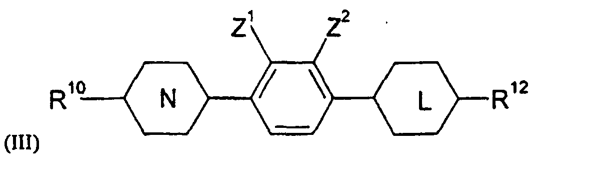 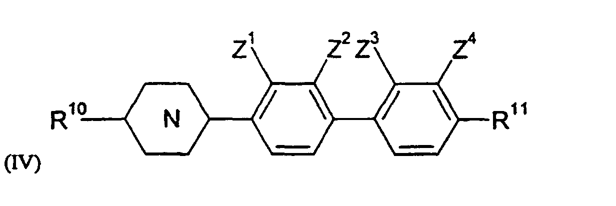 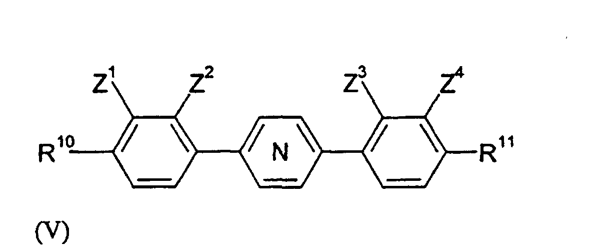 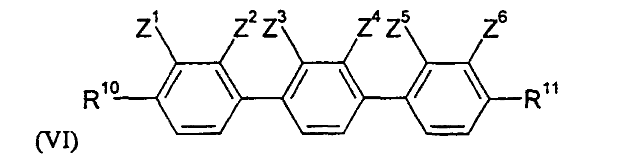 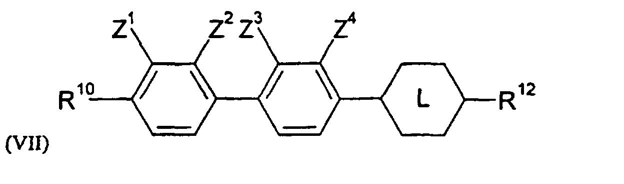 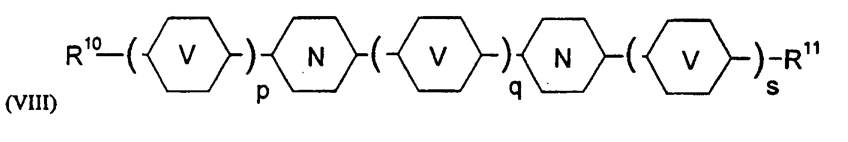 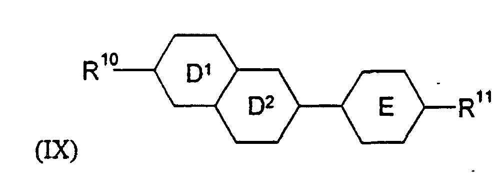 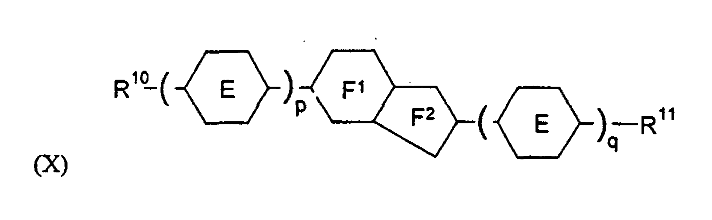 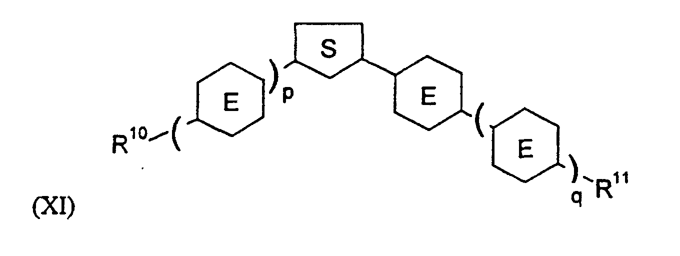 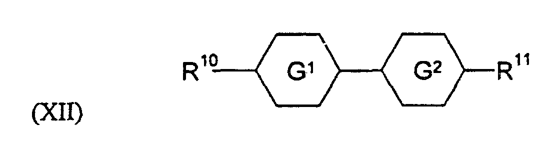 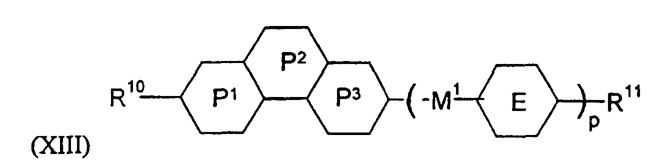 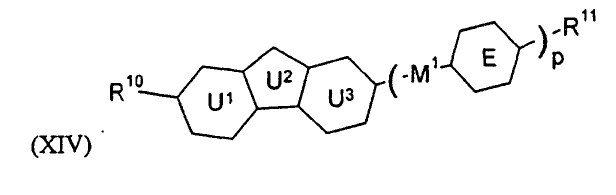 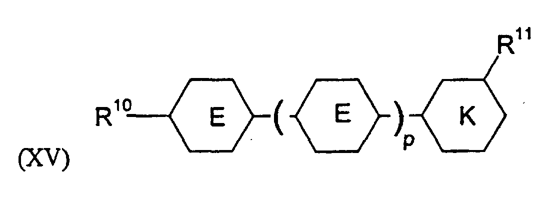 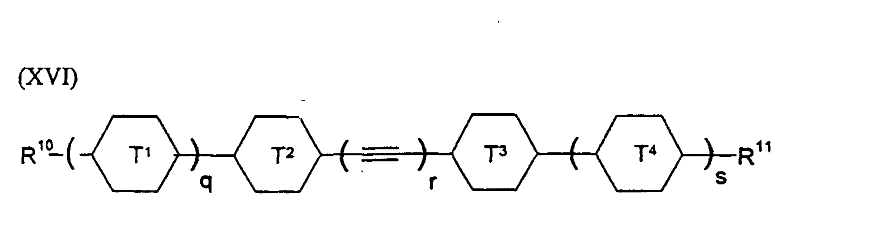 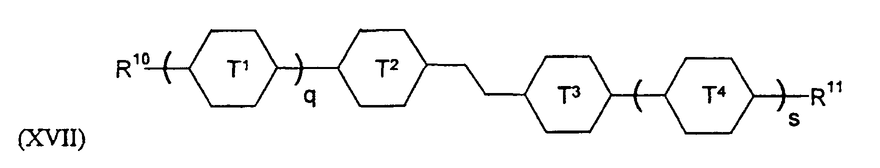 worin bedeuten:
R¹⁰, R¹¹ wie R¹, R^{2,} wobei zusätzlich jeweils die terminale -CH₃-Gruppe durch eine der folgenden chiralen Gruppen (optisch aktiv oder racemisch) ersetzt sein kann: 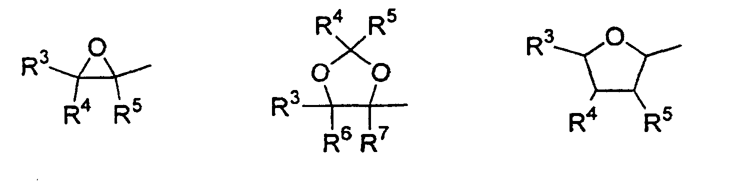 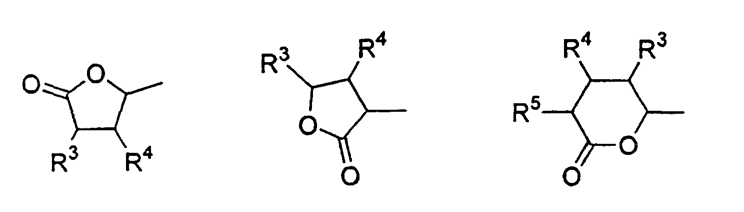 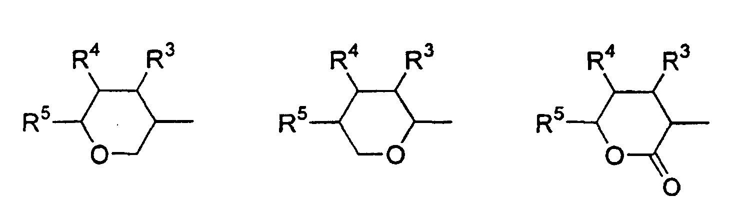 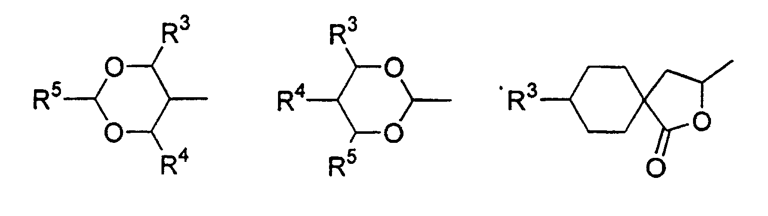 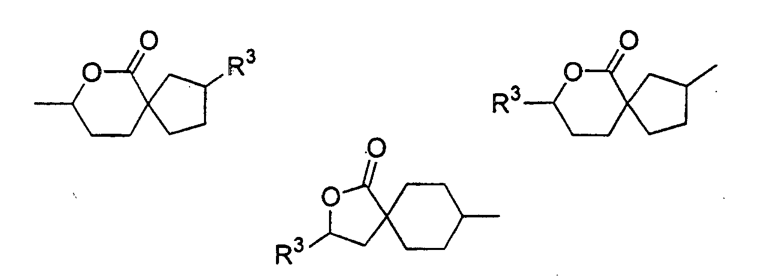 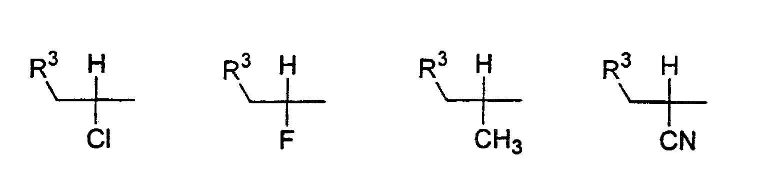 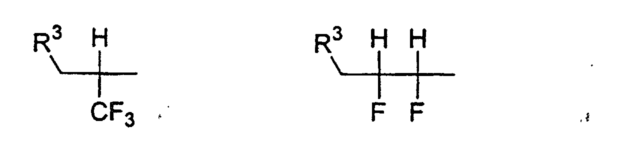
R³, R⁴, R⁵, R⁶, R⁷ sind gleich oder verschieden
   a) Wasserstoff
   b) ein geradkettiger oder verzweigter Alkylrest (mit oder ohne asymmetrischen Kohlenstoffatomen) mit 1 bis 16 C-Atomen, wobei
      b1) eine oder mehrere nicht benachbarte und nicht terminale CH₂-Gruppen durch -O- ersetzt sein können und/oder
      b2) eine oder zwei CH₂-Gruppen durch -CH=CH- ersetzt sein können,
   c) R⁴ und R⁵ zusammen auch -(CH₂)₄- oder -(CH₂)₅-, wenn sie an ein Oxiran-, Dioxolan-, Tetrahydrofuran-, Tetrahydropyran-, Butyrolacton- oder Valerolacton-System gebunden sind;
R¹² Wasserstoff oder einen geradkettigen oder verzweigten Alkylrest (mit oder ohne asymmetrische C-Atome) mit 1 bis 16 C-Atomen, worin auch ein oder mehrere H durch F ersetzt sein können und worin auch eine oder zwei nicht benachbarte, nicht terminale - CH₂-Gruppen durch -O- ersetzt sein können
Z¹,Z²,Z³,Z⁴ ,Z⁵,Z⁶ unabhängig voneinander H oder F 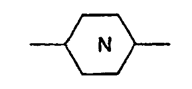 einen bivalenten Rest ausgewählt aus der Gruppe Pyridin-2,5-diyl, gegebenenfalls einfach durch F substituiert, Pyrimidin-2,5-diyl, gegebenenfalls einfach durch F substituiert, Pyrazin-2,5-diyl, gegebenenfalls einfach durch F substituiert, 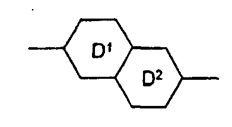 einen bivalenten Rest ausgewählt aus der Gruppe Naphthalin-2,6-diyl, worin auch ein oder zwei Ring-C-Atome durch N ersetzt sein können und das auch einfach oder zweifach durch F oder CN substituiert sein kann und worin auch D¹ oder D² einen (gesättigten) Alicyclus bedeuten kann 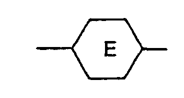 einen bivalenten Rest ausgewählt aus der Gruppe Phenylen-1,4-diyl, gegebenenfalls einfach oder zweifach substituiert durch CN oder einfach, zweifach, dreifach oder vierfach substituiert durch F, Pyridin-2,5-diyl, gegebenenfalls einfach substituiert durch F, Pyrimidin-2,5-diyl, gegebenenfalls einfach substituiert durch F, Cyclohexan-1,4-diyl 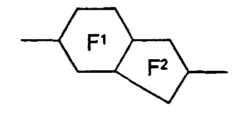 einen bivalenten Rest ausgewählt aus der Gruppe Indan-2,5-diyl, gegebenenfalls im aromatischen Ring einfach oder zweifach substituiert durch F, Indan-1-on-2,6-diyl, gegebenenfalls im aromatischen Ring einfach oder zweifach substituiert durch F, Benzothiazol-2,6-diyl, Benzothiazol-2,5-diyl, Benzo[b]thiophen-2,5-diyl, Benzo[b]thiophen-2,6-diyl 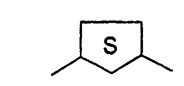 einen bivalenten Rest ausgewählt aus der Gruppe (1,3,4)-Thiadiazol-2,5-diyl, (1,3)-Thiazol-2,5-diyl, Thiophen-2,5-diyl, (1,3,4)-Oxadiazol-2,5-diyl, (1,3)-Oxazol-2,5-diyl, Isoxazol-2,5-diyl 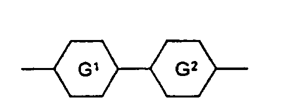 einen bivalenten Rest ausgewählt aus der Gruppe 1,1'-Biphenyl-4,4'-diyl, gegebenenfalls einfach oder zweifach substituiert durch CN oder einfach, zweifach, dreifach oder vierfach substituiert durch F , 1,1'-Phenylcyclohexyl-4,4'-diyl, 5,5'-Pyridylpyrimidin-2,2'-diyl, gegebenenfalls in einem oder beiden der Heterocyclen einfach durch F substituiert, 5,2'-Pyridylpyrimidin-2,5'diyl, gegebenenfalls in einem oder beiden der Heterocyclen einfach durch F substituiert, 1,2'-Phenyldioxan-4,5'-diyl, 1,2'-(2-Fluorphenyl)dioxan-4,5'-diyl, 1,2'-(3-Fluorphenyl)dioxan-4,5'-diyl, 1,2'- (2,3-Difluorphenyl)dioxan-4,5'-diyl 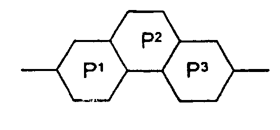 einen bivalenten Phenanthren-2,7-diyl-Rest, bei dem auch ein oder zwei Ring-C-Atome durch N ersetzt sein können und der auch einfach, zweifach, dreifach oder vierfach durch F substituiert sein kann und bei dem P² und / oder P³ auch einen (gesättigten) Alicyclus bedeuten können 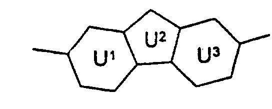 einen bivalenten Fluoren-2,7-diyl-Rest, dem auch die -CH₂-Gruppe in U² durch -C(=O)-, -CHF- oder -CF₂- ersetzt sein kann 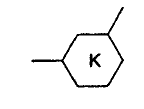 einen bivalenten Rest ausgewählt aus der Gruppe Phenylen-1,3-diyl, gegebenenfalls einfach oder zweifach substituiert durch F, Cyclohexan-1,3-diyl, gegebenenfalls einfach substituiert durch F oder CN, Pyridin-2,6-diyl, Pyridin-2,4-diyl, Pyridin-3,5-diyl, Pyridin-4,6-diyl, Pyrimidin-4,6-diyl, 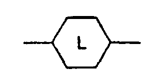 einen bivalenten Rest ausgewählt aus der Gruppe Cyclohexan-1,4-diyl, gegebenenfalls einfach substituiert durch CN, CH₃ oder zweifach durch F, Cyclohex-1-en-1,4-diyl , Perfluorcyclohexan-1,4-diyl, Cyclohex-2-en-1,4-diyl, 1-Alkyl-1-sila-cyclohexan-1,4-diyl, Bicyclo[2.2.2]octan-1,4-diyl 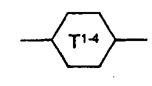 einen bivalenten Rest ausgewählt aus der Gruppe Phenylen-1,4-diyl, gegebenenfalls einfach oder zweifach substituiert durch CN oder F, Naphthalin-2,6-diyl, bei dem auch ein oder zwei Ring-C-Atome durch N ersetzt sein können und das gegebenenfalls einfach oder zweifach substituiert ist durch CN oder F, Cyclohexan-1,4-diyl, Cyclohex-1-en-1,4-diyl, Bicyclo[2.2.2]octan-1,4-diyl, (1,3)-Dioxan-2,5-diyl, Pyridin-2,5-diyl, gegebenenfalls einfach substituiert durch F, Pyrimidin-2,5-diyl, gegebenenfalls einfach substituiert durch F, (1,3,4)-Thiadiazol-2,5-diyl, Indan-2,5-diyl, gegebenenfalls im aromatischen Ring einfach oder zweifach substituiert durch F , Thiophen-2,5-diyl 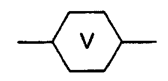 einen bivalenten Rest ausgewählt aus der Gruppe Phenylen-1,4-diyl, gegebenenfalls einfach oder zweifach substituiert durch CN oder F, Naphthalin-2-6-diyl, bei dem auch ein oder zwei Ring-C-Atome durch N ersetzt sein können und das gegebenenfalls einfach oder zweifach substituiert ist durch CN oder F, Cyclohexan-1,4-diyl, Cyclohex-1-en-1,4-diyl, Bicyclo[2.2.2]octan-1,4-diyl, (1,3)-Dioxan-2,5-diyl, Indan-2,5-diyl, gegebenenfalls im aromatischen Ring einfach oder zweifach substituiert durch F , Thiophen-2,5-diyl
p, q , s Null oder 1
r 1 oder 2.

Bevorzugt bedeuten in
**(II)** 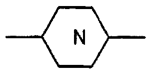 einen bivalenten Rest ausgewählt aus der Gruppe Pyridin-2,5-diyl, gegebenenfalls einfach durch F substituiert, Pyrimidin-2,5-diyl, gegebenenfalls einfach durch F substituiert
   Z¹, Z² beide H oder beide F
   R¹⁰, R¹¹unabhängig voneinander gleich oder verschieden Wasserstoff oder ein geradkettiger oder verzweigter (mit oder ohne asymmetrische C-Atome) Alkyl- oder Alkyloxy-Rest mit 2 - 16 C-Atomen, worin eine oder zwei nicht terminale -CH₂-Gruppen ersetzt sein können durch - CH=CH-, -OC(=O)-, -C(=O)O- und worin auch ein oder mehrere H-Atome durch F ersetzt sein können
   mit der Maßgabe, daß nur einer der Reste R¹⁰, R¹¹ Wasserstoff sein kann
**(III)** 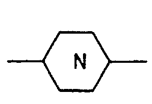 einen bivalenten Rest ausgewählt aus der Gruppe Pyridin-2,5-diyl, gegebenenfalls einfach durch F substituiert, Pyrimidin-2,5-diyl, gegebenenfalls einfach durch F substituiert 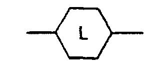 einen bivalenten Rest ausgewählt aus der Gruppe Cyclohexan-1,4-diyl, gegebenenfalls einfach substituiert durch CN, Cyclohex-1-en-1,4-diyl , Cyclohex-2-en-1,4-diyl, Z¹, Z² beide H oder beide F,
**(IV)** 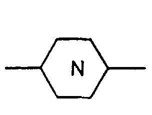 einen bivalenten Rest ausgewählt aus der Gruppe Pyridin-2,5-diyl, gegebenenfalls einfach durch F substituiert, Pyrimidin-2,5-diyl, gegebenenfalls einfach durch F substituiert
   Z¹, Z² beide H oder beide F,
   Z³, Z⁴ beide H oder beide F
(V) 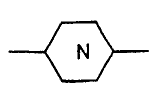 einen bivalenten Rest ausgewählt aus der Gruppe Pyridin-2,5-diyl, gegebenenfalls einfach durch F substituiert, Pyrimidin-2,5-diyl, gegebenenfalls einfach durch F substituiert
   Z¹, Z² beide H oder beide F,
   Z³, Z⁴ beide H oder beide F mit der Maßgabe, daß nicht Z¹, Z² und Z³, Z⁴ zugleich F bedeuten sollen
**(VI)**
   Z¹,Z²,Z³,Z⁴ ,Z⁵,Z⁶ ein Element dieser Gruppe ist gleich F oder (Z¹ und Z²) oder (Z³ und Z⁴) sind beide gleich F
**(VII)**
   Z¹ und Z² sind beide gleich H oder beide gleich F; Z³ und Z⁴ sind beide gleich H
**(VIII)** 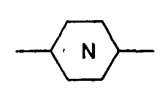 einen bivalenten Rest ausgewählt aus der Gruppe Pyridin-2,5-diyl, gegebenenfalls einfach durch F substituiert, Pyrimidin-2,5-diyl, gegebenenfalls einfach durch F substituiert 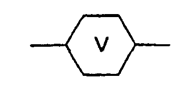 einen bivalenten Rest ausgewählt aus der Gruppe Phenylen-1,4-diyl, gegebenenfalls einfach oder zweifach substituiert durch F, Naphthalin-2,6-diyl, gegebenenfalls einfach oder zweifach substituiert durch F, Cyclohexan-1,4-diyl
   p, q, s Null oder 1; in der Summe Null oder 1
**(IX)** 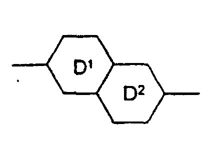 Naphthalin-2,6-diyl, das auch einfach oder zweifach durch F substituiert sein kann 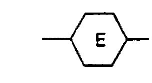 einen bivalenten Rest ausgewählt aus der Gruppe Phenylen-1,4-diyl, gegebenenfalls einfach oder zweifach substituiert durch F, Pyridin-2,5-diyl, gegebenenfalls einfach substituiert durch F, Pyrimidin-2,5-diyl, gegebenenfalls einfach substituiert durch F
**(X)** 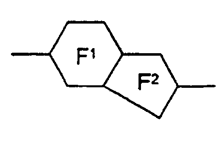 einen bivalenten Rest ausgewählt aus der Gruppe Indan-2,5-diyl, gegebenenfalls im aromatischen Ring einfach oder zweifach substituiert durch F, Indan-1-on-2,6-diyl, gegebenenfalls auch Benzothiazol-2,6-diyl 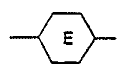 einen bivalenten Rest ausgewählt aus der Gruppe Phenylen-1,4-diyl, gegebenenfalls einfach oder zweifach substituiert durch F, Pyridin-2,5-diyl, gegebenenfalls einfach substituiert durch F, Pyrimidin-2,5-diyl, gegebenenfalls einfach substituiert durch F
   p gleich 1
   q gleich Null
**(XI)** 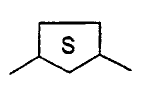 (1,3,4)-Thiadiazol-2,5-diyl 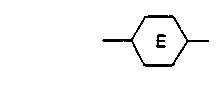 einen bivalenten Rest ausgewählt aus der Gruppe Phenylen-1,4-diyl, gegebenenfalls einfach oder zweifach substituiert durch F, Pyridin-2,5-diyl, gegebenenfalls einfach substituiert durch F, Pyrimidin-2,5-diyl, gegebenenfalls einfach substituiert durch F, Cyclohexan-1,4-diyl
   p gleich Null oder 1
   q gleich Null oder 1, mit der Maßgabe, daß q Null bedeutet, wenn p 1 bedeutet
**(XII)** 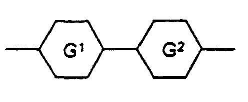 einen bivalenten Rest ausgewählt aus der Gruppe 1,1'-Biphenyl-4,4'-diyl, gegebenenfalls einfach oder zweifach substituiert durch F, 1,1'-Phenylcyclohexyl-4,4'-diyl, 5,5'-Pyridylpyrimidin-2,2'-diyl, gegebenenfalls in einem oder beiden der Heterocyclen einfach durch F substituiert, 5,2'-Pyridylpyrimidin-2,5'diyl, gegebenenfalls in einem oder beiden der Heterocyclen einfach durch F substituiert
**(XIII)** 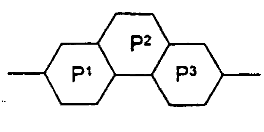 einen bivalenten Phenanthren-2,7-diyl-Rest, bei dem auch ein oder zwei Ring-C-Atome durch N ersetzt sein können und der auch einfach oder zweifach durch F substituiert sein kann und bei dem P² einen (gesättigten) Alicyclus bedeuten kann
   p gleich Null
**(XIV)** 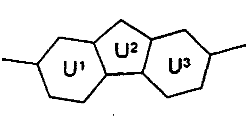 einen bivalenten Fluoren-2,7-diyl-Rest 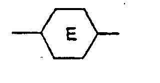 einen Phenylen-1,4-diyl-Rest
   p Null oder 1
**(XV)** 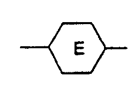 einen bivalenten Rest ausgewählt aus der Gruppe Phenylen-1,4-diyl, gegebenenfalls einfach oder zweifach substituiert durch F, Pyridin-2,5-diyl, gegebenenfalls einfach substituiert durch F, Pyrimidin-2,5-diyl, gegebenenfalls einfach substituiert durch F 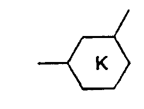 einen bivalenten Rest ausgewählt aus der Gruppe Phenylen-1,3-diyl, gegebenenfalls einfach oder zweifach substituiert durch F
   p Null oder 1
**(XVI)** 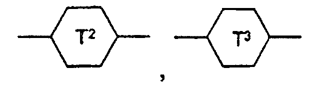 einen bivalenten Rest ausgewählt aus der Gruppe Phenylen-1,4-diyl, gegebenenfalls einfach oder zweifach substituiert durch F, Naphthalin-2,6-diyl, das gegebenenfalls einfach oder zweifach substituiert ist durch F 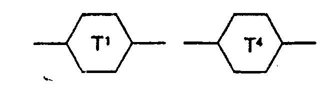 einen bivalenten Rest ausgewählt aus der Gruppe Phenylen-1,4-diyl, gegebenenfalls einfach oder zweifach substituiert durch F, Naphthalin-2-6-diyl, gegebenenfalls einfach oder zweifach substituiert durch F, Cyclohexan-1,4-diyl, Pyridin-2,5-diyl, gegebenenfalls einfach substituiert durch F, Pyrimidin-2,5-diyl, gegebenenfalls einfach substituiert durch F
   r gleich 1
   q, s Null oder 1, in Summe 1
**(XVII)** 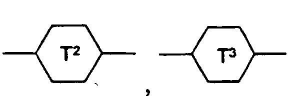 einen bivalenten Rest ausgewählt aus der Gruppe Phenylen-1,4-diyl, gegebenenfalls einfach oder zweifach substituiert durch F, Naphthalin-2,6-diyl, gegebenenfalls einfach oder zweifach substituiert durch F, Cyclohexan-1,4-diyl, Pyridin-2,5-diyl, gegebenenfalls einfach substituiert durch F, Pyrimidin-2,5-diyl, gegebenenfalls einfach substituiert durch F, (1,3,4)Thiadiazol-2,5-diyl, Indan-2,5-diyl 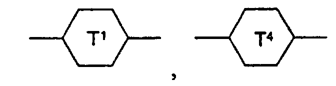 einen bivalenten Rest ausgewählt aus der Gruppe Phenylen-1,4-diyl, gegebenenfalls einfach oder zweifach substituiert durch F, Naphthalin-2-6-diyl, gegebenenfalls einfach oder zweifach substituiert durch F, Cyclohexan-1,4-diyl, Cyclohex-1-en-1,4-diyl, Bicyclo[2.2.2]octan-1,4-diyl, (1,3)-Dioxan-2,5-diyl, Pyridin-2,5-diyl, gegebenenfalls einfach substituiert durch F, Pyrimidin-2,5-diyl, gegebenenfalls einfach substituiert durch F, (1,3,4)Thiadiazol-2,5-diyl, Indan-2,5-diyl, gegebenenfalls im aromatischen Ring einfach oder zweifach substituiert durch F , Thiophen-2,5-diyl
   q, s Null oder 1; in Summe 0 oder 1

Besonders bevorzugt bedeuten in
**(II)** 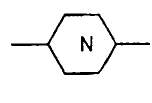 Pyridin-2,5-diyl, 2-Fluor-pyridin-3,6-diyl oder Pyrimidin-2,5-diyl
   Z¹, Z² beide H oder beide F
   R¹⁰, R¹¹ unabhängig voneinander gleich oder verschieden Wasserstoff oder ein geradkettiger oder verzweigter (mit oder ohne asymmetrische C-Atome) Alkyl- oder Alkyloxy-Rest mit 2 - 16 C-Atomen, worin eine oder zwei nicht terminale -CH₂-Gruppen ersetzt sein können durch - CH=CH-, -OC(=O)- , -C(=O)O- und worin auch ein oder mehrere H-Atome durch F ersetzt sein können
   mit der Maßgabe, daß nur einer der Reste R¹⁰, R¹¹ Wasserstoff sein kann.
**(III)** 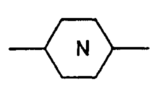 einen bivalenten Rest ausgewählt aus der Gruppe Pyridin-2,5-diyl, 2-Fluor-pyridin-3,6-diyl, Pyrimidin-2,5-diyl 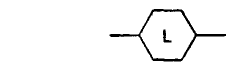 Cyclohexan-1,4-diyl,
   Z¹, Z² beide H oder beide F
   R¹⁰, R¹¹ unabhängig voneinander gleich oder verschieden Wasserstoff oder ein geradkettiger oder verzweigter (mit oder ohne asymmetrische C-Atome) Alkyl- oder Alkyloxy-Rest mit 2 - 16 C-Atomen, worin eine oder zwei nicht terminale -CH₂-Gruppen ersetzt sein können durch -CH=CH-, - OC(=O)- , -C(=O)O- und worin auch ein oder mehrere H-Atome durch F ersetzt sein können
   mit der Maßgabe, daß nur einer der Reste R¹⁰, R¹¹ Wasserstoff sein kann.
**(IV)** 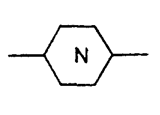 Pyridin-2,5-diyl, 2-Fluor-pyridin-3,6-diyl, Pyrimidin-2,5-diyl
   Z¹, Z², Z³, Z⁴ gleich H
   R¹⁰, R¹¹ unabhängig voneinander gleich oder verschieden Wasserstoff oder ein geradkettiger oder verzweigter (mit oder ohne asymmetrische C-Atome) Alkyl- oder Alkyloxy-Rest mit 2 - 16 C-Atomen, worin eine oder zwei nicht terminale -CH₂-Gruppen ersetzt sein können durch -CH=CH-, -OC(=O)- , -C(=O)O- und worin auch ein oder mehrere H-Atome durch F ersetzt sein können
   mit der Maßgabe, daß nur einer der Reste R¹⁰, R¹¹ Wasserstoff sein kann
(V) 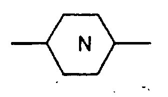 Pyridin-2,5-diyl, 2-Fluor-pyridin-3,6-diyl, Pyrimidin-2,5-diyl Z¹, Z², Z³, Z⁴ gleich H
   R¹⁰, R¹¹ unabhängig voneinander gleich oder verschieden Wasserstoff oder ein geradkettiger oder verzweigter (mit oder ohne asymmetrische C-Atome) Alkyl- oder Alkyloxy-Rest mit 2 - 16 C-Atomen, worin eine oder zwei nicht terminale -CH₂-Gruppen ersetzt sein können durch -CH=CH-, -OC(=O)- , -C(=O)O- und worin auch ein oder mehrere H-Atome durch F ersetzt sein können
   mit der Maßgabe, daß nur einer der Reste R¹⁰, R¹¹ Wasserstoff sein kann
**(VI)**
   Z¹, Z², Z³, Z⁴, Z⁵, Z⁶ ein Element dieser Gruppe ist gleich F oder
   Z¹ und Z² = F, Z³, Z⁴, Z⁵, Z⁶ = H
   Z³ und Z⁴ = F, Z¹, Z², Z⁵, Z⁶ = H
   R¹⁰, R¹¹ unabhängig voneinander gleich oder verschieden Wasserstoff oder ein geradkettiger oder verzweigter (mit oder ohne asymmetrische C-Atome) Alkyloder Alkyloxy-Rest mit 2 - 16 C-Atomen, worin eine oder zwei nicht terminale -CH₂-Gruppen ersetzt sein können durch -CH=CH-, -OC(=O)- , -C(=O)O- und worin auch ein oder mehrere H-Atome durch F ersetzt sein können
   mit der Maßgabe, daß nur einer der Reste R¹⁰, R¹¹ Wasserstoff sein kann.
**(VII)**
   Z¹ und Z² sind beide F ; Z³ und Z⁴ sind beide gleich H
   R¹⁰, R¹¹ unabhängig voneinander gleich oder verschieden Wasserstoff oder ein geradkettiger oder verzweigter (mit oder ohne asymmetrische C-Atome) Alkyl- oder Alkyloxy-Rest mit 2 - 16 C-Atomen, worin eine oder zwei nicht terminale -CH₂-Gruppen ersetzt sein können durch -CH=CH-, -OC(=O)- , -C(=O)O- und worin auch ein oder mehrere H-Atome durch F ersetzt sein können
   mit der Maßgabe, daß nur einer der Reste R¹⁰, R¹¹ Wasserstoff sein kann
**(VIII)** 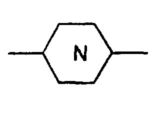 Pyridin-2,5-diyl, Pyrimidin-2,5-diyl 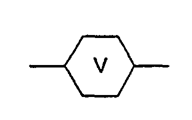 Phenylen-1,4-diyl, gegebenenfalls einfach oder zweifach substituiert durch F,
   p, q, s Null oder 1; in der Summe Null oder 1
   R¹⁰, R¹¹ unabhängig voneinander gleich oder verschieden Wasserstoff oder ein geradkettiger oder verzweigter (mit oder ohne asymmetrische C-Atome) Alkyl- oder Alkyloxy-Rest mit 2 - 16 C-Atomen, worin eine oder zwei nicht terminale -CH₂-Gruppen ersetzt sein können durch -CH=CH-, -OC(=O)- , -C(=O)O- und worin auch ein oder mehrere H-Atome durch F ersetzt sein können
   mit der Maßgabe, daß nur einer der Reste R¹⁰, R¹¹ Wasserstoff sein kann
**(IX)** 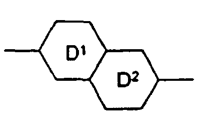 Naphthalin-2,6-diyl oder 1-Fluor-naphthalin-2,6-diyl 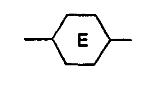 Phenylen-1,4-diyl, gegebenenfalls einfach oder zweifach substituiert durch F, Pyridin-2,5-diyl, 2-Fluor-pyridin-3,6-diyl, Pyrimidin-2,5-diyl
   R¹⁰, R¹¹ unabhängig voneinander gleich oder verschieden Wasserstoff oder ein geradkettiger oder verzweigter (mit oder ohne asymmetrische C-Atome) Alkyl- oder Alkyloxy-Rest mit 2 - 16 C-Atomen, worin eine oder zwei nicht terminale -CH₂-Gruppen ersetzt sein können durch -CH=CH-, -OC(=O)- , -C(=O)O- und worin auch ein oder mehrere H-Atome durch F ersetzt sein können
   mit der Maßgabe, daß nur einer der Reste R¹⁰, R¹¹ Wasserstoff sein kann
**(X)** 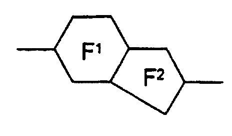 Benzothiazol-2,6-diyl, gegebenenfalls auch Indan-2,5-diyl 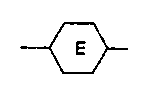 Phenylen-1,4-diyl, Pyridin-2,5-diyl, Pyrimidin-2,5-diyl
   p gleich 1
   q gleich Null
   R¹⁰, R¹¹ unabhängig voneinander gleich oder verschieden Wasserstoff oder ein geradkettiger oder verzweigter (mit oder ohne asymmetrische C-Atome) Alkyl- oder Alkyloxy-Rest mit 2 - 16 C-Atomen, worin eine oder zwei nicht terminale -CH₂-Gruppen ersetzt sein können durch -CH=CH-, -OC(=O)- , -C(=O)O- und worin auch ein oder mehrere H-Atome durch F ersetzt sein können
   mit der Maßgabe, daß nur einer der Reste R¹⁰, R¹¹ Wasserstoff sein kann
**(XI)** 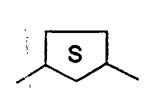 (1,3,4)-Thiadiazol-2,5-diyl 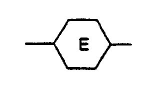 Phenylen-1,4-diyl, Pyridin-2,5-diyl, Cyclohexan-1,4-diyl
   p gleich Null oder 1
   q gleich Null oder 1, mit der Maßgabe, daß q Null bedeutet, wenn p 1 bedeutet
   R¹⁰, R¹¹ unabhängig voneinander gleich oder verschieden Wasserstoff oder ein geradkettiger oder verzweigter (mit oder ohne asymmetrische C-Atome) Alkyl- oder Alkyloxy-Rest mit 2 - 16 C-Atomen, worin eine oder zwei nicht terminale -CH₂-Gruppen ersetzt sein können durch -CH=CH-, -OC(=O)- , -C(=O)O- und worin auch ein oder mehrere H-Atome durch F ersetzt sein können
   mit der Maßgabe, daß nur einer der Reste R¹⁰, R¹¹ Wasserstoff sein kann
**(XII)** 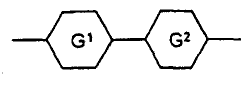 einen bivalenten Rest ausgewählt aus der Gruppe 1,1'-Biphenyl-4,4'-diyl, gegebenenfalls einfach oder zweifach substituiert durch F , 1,1'-Phenylcyclohexyl-4,4'-diyl
   R¹⁰, R¹¹ unabhängig voneinander gleich oder verschieden Wasserstoff oder ein geradkettiger oder verzweigter (mit oder ohne asymmetrische C-Atome) Alkyl- oder Alkyloxy-Rest mit 2 - 16 C-Atomen, worin eine oder zwei nicht terminale -CH₂-Gruppen ersetzt sein können durch -CH=CH-, -OC(=O)- , -C(=O)O- und worin auch ein oder mehrere H-Atome durch F ersetzt sein können
   mit der Maßgabe, daß nur einer der Reste R¹⁰, R¹¹ Wasserstoff sein kann
**(XIII)** 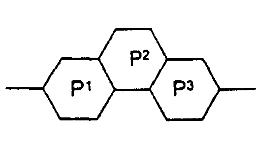 Phenanthren-2,7-diyl, 1-Fluor-phenanthren-2,7-diyl oder 1,8-Difluor-phenanthren-2,7-diyl, bei denen P² auch einen (gesättigten) Alicyclus bedeuten kann.
   R¹⁰, R¹¹ unabhängig voneinander gleich oder verschieden Wasserstoff oder ein geradkettiger oder verzweigter (mit oder ohne asymmetrische C-Atome) Alkyl- oder Alkyloxy-Rest mit 2 - 16 C-Atomen, worin eine oder zwei nicht terminale -CH₂-Gruppen ersetzt sein können durch -CH=CH-, -OC(=O)- , -C(=O)O- und worin auch ein oder mehrere H-Atome durch F ersetzt sein können
   mit der Maßgabe, daß nur einer der Reste R¹⁰, R¹¹ Wasserstoff sein kann
   p gleich Null
**(XIV)** 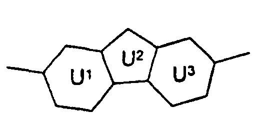 einen bivalenten Fluoren-2,7-diyl-Rest 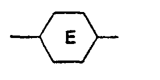 einen Phenylen-1,4-diyl-Rest
   p Null oder 1
   R¹⁰, R¹¹ unabhängig voneinander gleich oder verschieden Wasserstoff oder ein geradkettiger oder verzweigter (mit oder ohne asymmetrische C-Atome) Alkyl- oder Alkyloxy-Rest mit 2 - 16 C-Atomen, worin eine oder zwei nicht terminale -CH₂-Gruppen ersetzt sein können durch -CH=CH-, -OC(=O)- , -C(=O)O- und worin auch ein oder mehrere H-Atome durch F ersetzt sein können
   mit der Maßgabe, daß nur einer der Reste R¹⁰, R¹¹ Wasserstoff sein kann
**(XV)** 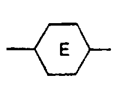 Phenylen-1,4-diyl, Pyridin-2,5-diyl, Pyrimidin-2,5-diyl, 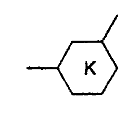 Phenylen-1,3-diyl
   p gleich 1
   R¹⁰, R¹¹ unabhängig voneinander gleich oder verschieden Wasserstoff oder ein geradkettiger oder verzweigter (mit oder ohne asymmetrische C-Atome) Alkyl- oder Alkyloxy-Rest mit 2 - 16 C-Atomen, worin eine oder zwei nicht terminale -CH₂-Gruppen ersetzt sein können durch -CH=CH-, -OC(=O)- , -C(=O)O- und worin auch ein oder mehrere H-Atome durch F ersetzt sein können
   mit der Maßgabe, daß nur einer der Reste R¹⁰, R¹¹ Wasserstoff sein kann
**(XVI)** 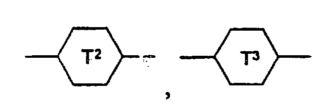 Phenylen-1,4-diyl, gegebenenfalls einfach oder zweifach substituiert durch F, Naphthalin-2,6-diyl, gegebenenfalls einfach oder zweifach substituiert durch F 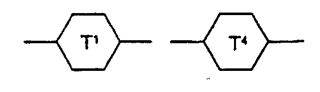 Phenylen-1,4-diyl, gegebenenfalls einfach oder zweifach substituiert durch F, Cyclohexan-1,4-diyl, Pyridin-2,5-diyl, 2-Fluorpyridin-3,6-diyl, Pyrimidin-2,5-diyl
   r gleich 1
   q, s Null oder 1, in Summe 1
   R¹⁰, R¹¹ unabhängig voneinander gleich oder verschieden Wasserstoff oder ein geradkettiger oder verzweigter (mit oder ohne asymmetrische C-Atome) Alkyl- oder Alkyloxy-Rest mit 2 - 16 C-Atomen, worin eine oder zwei nicht terminale - CH₂-Gruppen ersetzt sein können durch -CH=CH-, -OC(=O)- , -C(=O)O- und worin auch ein oder mehrere H-Atome durch F ersetzt sein können
   mit der Maßgabe, daß nur einer der Reste R¹⁰, R¹¹ Wasserstoff sein kann
**(XVII)** 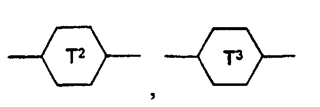 Phenylen-1,4-diyl, gegebenenfalls einfach oder zweifach substituiert durch F, Cyclohexan-1,4-diyl, Pyridin-2,5-diyl, Pyrimidin-2,5-diyl, (1,3,4)-Thiadiazol-2,5-diyl 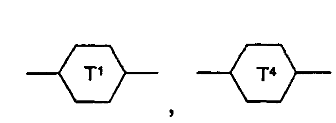 Phenylen-1,4-diyl, gegebenenfalls einfach oder zweifach substituiert durch F, Cyclohexan-1,4-diyl, Pyridin-2,5-diyl, 2-Fluorpyridin-3,6-diyl, Pyrimidin-2,5-diyl, (1,3,4)-Thiadiazol-2,5-diyl
   q, s Null oder 1; in Summe 0 oder 1
   R¹⁰, R¹¹ unabhängig voneinander gleich oder verschieden Wasserstoff oder ein geradkettiger oder verzweigter (mit oder ohne asymmetrische C-Atome) Alkyl- oder Alkyloxy-Rest mit 2 - 16 C-Atomen, worin eine oder zwei nicht terminale - CH₂-Gruppen ersetzt sein können durch -CH=CH-, -OC(=O)- , -C(=O)O- und worin auch ein oder mehrere H-Atome durch F ersetzt sein können
   mit der Maßgabe, daß nur einer der Reste R¹⁰, R¹¹ Wasserstoff sein kann.

Die Flüssigkristallmischung besteht vorzugsweise aus 3-30 Verbindungen und enthält mindestens eine Verbindung der Formel (I) und mindestens eine Verbindung der Formel (II) und gegebenenfalls mindestens eine Verbindung der Formel (III).

Vorzugsweise enthält die Flüssigkristallmischung zusätzlich mindestens eine Verbindung, ausgewählt aus den Gruppen (IV), (V), (VI), (VII).

Besonders bevorzugt enthält die Flüssigkristallmischung zusätzlich mindestens eine Verbindung, ausgewählt aus den Gruppen (VIII), (IX), (XII), (XVI, (XVII). Ebenfalls besonders bevorzugt enthält die Flüssigkristallmischung zusätzlich mindestens eine Verbindung, ausgewählt aus den Gruppen (X), (XI), (XIV), (XV).

Sie kann auch mindestens eine Verbindung der Formel (XIII) enthalten.

Bevorzugt enthält die Mischung ferner mindestens 1 Verbindung ausgewählt aus der Gruppe (I) bis (XVII), bei denen
R¹⁰, R¹¹ unabhängig voneinander gleich oder verschieden sind Wasserstoff oder ein geradkettiger oder verzweigter (mit oder ohne asymmetrische C-Atome) Alkyl- oder Alkyloxy-Rest mit 2 - 16 C-Atomen, worin eine oder zwei nicht terminale -CH₂-Gruppen ersetzt sein können durch -CH=CH-, -OC(=O)- , - C(=O)O- und worin auch ein oder mehrere H-Atome durch F ersetzt sein können mit der Maßgabe, daß nur einer der Reste R¹⁰, R¹¹ Wasserstoff sein kann und worin zusätzlich bei mindestens einem von R¹⁰, R¹¹ die terminale -CH₃-Gruppe durch eine der folgenden chiralen Gruppen (optisch aktiv) ersetzt ist: 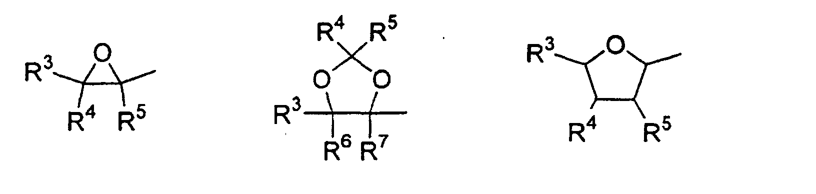 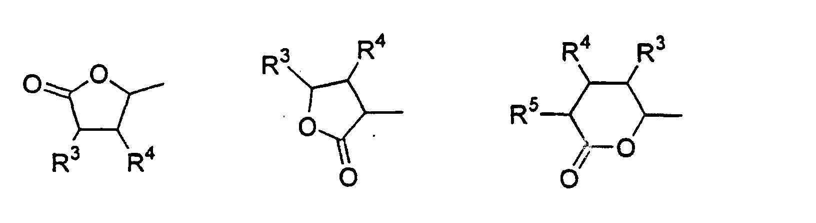 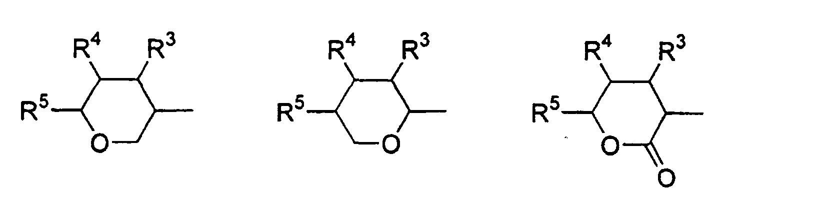 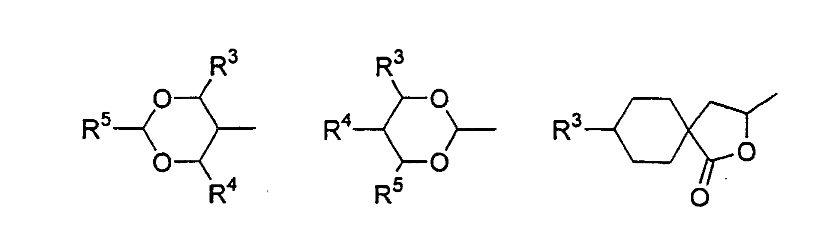 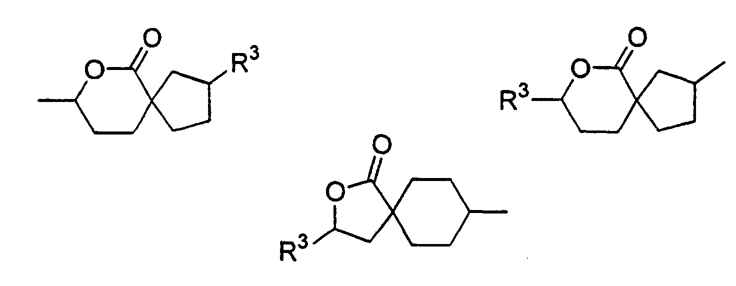 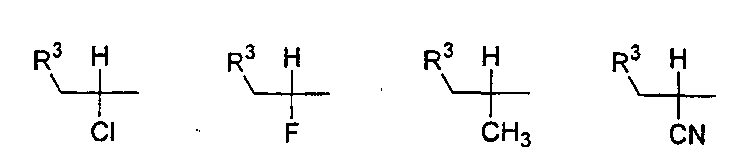 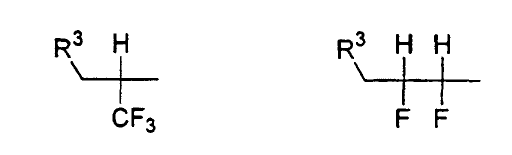 R³, R⁴, R⁵, R⁶, R⁷ sind gleich oder verschieden
a) Wasserstoff
b) ein geradkettiger oder verzweigter Alkylrest (mit oder ohne asymmetrischen Kohlenstoffatomen) mit 1 bis 16 C-Atomen, wobei
   b1) eine oder mehrere nicht benachbarte und nicht terminale CH₂-Gruppen durch -O- ersetzt sein können und/oder
   b2) eine oder zwei CH₂-Gruppen durch -CH=CH- ersetzt sein können,
c) R⁴ und R⁵ zusammen auch -(CH₂)₄- oder -(CH₂)₅-, wenn sie an ein Oxiran-, Dioxolan-, Tetrahydrofuran-, Tetrahydropyran-, Butyrolacton- oder Valerolacton-System gebunden sind;

Besonders bevorzugt enthält die Mischung 1 bis 5 Verbindungen ausgewählt aus der Gruppe (I) bis (XVII), bei denen
R¹⁰, R¹¹ unabhängig voneinander gleich oder verschieden sind Wasserstoff oder ein geradkettiger oder verzweigter (mit oder ohne asymmetrische C-Atome) Alkyl- oder Alkyloxy-Rest mit 2 - 16 C-Atomen, worin eine oder zwei nicht terminale -CH₂-Gruppen ersetzt sein können durch -CH=CH-, -OC(=O)- , -C(=O)O- und worin auch ein oder mehrere H-Atome durch F ersetzt sein können mit der Maßgabe, daß nur einer der Reste R¹, R² Wasserstoff sein kann und worin zusätzlich bei mindestens einem von R¹⁰, R¹¹ die terminale -CH₃-Gruppe durch eine der folgenden chiralen Gruppen (optisch aktiv) ersetzt ist : 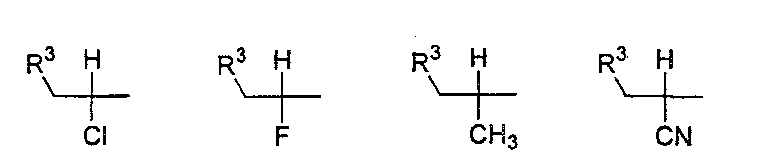 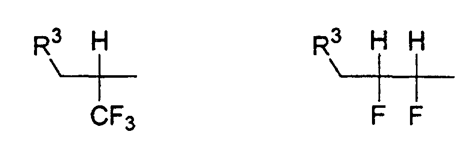
- R³: ist Wasserstoff oder ein geradkettiger oder verzweigter Alkylrest (mit oder ohne asymmetrischen Kohlenstoffatomen) mit 1 bis 16 C-Atomen.

Bevorzugt enthält die erfindungsgemäße Flüssigkristallmischung 3 bis 25 Komponenten, darunter
1 bis 15 Verbindungen der Formel (I)
1 bis 15 Verbindungen der Formel (II)
1 bis 7 Verbindungen der Formel (III).

Ebenfalls bevorzugt enthält die erfindungsgemäße Flüssigkristallmischung 3 bis 25 Komponenten, darunter
1 bis 15 Verbindungen der Formel (I)
1 bis 15 Verbindungen der Formel (II)
1 bis 7 Verbindungen der Formel (III)
1 bis 7 Verbindungen der Formel (IV).

Ebenfalls bevorzugt enthält die erfindungsgemäße Flüssigkristallmischung 3 bis 25 Komponenten, darunter
1 bis 15 Verbindungen der Formel (I)
1 bis 15 Verbindungen der Formel (II)
1 bis 7 Verbindungen der Formel (III)
1 bis 7 Verbindungen der Formel (IV)
1 bis 7 Verbindungen der Formel (V).

Ebenfalls bevorzugt enthält die erfindungsgemäße Flüssigkristallmischung 3 bis 25 Komponenten, darunter
1 bis 15 Verbindungen der Formel (I)
1 bis 15 Verbindungen der Formel (II)
1 bis 7 Verbindungen der Formel (III)
1 bis 7 Verbindungen der Formel (IV)
1 bis 7 Verbindungen der Formel (V)
1 bis 7 Verbindungen der Formel (VI).

Ebenfalls bevorzugt enthält die erfindungsgemäße Flüssigkristallmischung 3 bis 25 Komponenten, darunter
1 bis 15 Verbindungen der Formel (I)
1 bis 15 Verbindungen der Formel (II)
1 bis 7 Verbindungen der Formel (III)
1 bis 7 Verbindungen der Formel (IV)
1 bis 7 Verbindungen der Formel (VI)
1 bis 7 Verbindungen der Formel (VII).

Ebenfalls bevorzugt enthält die erfindungsgemäße Flüssigkristallmischung 3 bis 25 Komponenten, darunter
1 bis 15 Verbindungen der Formel (I)
1 bis 15 Verbindungen der Formel (II)
1 bis 7 Verbindungen der Formel (III)
1 bis 7 Verbindungen der Formel (IV)
1 bis 7 Verbindungen der Formel (VI).

Ebenfalls bevorzugt enthält die erfindungsgemäße Flüssigkristallmischung 3 bis 25 Komponenten , darunter
1 bis 15 Verbindungen der Formel (I)
1 bis 15 Verbindungen der Formel (II)
1 bis 7 Verbindungen der Formel (III)
1 bis 7 Verbindungen der Formel (IV)
1 bis 7 Verbindungen der Formel (VI)
1 bis 7 Verbindungen der Formel (XII).

Ebenfalls bevorzugt enthält die erfindungsgemäße Flüssigkristallmischung 3 bis 25 Komponenten, darunter
1 bis 15 Verbindungen der Formel (I)
1 bis 15 Verbindungen der Formel (II)
1 bis 7 Verbindungen der Formel (III)
1 bis 7 Verbindungen der Formel (IV)
1 bis 7 Verbindungen der Formel (V)
1 bis 7 Verbindungen der Formel (VI)
1 bis 7 Verbindungen der Formel (VII).

Ebenfalls bevorzugt enthält die erfindungsgemäße Flüssigkristallmischung 3 bis 25 Komponenten, darunter
1 bis 15 Verbindungen der Formel (I)
1 bis 15 Verbindungen der Formel (II)
1 bis 7 Verbindungen der Formel (IV).

Ebenfalls bevorzugt enthält die erfindungsgemäße Flüssigkristallmischung 3 bis 25 Komponenten, darunter
1 bis 15 Verbindungen der Formel (I)
1 bis 15 Verbindungen der Formel (II)
1 bis 7 Verbindungen der Formel (IV)
1 bis 7 Verbindungen der Formel (VI).

Ebenfalls bevorzugt enthält die erfindungsgemäße Flüssigkristallmischung 3 bis 25 Komponenten, darunter
1 bis 15 Verbindungen der Formel (I)
1 bis 15 Verbindungen der Formel (II)
1 bis 7 Verbindungen der Formel (IV)
1 bis 7 Verbindungen der Formel (XII).

Ebenfalls bevorzugt enthält die erfindungsgemäße Flüssigkristallmischung 3 bis 25 Komponenten, darunter
1 bis 15 Verbindungen der Formel (I)
1 bis 15 Verbindungen der Formel (II)
1 bis 7 Verbindungen der Formel (IV)
1 bis 7 Verbindungen der Formel (IX).

Besonders bevorzugt enthält die erfindungsgemäße Flüssigkristallmischung 3 bis 25 Komponenten, darunter
1 bis 12 Verbindungen der Formel (I)
2 bis 12 Verbindungen der Formel (II)
1 bis 5 Verbindungen der Formel (III).

Ebenfalls besonders bevorzugt enthält die erfindungsgemäße Flüssigkristallmischung 3 bis 25 Komponenten, darunter
1 bis 12 Verbindungen der Formel (I)
2 bis 12 Verbindungen der Formel (II)
1 bis 5 Verbindungen der Formel (III)
1 bis 5 Verbindungen der Formel (IV)
1 bis 5 Verbindungen der Formel (VI)
1 bis 5 Verbindungen der Formel (VII).

Ebenfalls besonders bevorzugt enthält die erfindungsgemäße Flüssigkristallmischung 3 bis 25 Komponenten, darunter
1 bis 12 Verbindungen der Formel (I)
2 bis 12 Verbindungen der Formel (II)
1 bis 5 Verbindungen der Formel (III)
1 bis 5 Verbindungen der Formel (IV)
1 bis 5 Verbindungen der Formel (VI).

Ebenfalls besonders bevorzugt enthält die erfindungsgemäße Flüssigkristallmischung 3 bis 25 Komponenten, darunter
1 bis 12 Verbindungen der Formel (I)
2 bis 12 Verbindungen der Formel (II)
1 bis 5 Verbindungen der Formel (III)
1 bis 5 Verbindungen der Formel (IV)
1 bis 5 Verbindungen der Formel (VI)
1 bis 5 Verbindungen der Formel (XII).

Ebenfalls besonders bevorzugt enthält die erfindungsgemäße Flüssigkristallmischung 3 bis 25 Komponenten, darunter
1 bis 12 Verbindungen der Formel (I)
2 bis 12 Verbindungen der Formel (II)
1 bis 5 Verbindungen der Formel (IV).

Ebenfalls besonders bevorzugt enthält die erfindungsgemäße Flüssigkristallmischung 3 bis 25 Komponenten, darunter
1 bis 12 Verbindungen der Formel (I)
2 bis 12 Verbindungen der Formel (II)
1 bis 5 Verbindungen der Formel (IV)
1 bis 5 Verbindungen der Formel (VI).

Ebenfalls besonders bevorzugt enthält die erfindungsgemäße Flüssigkristallmischung 3 bis 25 Komponenten, darunter
1 bis 12 Verbindungen der Formel (I)
2 bis 12 Verbindungen der Formel (II)
1 bis 5 Verbindungen der Formel (IV)
1 bis 5 Verbindungen der Formel (VI)
1 bis 5 Verbindungen der Formel (VII).

Ganz besonders bevorzugt enthält die erfindungsgemäße Flüssigkristallmischung 3 bis 23 Komponenten, darunter,
1 bis 8 Verbindungen der Formel (I)
2 bis 10 Verbindungen der Formel (II)
1 bis 3 Verbindungen der Formel (III).

Ebenfalls ganz besonders bevorzugt enthält die erfindungsgemäße Flüssigkristallmischung 3 bis 23 Komponenten, darunter
1 bis 8 Verbindungen der Formel (I)
2 bis 10 Verbindungen der Formel (II), worin in mindestens 1 Verbindung eine -CH₂-Gruppe ersetzt ist durch -OC(=O)-
1 bis 3 Verbindungen der Formel (III).

Ebenfalls ganz besonders bevorzugt enthält die erfindungsgemäße Mischung gemäß einer Ausführungsform der Erfindung 3 bis 30 Komponenten, darunter
4 bis 8 Verbindungen der Formel (I)
1 bis 10 Verbindungen der Formel (II)
1 bis 4 Verbindungen der Formel (VI)
1 bis 4 Verbindungen der Formel (X)
1 bis 4 Verbindungen der Formel (XI).

In einer speziellen Ausführungsform dieser ganz besonders bevorzugten Mischung enthält die Mischung mindestens eine Verbindung der Formel (Ia), mindestens eine der Formel (Ib), mindestens 3 der Formel (II) sowie jeweils mindestens eine der Formeln (VI), (X) und (XI).

In einer im höchsten Maße bevorzugten Ausführungsform entfallen auf die mindestens je eine Verbindung der Formel (Ia) bzw. (Ib) mindestens 1 Verbindung der Formel (Ialh) und mindestens 1 Verbindung der Formel (Ia1v) sowie mindestens 1 Verbindung der Formel (Ibla), wobei in (II) 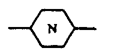 Pyrimidin-2,5-diyl, in (VI) Z¹ und Z² F, in (X) 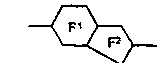 Benzothiazol-2,6-diyl und in (XI) 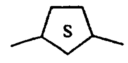 Thiazol-2,5-diyl bedeuten.

Ebenfalls ganz besonders bevorzugt enthält die erfindungsgemäße Mischung gemäß einer Ausführungsform der Erfindung 3 bis 30 Komponenten, darunter
4 bis 8 Verbindungen der Formel (I)
1 bis 10 Verbindungen der Formel (II)
1 bis 4 Verbindungen der Formel (IV)
1 bis 4 Verbindungen der Formel (VI)
1 bis 4 Verbindungen der Formel (X)
1 bis 4 Verbindungen der Formel (XI).

In einer speziellen Ausführungsform dieser ganz besonders bevorzugten Mischung enthält die Mischung mindestens eine Verbindung der Formel (Ia), mindestens eine Verbindung der Formel (Ib), mindestens drei Verbindungen der Formel (II) sowie jeweils mindestens eine der Formeln (IV), (VI), (X) und (XI).

In einer im höchsten Maße bevorzugten Ausführungsform entfallen auf die mindestens je eine Verbindung der Formel (Ia) bzw. (Ib) mindestens 1 Verbindung der Formel (Ia1h) und mindestens 1 Verbindung der Formel (Ia1v) optional noch mindestens eine Verbindung der Formel (Ia1n) sowie mindestens 1 Verbindung der Formel (Ib1a), wobei in (II) 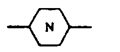 Pyrimidin-2,5-diyl, in (IV) 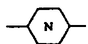 Pyrimidin-2,5-diyl, Pyridin-2,5-diyl oder 2-Fluor-pyridin-3,6-diyl, in (VI) Z¹ und Z² F, in (X) 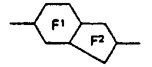 Benzothiazol-2,6-diyl und in (XI) 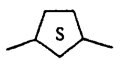 Thiazol-2,5-diyl bedeutet.

In einer speziellen Ausführungsform der ganz besonders bevorzugten Flüssigkristallmischung bedeuten in
**(II)** 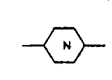 Pyrimidin-2,5-diyl,
   - Z¹, Z²: beide H oder beide F,
   - R¹⁰: einen geradkettigen oder verzweigten Alkyl- oder Alkyloxy-Rest von 6 bis 14 C-Atomen, worin auch eine oder zwei -CH₂-Gruppen ersetzt sein können durch -O- und/oder -C(=O)-,
   - R¹¹: einen geradkettigen oder verzweigten Alkyl- oder Alkyloxy-Rest von 6 bis 14 C-Atomen, worin auch eine oder zwei -CH₂-Gruppen ersetzt sein können durch -O- und/oder -C(=O)-,
**(III)** 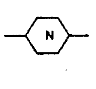 2-Fluor-pyridin-3,6-diyl 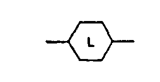 Cyclohexan-1,4-diyl
   - R¹⁰: einen geradkettigen oder verzweigten Alkyl- oder Alkyloxy-Rest von 6 bis 14 C-Atomen, worin auch eine oder zwei -CH₂-Gruppen ersetzt sein können durch -O- und/oder -C(=O)- und worin auch ein H-Atom ersetzt sein kann durch F
   - R¹²: Wasserstoff oder einen geradkettigen oder verzweigten Alkyl- oder Alkyloxy- Rest von 6 bis 14 C-Atomen, worin auch eine oder zwei -CH₂-Gruppen ersetzt sein können durch -O- und/oder -C(=O)-

In einer ganz speziellen Ausführungsform der ganz besonders bevorzugten Flüssigkristallmischung bedeuten
**(II)** 5-Alkyl-2-(4-alkyloxyphenyl)pyrimidin, 5-Alkyl-2-(4-alkylcarbonyloxyphenyl)pyrimidin, 5- Alkylcarbonyloxy-2-(4-alkyloxyphenyl)pyrimidin oder 5-Alkyl-2-(4-alkyloxy-2,3-difluorphenyl)pyrimidin
   und in
**(III)** R¹⁰ einen geradkettigen Alkyloxy-Rest von 6 bis 14 C-Atomen, worin ein H-Atom durch F ersetzt ist
   R¹² Wasserstoff

Vorzugsweise enthält die chiral-smektische Flüssigkristallmischung 10-60 % einer oder mehrerer Verbindungen der Formel (I). Besonders bevorzugt enthält die Mischung 10-60 % von 1-15 Verbindungen der Formel (1). Besonders bevorzugt enthält die Mischung 10-60 % von 1-15 Verbindungen der Formel (I) und 40-90 % von 2-15 Verbindungen der Formel (II). Insbesondere enthält sie 10-60 % von 1-15 Verbindungen der Formel (I), 40-90 % von 2-15 Verbindungen der Formel (II) und 1-40 % von 1-15 Verbindungen aus der Gruppe (III), (IV), (V), (VI) und (VII), wobei die Gesamtmenge 100 % ergibt. Die Prozentangaben sind Gew.-%.

Die Erfindung betrifft auch Verbindungen der allgemeinen Formel (I), ausgewählt aus

Verbindungen der Formel (XXIII), worin bedeuten:

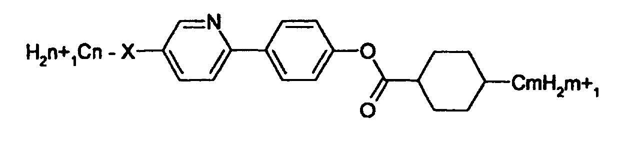

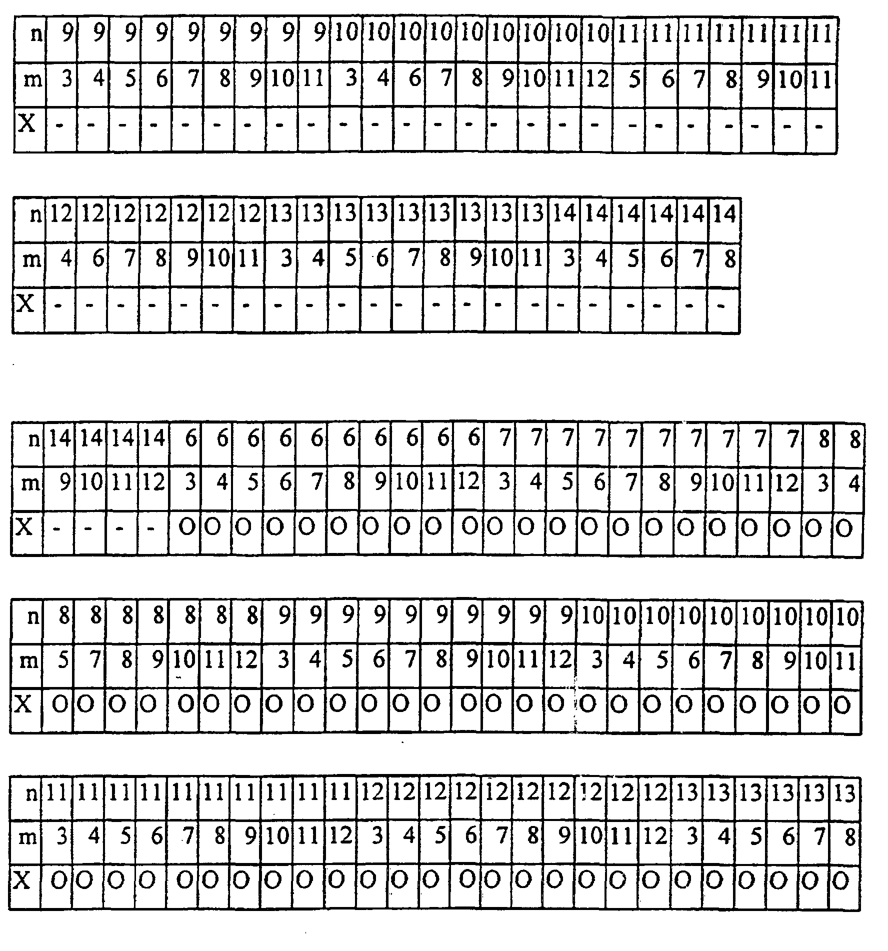

Verbindungen der Formel (XXIV), worin bedeuten:

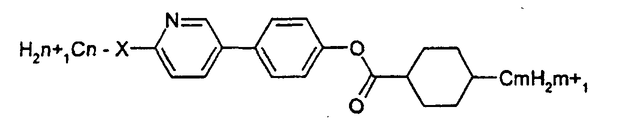

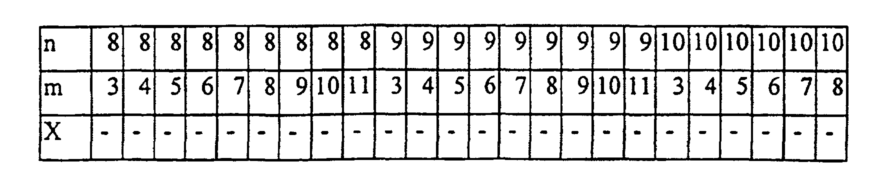

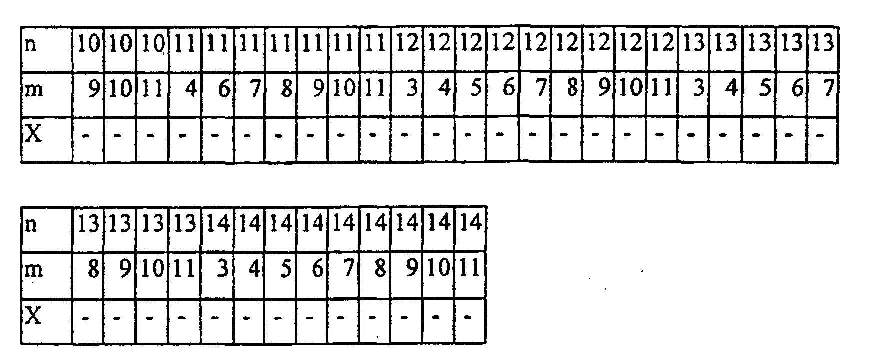

Verbindungen der Formel (XXV), worin bedeuten:

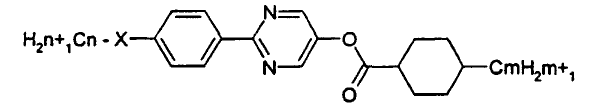

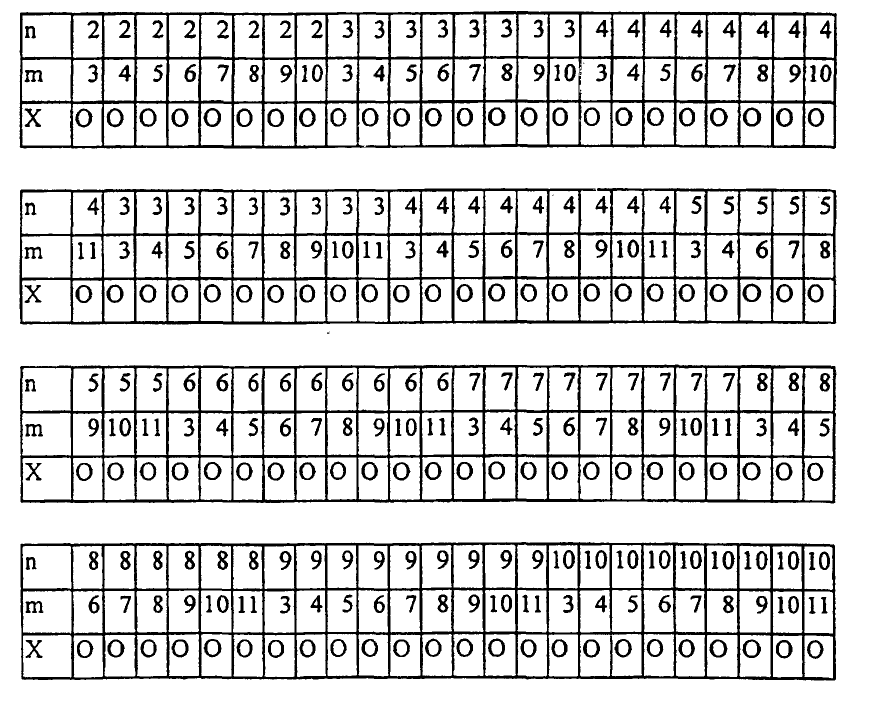

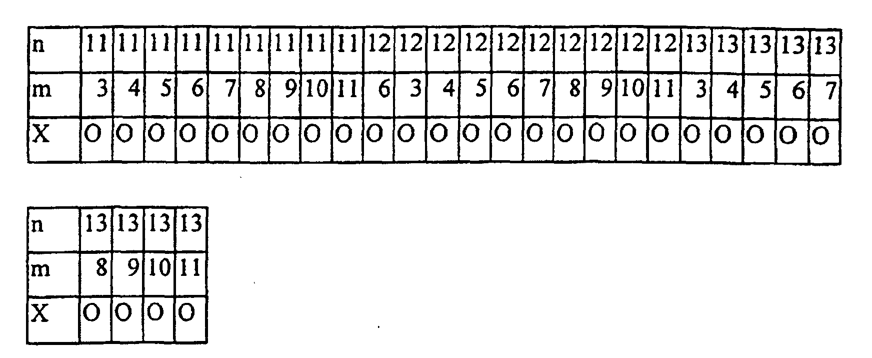

oder gegebenenfalls

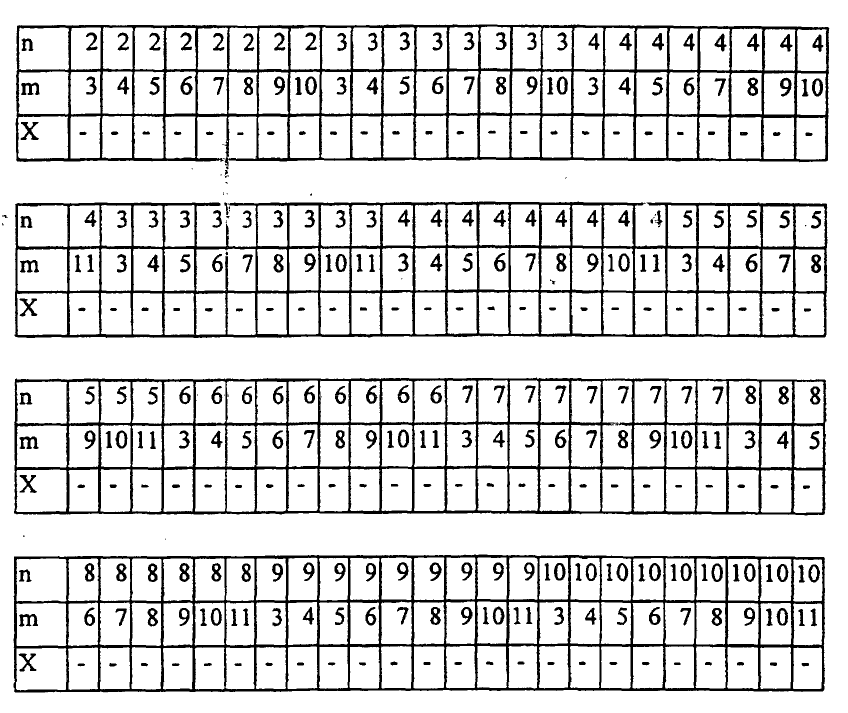

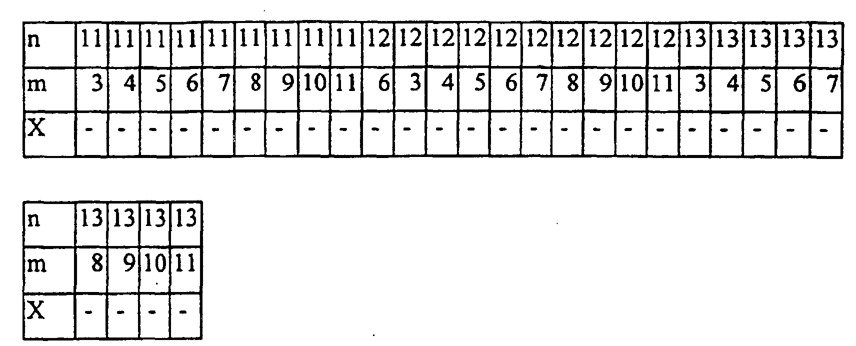

Verbindungen der Formel (XXVI), worin bedeuten:

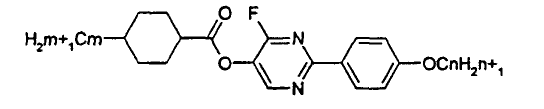

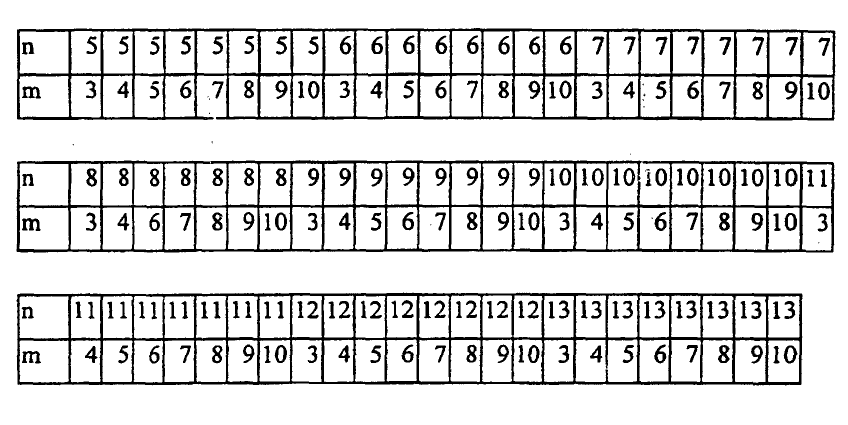

Verbindungen der Formel (XXVII), worin bedeuten:

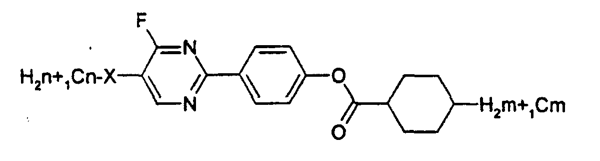

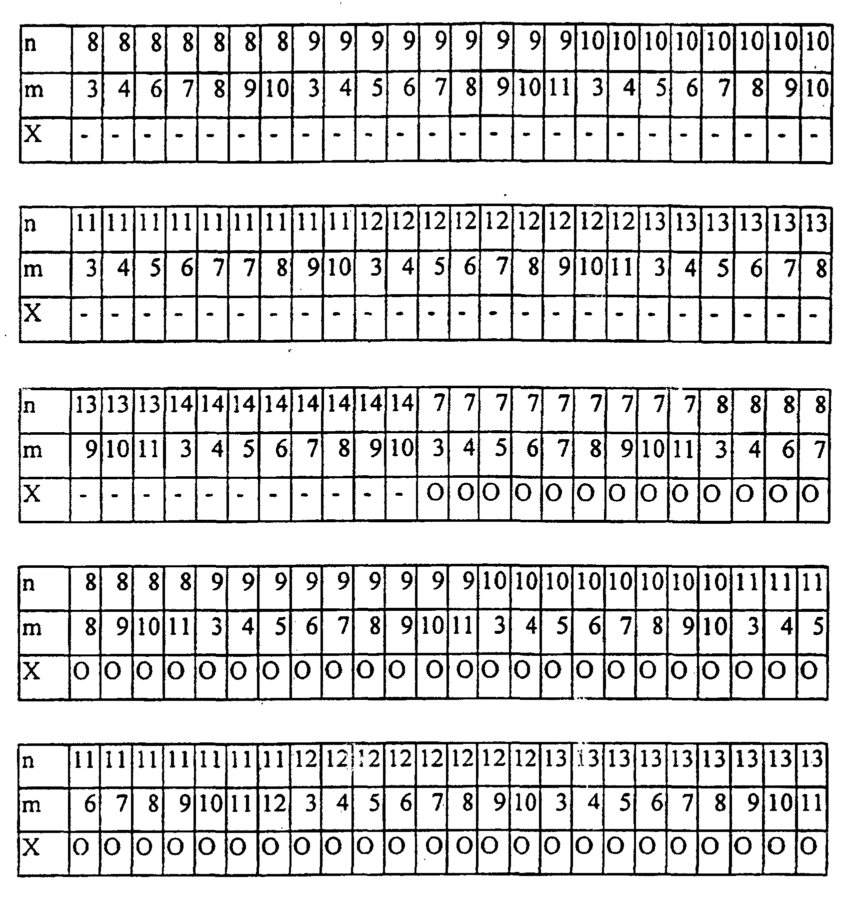

Verbindungen der Formel (XXIX), worin bedeuten:

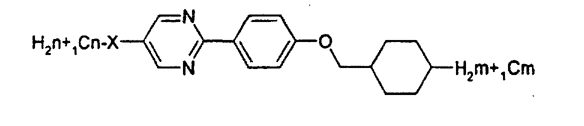

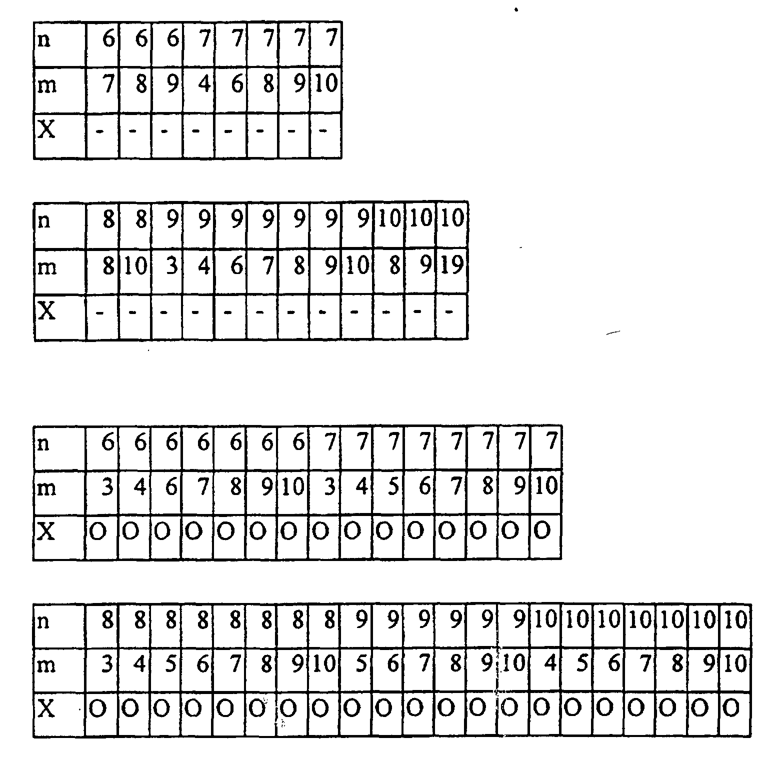

Verbindungen der Formel (XXX), worin bedeuten:

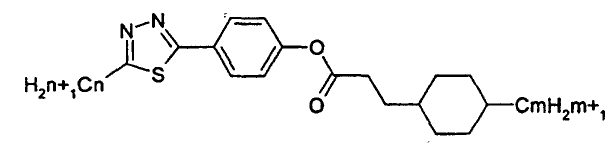

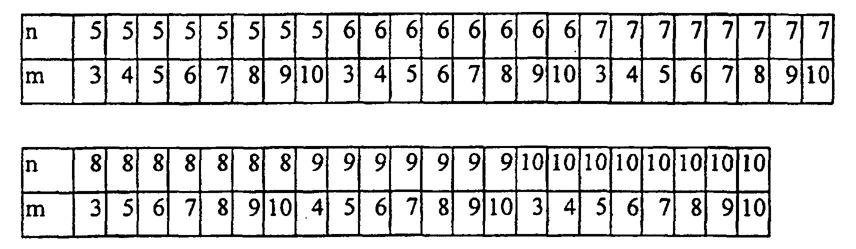

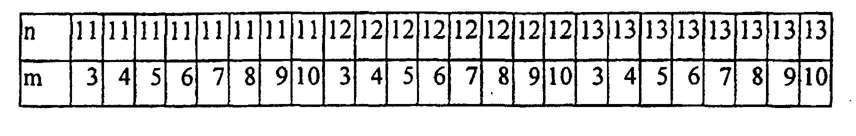

Die Erfindung betrifft auch Verbindungen der allgemeinen Formel (II), ausgewählt aus Verbindungen der Formel (XXXI), worin bedeuten:

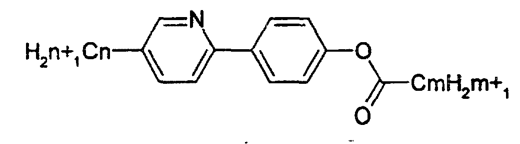

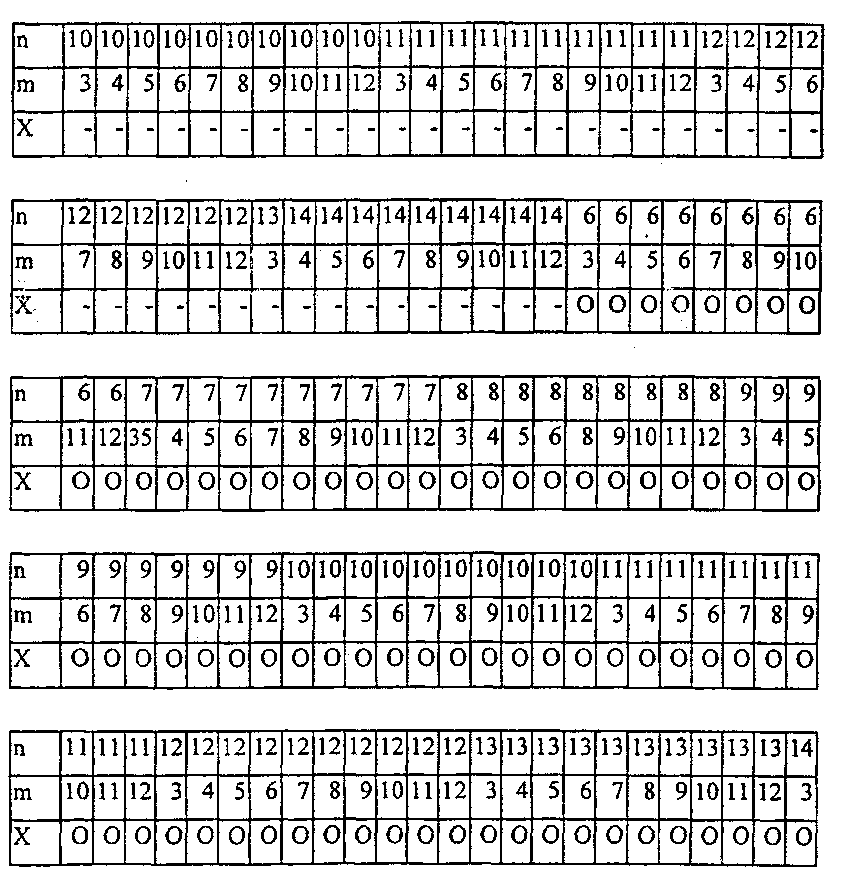

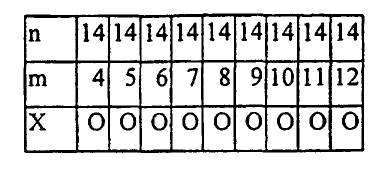

Verbindungen der Formel (XXVIII), worin bedeuten:

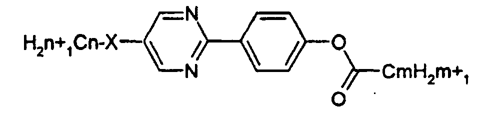

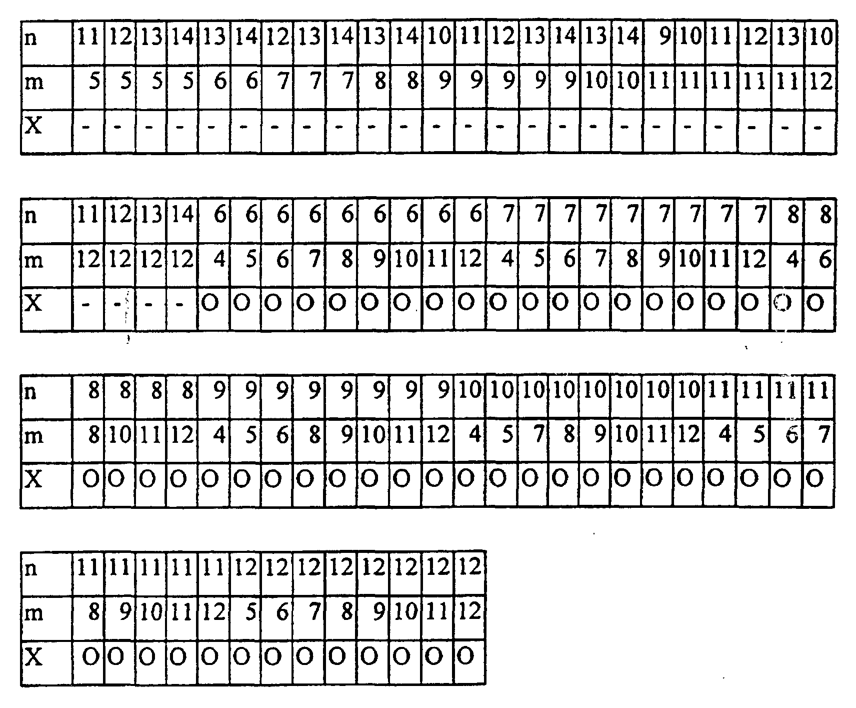

Gegebenenfalls Verbindungen der Formel (XXXII), worin bedeuten:

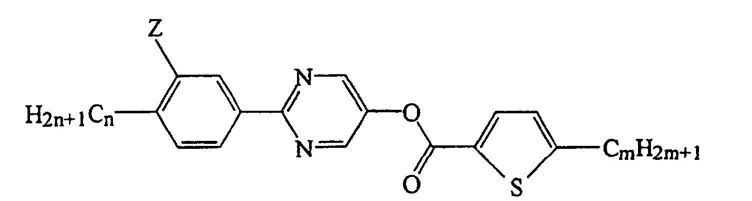

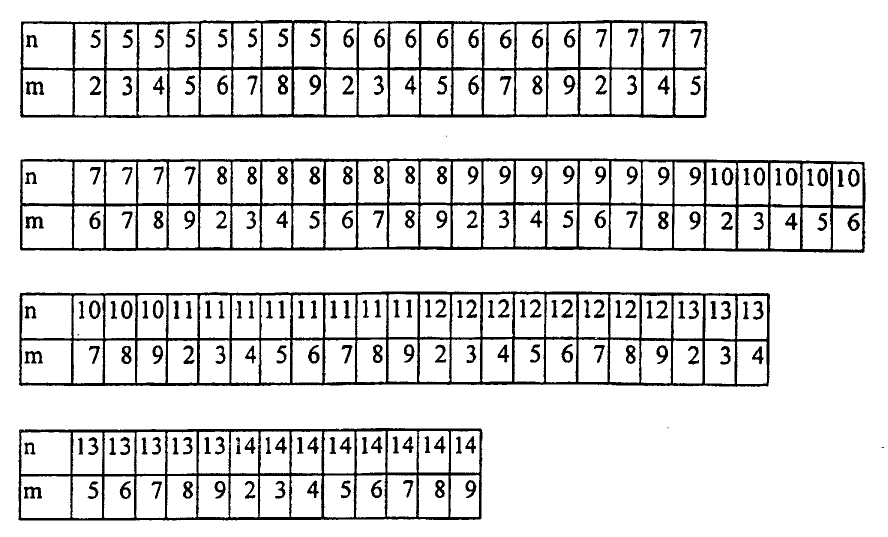

und Z in allen Fällen H oder F bedeutet.

Allgemein sind Thiophencarbonsäureester, bei denen der Heterocyklus nicht fluoriert sein kann, in der EP-A-0 364 923 beschrieben. In EP-A-0 459 406 sind Thiophencarbonsäureester beschrieben, in denen die Phenylgruppe durch Fluor substituiert sein muß. In EP-A-0 392 510 muß die Phenylengruppe 2,3-Cyanosubstituiert sein.

Allgemein sind Tetrahydrochinazoline in US 4,402,849 beschrieben. Ein Beispiel für derartige Verbindungen findet sich in JP-A-08059629, wie auch in JP-A-08062559 und JP-A-07207267.

Die Erfindung wird durch die nachstehenden Beispiele näher erläutert. In den Beispielen 1-15 werden erfindungsgemäße Mischungen angegeben.

### Beispiel 1

Eine LCD Testzelle wird hergestellt aus zwei handelsüblichen, mit Indium-Zinnoxid leitfähig transparent beschichteten Glasplatten. Diese werden mit der Orientierungsschicht LQT-120 (Hersteller: Hitachi Chemicals KK), diese mit N-Methylpyrrolidon auf 8.3% ihres ursprünglichen Feststoffgehaltes verdünnt, durch Spin-coating beschichtet (2500 U/min, 10 sec), durch Erhitzen gehärtet (230°C, 1 Stunde) und anschließend einem Reibeprozeß zwecks Orientierung unterzogen (Reibestoff: Rayon-Typ YA-20-R*, clearance 0.2 mm, 1 mal, 700 U/min Walzendrehzahl, 10 cm/s Substratgeschwindigkeit, 10 cm Rollendurchmesser).
Die geriebenen Gläser werden bei paralleler Ausrichtung der Reiberichtung zu Testzellen verklebt und mittels Abstandhalter auf einen Abstand von 1,3 µm eingestellt.
Eine Mischung bestehend aus

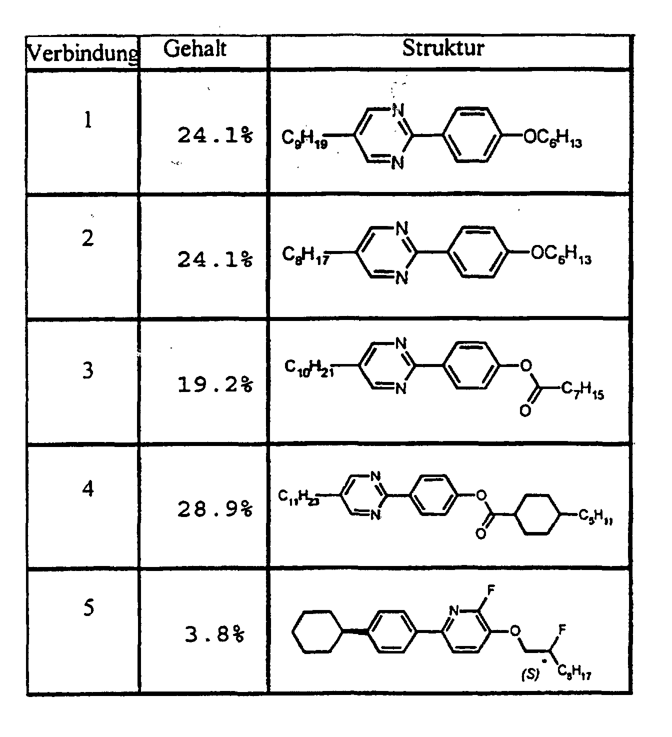

mit den Phasenübergängen I / N* 81.6-85.9 und N* / Sc* 59.3°C wird in die Zelle gefüllt und durch Abkühlen zunächst in der nematischen bzw. cholesterischen Phase orientiert. Beim weiteren Abkühlen wird eine Gleichspannung von 3 Volt angelegt und die Zelle im Bereich von 61.3°C bis 57.3°C mit einer Abkühlrate von 2 K/min in den Bereich der Sc* -Phase (chiral smektisch C) überführt. Dabei bildet sich eine monostabile Monodomäne aus. Diese ist gekennzeichnet durch eine gewisse Temperaturabhängigkeit des Tiltwinkels, die durch Untersuchungen im Polarisationsmikroskop beurteilt wurde.

Die Ergebnisse werden angegeben durch den Wert DT(T1, 1), was bedeutet, daß ausgehend von einer unteren Temperatur T1 im gesamten Bereich von T1 bis (T1+DT) der Tiltwinkel sich weniger als 1 ° verändert. Z.B. bedeutet DT(15,1)=22, daß im Bereich 15 °C bis 37 °C sich der Tiltwinkel um maximal 1 ° verändert.

Die Werte für DT sollen generell möglichst groß sein, um einen breiten Arbeitstemperaturbereich ohne größere Abweichung des Direktors zu ermöglichen. Angaben von-DT erfolgen stets in Grad Celsius.

Für die nachstehenden Beispiele und Vergleichsbeispiele wird die oben beschriebene Orientierung so durchgeführt, daß die Spannung von 3 Volt im Temperaturbereich von +- 2 °C am N/Sc* Phasenübergang angelegt wird.

Die Mischung gem. Beispiel 1 weist die folgenden Werte auf : DT(15,1) / DT (20,1) / DT (25,1) / DT (30,1) : 25 / 21 / 18 / 16 und somit, wie auch die folgenden Beispiele belegen, einen breiten Bereich der Arbeitstemperatur.

### Beispiel 2

Eine Mischung aus 19,28 % der Verbindung 1, 19,28 % der Verbindung 2, 15,36% der Verbindung 3, 23,12% der Verbindung 4 und 3,04 % der Verbindung 5 aus Beispiel 1 sowie 20% der Verbindung 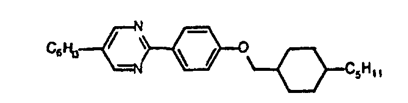 hat die Phasenübergänge I / N* 97.7-92.8 und N* / Sc* 58.9 ° C und die Werte DT(15,1) / DT (20,1) / DT (25,1) / DT (30,1): 30/27/25/21.

### Beispiel 3

Eine Mischung nachstehender Zusammensetzung weist die Phasenübergänge I /N* 78.9 - 74.4 und N* / Sc* 57.3 °C auf sowie die Werte DT(10,1) / DT (15,1) /DT (30,1): 22,5/20/17,5.

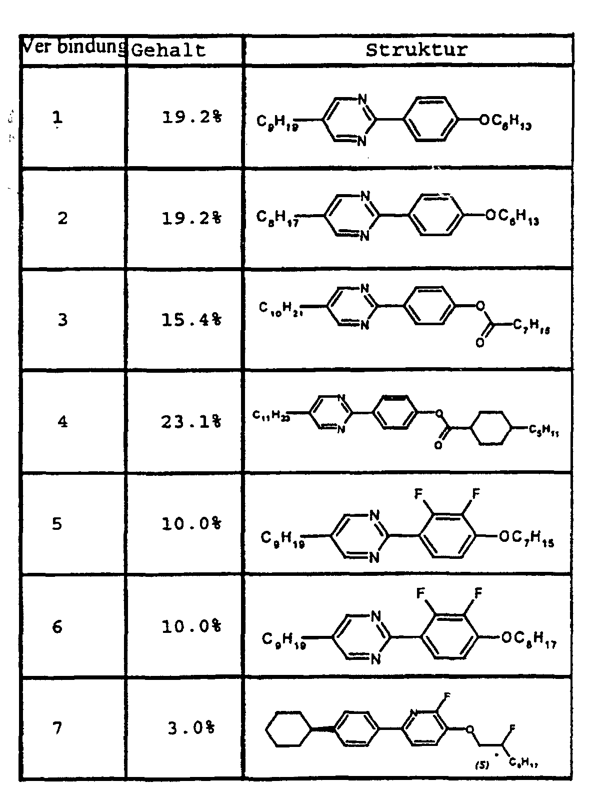

### Beispiel 4

Eine Mischung aus 16,32 % der Verbindung 1, 16,32% der Verbindung 2, 18,1% der Verbindung 3, 19,6% der Verbindung 4, 8,5% der Verbindung 5, 8,5 % der Verbindung 6 , 2,55% der Verbindung 7 aus Beispiel 3 und 15% der Verbindung 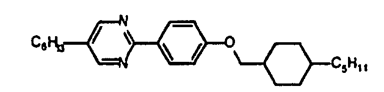 hat die Phasenübergänge I / N* 92.2-87.8 und N* / Sc* 57.7 ° C und die Werte : DT(10,1)/DT(15,1)/DT(30,1): 27,5/23,8/18.

### Beispiel 5

Eine Mischung aus

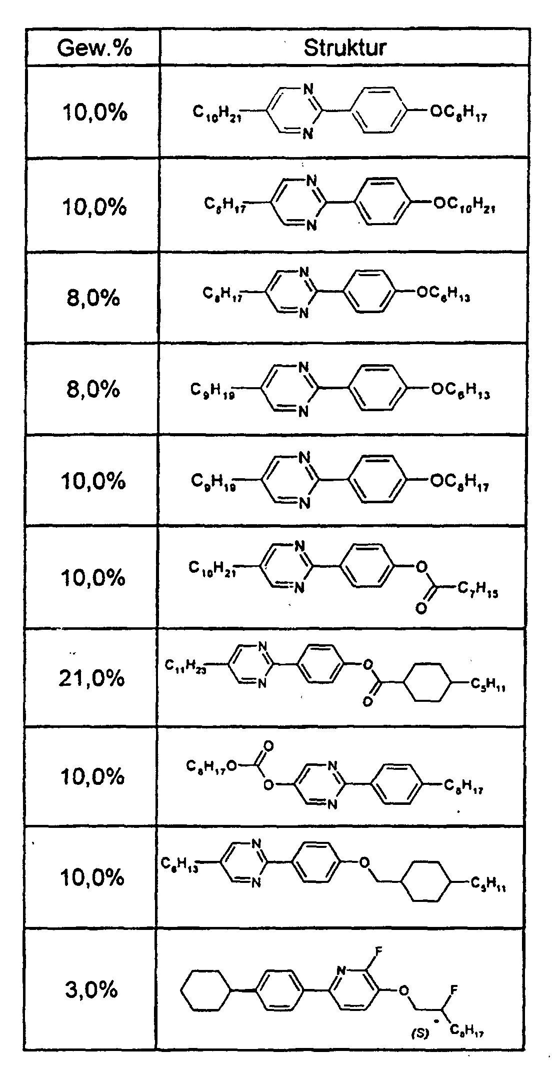

besitzt die Phasenübergänge I / N* 90.0 - 87.2 und N* / S_{C}* 65.1 °C sowie die Werte DT(15,1) / DT (20,1) / DT (25,1) / DT (30,1): 30 / 27 / 25 / 25.

### Beispiel 6

Eine Mischung aus 85% der Mischung aus Beispiel 5 und 15% der Verbindung 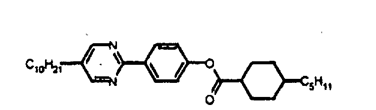 hat die Phasenübergänge I / N* 94.9 - 92.2 und N* / Sc* 65.7 °C und die Werte DT(15,1) / DT (20,1) / DT (25,1) / DT (30,1) 33,8 / 30 / 27,5 / 26,3 .

### Beispiel 7

Eine Mischung aus 85% der Mischung aus Beispiel 5 und 15% der Verbindung 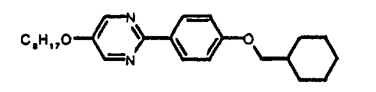 hat die Phasenübergänge I / N* 89.7 - 87.5 und N* / Sc* 66.3 °C und die Werte DT(15,1)/DT(20,1)/DT(25,1)/DT(30,1) 27,5/25/22,5/20.

### Beispiel 8

Eine Mischung aus 85% der Mischung aus Beispiel 5 und 15% der Verbindung 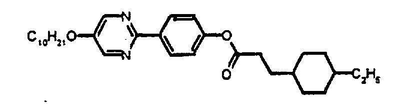 hat die Phasenübergänge I / N* 93.9 - 91.1 und N* / Sc* 67.6 °C und die Werte DT(15,1) / DT (20,1) / DT (25,1) / DT (30,1) 30 / 27,5 / 25 / 25.

### Beispiel 9

Eine Mischung aus 85% der Mischung aus Beispiel 5 und 15% der Verbindung 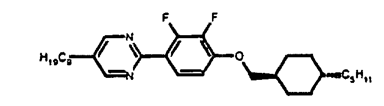 hat die Phasenübergänge I / N* 92.1 - 89.6 und N* / Sc* 63.1 °C und die Werte DT(15,1) / DT (20,1) / DT (25,1) / DT (30,1) 26,3/23,8/22,5/20.

### Beispiel 11

Eine Mischung aus 85% der Mischung aus Beispiel 5 und 15% der Verbindung 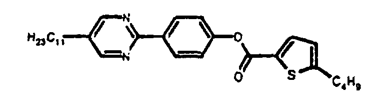 hat die Phasenübergänge I / N* 89.1 - 86.7 und N* / Sc* 61.4 °C und die Werte DT(15,1)/DT(20,1)/DT(25,1)/DT(30,1) 27,5/26,3/22,%/21,3.

### Beispiel 12

Eine Mischung aus 85% der Mischung aus Beispiel 5 und 15% der Verbindung 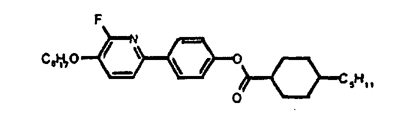 hat die Phasenübergänge I / N* 98.0 - 94.2 und N* / Sc* 71.7 °C sowie die Werte: DT(15,1)/DT(20,1)/DT(25,1)/DT(30,1) 32,5/31,3/32,5/30.

### Beispiel 13

Eine Mischung aus 85% der Mischung aus Beispiel 5 und 15% der Verbindung 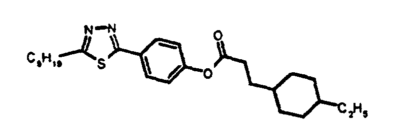 hat die Phasenübergänge I / N* 89.5 - 87.2 und N* / Sc* 69.7 °C sowie die Werte DT(15,1) / DT (20,1) / DT(25,1) / DT (30,1) 42,5 / 40 / 35,5 / 32.

### Beispiel 14

Eine Mischung aus 85% der Mischung aus Beispiel und 15% der Verbindung 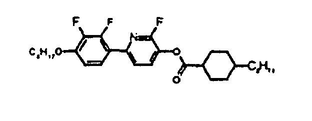 hat die Phasenübergänge I / N* 95.1 - 92.1 und N* / Sc* 64.6 °C sowie die Werte DT(15,1) / DT (20,1) / DT (25,1) / DT (30,1) 35 / 40 / 35,5 / 31,5.

### Beispiel 15

Eine Mischung aus 85% der Mischung aus Beispiel 5 und 15% der Verbindung 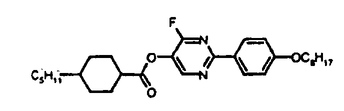 hat die Phasenübergänge I / N* 99.6 - 96.0 und N* / Sc* 63.2 °C sowie die Werte DT(15,1)/DT(20,1)/DT(25,1)/DT(30,1) 32,5 / 30 / 28,8 / 26.

Die erfindungsgemäßen Verbindungen werden anhand der Beispiele 16-25 näher erläutert.

### Beispiel 16

5-Octyl-thiophen-2-carbonsäure- 4-(2-fluor-3-hexyloxy-pyridin-6-yl)phenyl-ester

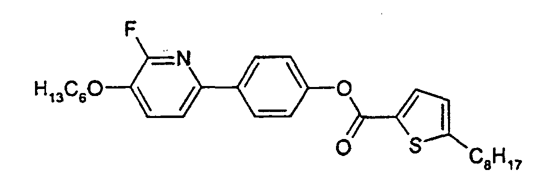

0,8 g 4-(2-Fluor-3-hexyloxy-pyridin-6-yl)phenol und 0,7 g 5-Octylthiophen-2-carbonsäure werden in Gegenwart von 0,6 g Dicyclohexylcarbodiimid in 100 ml Dichlormethan zur Reaktion gebracht. Nach Aufarbeitung durch Filtration, Säulenchromatografie und Umkristallisation resultieren 1 g farblose Kristalle mit dem Schmp. 101°C und dem Klärpunkt 124 °C.

Analog werden erhalten:

### Beispiel 17

5-Hexyl-thiophen-2-carbonsäure- 4-(2-fluor-3-hexyloxy-pyridin-6-yl)phenyl-ester mit Schmp. 95°C und Klärpunkt 126 °C.

### Beispiel 18

5-Butyl-thiophen-2-carbonsäure- 6-(4-octyloxyphenyl)-2-fluor-pyridin-3-yl-ester 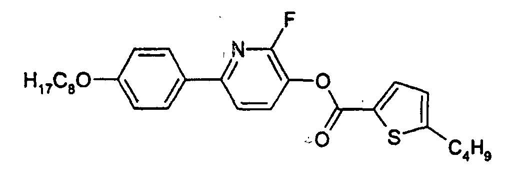 mit Schmp. 86°C und Klärpunkt 114 °C.

### Beispiel 19

5-Butyl-thiophen-2-carbonsäure-4-(5-decyl-4-fluor-pyrimidin-2-yl)phenyl-ester

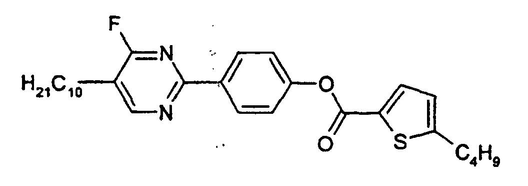

### Beispiel 20

trans-4-Pentylcyclohexancarbonsäure-4-(6-ethyl-1,2,3,4-tetrahydrochinazolin-2-yl)phenyl-ester 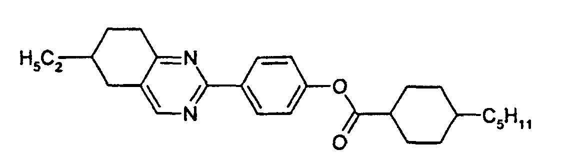 Phasenfolge X 114 N 216 I

### Beispiel 21

trans-4-Pentylcyclohexancarbonsäure-4-(6-nonyl-1,2,3,4-tetrahydrochinazolin-2-yl)phenyl-ester
Phasenfolge X 112 S_{C} 124 S_{A} 143 N 204 I

### Beispiel 22

trans-4-Propylcyclohexancarbonsäure-4-(6-nonyl- 1,2,3,4-tetrahydrochinazolin-2-yl)phenyl-ester
Phasenfolge X 111 (S_{C} 100) S_{A} 124 N 202 I

### Beispiel 23

trans-4-Pentylcyclohexancarbonsäure-4-(6-propyloxy-1,2,3,4-tetrahydrochinazolin-2-yl)phenyl-ester
Phasenfolge X 99 N 175 I

### Beispiel 24

trans-4-Pentylcyclohexancarbonsäure-4-(6-hexyloxy-1,2,3,4-tetrahydrochinazolin-2-yl)phenyl-ester
Phasenfolge X 100 N 155

### Beispiel 25

trans-4-Pentylcyclohexancarbonsäure-4-(6-octyloxy-1,2,3,4-tetrahydrochinazolin-2-yl)phenyl-ester
Phasenfolge X 97 (S_{C} 95) N 145 I

### Beispiel 26

trans-4-Pentylcyclohexancarbonsäure-4-(5-tetradecyl-pyrimidin-2-yl)phenylester Phasenfolge X 555₂ 96 S_{c} 130 N 151 I

### Beispiel 27

trans-4-Hexylcyclohexancarbonsäure-4-(5-tetradecyl-pyrimidin-2-yl)phenylester Phasenfolge X 77 S₂ 105 S_{c} 133 N 147 I

### Beispiel 28

trans-4-Heptylcyclohexancarbonsäure-4-(5-tetradecyl-pyrimidin-2-yl)phenylester Phasenfolge X 41 S₂ 108 S_{c} 136 N 148 I

### Beispiel 29

trans-4-Propylcyclohexancarbonsäure-2-(4-undecylphenyl)pyrimidin-5-yl-ester Phasenfolge X 77 S_{A} 165 N 171 I

### Beispiel 30

5-Pentylthiophen-2-carbonsäure-2-(4-undecylphenyl)pyrimidin-2-yl-ester Phasenfolge X 86 S_{A} 91 N 111 I

### Beispiel 31

5-Pentyl-thiophen-2-carbonsäure-4-(2-fluor-4-undecyl-phenyl)phenylester Phasenfolge X 41 N 79 I

### Beispiel 32

5-Pentyl-thiophen-2-carbonsäure-[4-(5-undecyl-pyridin-2-yl)-2-fluorphenyl]ester Phasenfolge X 74 N 89 I

### Beispiel 33

5-Pentyl-thiophen-2-carbonsäure-4-(5-undecyl-pyridin-2-yl)phenylester Phasenfolge X 61 S₂ 65 S_{c} 89 N 112 I

## Patentansprüche

1. Aktivmatrix-Display, enthaltend eine ferroelektrische (chiral-smektische) Flüssigkristallmischung, die mindestens 1 Verbindung der Formel (I) enthält
R¹-(A¹-M¹)ₐ -(A²-M²)_{b}-A³-X-B¹-(B²)_{c}-R² (I)
worin die Symbole die folgenden Bedeutungen haben:
R¹, R² unabhängig voneinander gleich oder verschieden
a) Wasserstoff, Fluor, oder CN
ein geradkettiger oder verzweigter (mit oder ohne asymmetrische C-Atome) Alkenyl-, Alkenyloxy-, Alkyl- oder Alkyloxy-Rest mit 2 - 16 C-Atomen, worin
b1) eine oder zwei nicht terminale -CH₂-Gruppen ersetzt sein können durch -O-, -OC(=O)-, -(C=O), -C(=O)O-, - Si(CH₃)₂-, -CH(Cl)- und / oder eine oder zwei -CH₂-Gruppen ersetzt sein können durch -CH=CH- oder -C≡C und worin auch ein oder mehrere H-Atome durch F ersetzt sein können und/oder
b2) eine oder mehrere -CH₂-Gruppen ersetzt sein können durch Phenylen-1,4-diyl (gegebenenfalls 1- oder 2-fach durch F substituiert), Phenylen-1,3-diyl (gegebenenfalls 1- oder 2-fach durch F substituiert), Cyclohexan-1,4-diyl (gegebenenfalls 1-fach durch F oder CN substituiert) oder Cylopropan-1,2-diyl
und worin auch ein oder mehrere H-Atome durch F ersetzt sein können
mit den Maßgaben, daß nur einer der Reste R¹, R² Wasserstoff , F oder CN sein kann und zwei benachbarte -CH₂-Gruppen nicht durch -0-ersetzt sein können
M¹, M² unabhängig voneinander gleich oder verschieden
-C(=O)O-, -OC(=O)-, -CH₂O-, -OCH₂-, -CF₂O-, -OCF₂-, -CH₂CH₂-, -CF₂CF₂-, -CH=CH-, -CH=CF-, -CF=CF-, -C≡C-, - CH₂CH₂C(=O)O-, -OC(=O)CH₂CH₂-, -(CH₂)₄- , -OCH₂CH₂CH₂-, - CH₂CH₂CH₂O- -OCH₂CF₂CH₂- , - CH₂CF₂CH₂O- oder eine Einfachbindung
A1, A2, A3 unabhängig voneinander gleich oder verschieden Cyclohexan-1,4-diyl (gegebenenfalls 1-fach substituiert durch F, CH3, CN), Cyclohex-1-en-1,4-diyl, Cyclohex-2-en-1,4-diyl, 2-Oxocyclohexan-1,4-diyl, 2-Cyclohexen-1-on-3,6-diyl, 1-Alkyl-1-sila-cyclohexan-1,4-diyl, Bicyclo[2.2.2]octan-1,4-diyl, Spiro[4.5]decan-2,8-diyl, Spiro[5.5]undecan-3,9-diyl, Phenylen-1,4-diyl (gegebenenfalls 1- oder 2-fach substituiert durch CN, CH3, CF3, OCF3 , gegebenenfalls 1-, 2-, 3- oder 4-fach substituiert durch F), Phenylen-1,3-diyl (gegebenenfalls 1- oder 2-fach substituiert durch CN, CH3, CF3, OCF3, gegebenenfalls 1-, 2- , 3- oder 4-fach substituiert durch F), Thiophen-2,5-diyl, Thiophen-2,4-diyl, (1,3,4)-Oxadiazol-2,5-diyl, (1,3,4)-Thiadiazol-2,5-diyl, 1,3-Thiazol-2,5-diyl, 1,3-Thiazol-2,4-diyl, (1,3)-Oxazol-2,5-diyl, Isoxazol-2,5-diyl, Indan-2,6-diyl, Naphthalin-2,6-diyl (gegebenenfalls 1- oder 2-fach substituiert durch F oder CN), 1,2,3,4-Tetrahydronaphthalin-2,6-diyl, Decalin-2,6-diyl, Pyrimidin-2,5-diyl (gegebenenfalls 1-fach substituiert durch F), Pyridin-2,5-diyl (gegebenenfalls 1- oder 2-fach substituiert durch F), Pyrazin-2,5-diyl (gegebenenfalls 1-fach substituiert durch F), Pyridazin-3,6-diyl, Chinolin-2,6-diyl, Chinolin-3,7-diyl, Isochinolin-3,7-diyl, Chinazolin-2,6-diyl, 5,6,7,8-Tetrahydrochinazolin-2,6-diyl, Chinoxalin-2,6-diyl, 1,3-Dioxan-2,5-diyl (gegebenenfalls 1-fach substituiert durch CN), Benzothiazol-2,6-diyl, Piperidin-1,4-diyl, Piperazin-1,4-diyl
B1 Cyclohexan-1,4-diyl (gegebenenfalls 1- oder 2-fach substituiert durch F, CH3, CN), Perfluorcyclohexan-1,4-diyl, Cyclohex-1-en-1,4-diyl, Cyclohex-2-en-1,4-diyl, 1-Alkyl-1-sila-cyclahexan-1,4-diyl, Bicyclo[2.2.2]octan-1,4-diyl, Cyclopentan-l,3-diyl, Cycloheptan-1,4-diyl, Tetrahydrofuran-2,5-diyl, Tetrahydrofuran-2,4-diyl, Phenylen-1,4-diyl (gegebenenfalls 1- oder 2-fach substituiert durch CN, CH3, CF3, OCF3, gegebenenfalls 1-, 2-, 3- oder 4-fach substituiert durch F), Phenylen-1,3-diyl (gegebenenfalls 1- oder 2-fach substituiert durch CN, CH3, CF3, OCF3, gegebenenfalls 1- oder 2- oder 3-fach substituiert durch F), Thiophen-2,5-diyl (gegebenenfalls 1-fach substituiert durch F), Thiophen-2,4-diyl (gegebenenfalls 1-fach substituiert durch F), 1,3-Thiazol-2,5-diyl (gegebenenfalls 1-fach substituiert durch F), 1,3-Thiazol-2,4-diyl (gegebenenfalls 1-fach substituiert durch F), (1,3,4)-Thiadiazol-2,5-diyl, 1,3-Dioxan-2,5-diyl (gegebenenfalls 1-fach substituiert durch CN), Tetrahydropyran-2,5-diyl, 6,6-Difluortetrahydropyran-2,5-diyl, 6,6-Difluor-2,3-dihydro-6H-pyran-2,5-diyl, 6-FIuor-3,4-dihydro-2H-pyran-2,5-diyl, Piperidin-1,4-diyl, Piperazin-1,4-diyl, Pyrimidin-2,5-diyl (gegebenenfalls 1-fach substituiert durch F), Pyridin-2,5-diyl (gegebenenfalls 1-fach substituiert durch F), 1,2,3,4-Tetrahydronaphthalin-2,6-diyl, Decalin-2,6-diyl
B² Cyclohexan-1,4-diyl (gegebenenfalls 1- oder 2-fach substituiert durch F, CH₃, CN), Cyclohex-1-en-1,4-diyl (gegebenenfalls 1-fach substituiert durch F), Cyclohex-2-en-1,4-diyl, 1-Alkyl-1-silacyclohexan-1,4-diyl, Bicycio[2.2.2]octan-1,4-diyl, Phenylen-1,4-diyl (gegebenenfalls 1- oder 2-fach substituiert durch CN, CH₃, CF₃, OCF₃, gegebenenfalls 1-, 2-, 3- oder 4-fach substituiert durch F), Phenylen-1,3-diyl (gegebenenfalls 1- oder 2-fach substituiert durch CN, CH₃, CF₃, OCF₃, gegebenenfalls 1- oder 2- oder 3-fach substituiert durch F), Thiophen-2,5-diyl, Thiophen-2,4-diyl, 1,3-Thiazol-2,5-diyl, 1,3-Thiazol-2,4-diyl, (1,3,4)-Thiadiazol-2,5-diyl, 1,3-Dioxan-2,5-diyl (gegebenenfalls 1-fach substituiert durch CN), Tetrahydrofuran-2,5-diyl, Tetrahydropyran-2,5-diyl, 6,6-Difluortetrahydropyran-2,5-diyl, 6,6-Difluor-2,3-dihydro-6H-pyran-2,5-diyl, 6-Fluor-3,4-dihydro-2H-pyran-2,5-diyl, Pyrimidin-2,5-diyl (gegebenenfalls 1-fach substituiert durch F), Pyridin-2,5-diyl (gegebenenfalls 1-fach substituiert durch F), Indan-2,6-diyl, Piperidin-1,4-diyl, Piperazin-1,4-diyl, Pyrimidin-2,5-diyl (gegebenenfalls 1-fach substituiert durch F)
X -(CH₂)ₙ- , wobei
a) eine oder zwei -CH₂-Gruppen durch -O- oder -C(=O)-ersetzt sein können und/oder
b) eine -CH₂CH₂-Gruppe durch -CH=CH- ersetzt sein kann und ein oder mehrere H der -CH₂-Gruppen durch F ersetzt sein können
mit den Maßgaben, daß
1) n 2, 3 oder 4 bedeutet
2) zwei benachbarte -CH2-Gruppen nicht durch -O- ersetzt sein können
a, b, c Null, 1 oder 2 mit den Maßgaben, daß
1) a 1 sein muß, wenn R¹ Wasserstoff, F oder CN bedeutet
2) die Summe a+b+c mindestens 1 ist
3) die in der Klammer stehenden Reste A bzw. M unterschiedliche oder gleiche Bedeutung haben können, wenn der entsprechende Index 2 ist,
in einer Flüssigkristallschicht in Form einer Monodomäne mit einer eindeutig definierten Richtung der Schichtennormalen z der SmC*-Phase, wobei die Schichtennormalen z und die Vorzugsrichtung n der nematischen bzw. cholesterischen Phase (N*-Phase) einen Winkel von mehr als 5° ausbilden.

2. Display nach Anspruch 1 , **dadurch gekennzeichnet, daß** die Flüssigkristallmischung eine spontane Polarisation <200 nC / cm² aufweist und DT (15,1) > 20 ist.

3. Display nach einem der Ansprüche 1 oder 2, **dadurch gekennzeichnet, daß** in (I)
X -OC(=)O-, -OCH2- oder -OC(=O)CH2CH2- bedeutet.

4. Display nach einem der Ansprüche 1 bis 3, **dadurch gekennzeichnet, daß** in (I)
B1 Cyclohexan-1,4-diyl, Cyclohex-1-en-1,4-diyl, Phenylen-1,4-diyl, gegebenenfalls einfach oder zweifach durch F substituiert, oder Thiophen-2,5-diyl bedeutet.

5. Display nach einem der Ansprüche 1 bis 4, **dadurch gekennzeichnet, daß** in (I)
A1 Pyrimidin-2,5-diyl (gegebenenfalls 1-fach durch F substituiert), Pyridin-2,5-diyl (gegebenenfalls 1-fach durch F substituiert), Phenylen-1,4-diyl (gegebenenfalls 1- oder 2-fach durch F substituiert), oder (1,3,4)-Thiadiazol-2,5-diyl bedeutet.

6. Display nach einem der Ansprüche 1 bis 5, **dadurch gekennzeichnet, daß** die Flüssigkristallmischung aus 3 bis 30 Verbindungen besteht und mindestens 1 Verbindung der Formel (I) und mindestens 1 Verbindung der nachstehenden Formel (II) und gegebenenfalls mindestens 1 Verbindung der nachstehenden Formel (III) enthält 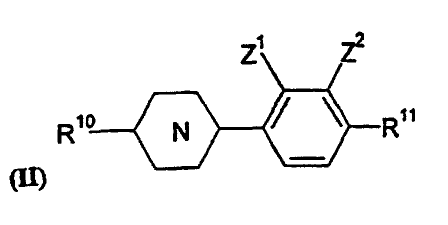 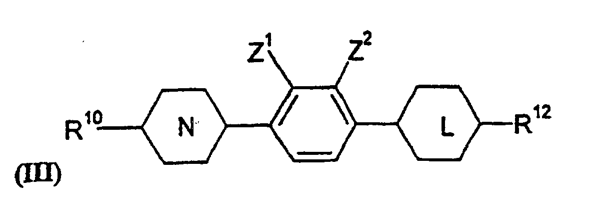 worin bedeuten:
R¹⁰, R¹¹ wie R¹ , R^{2,} wobei zusätzlich jeweils die terminale -CH₃-Gruppe durch eine der folgenden chiralen Gruppen (optisch aktiv oder racemisch) ersetzt sein kann: 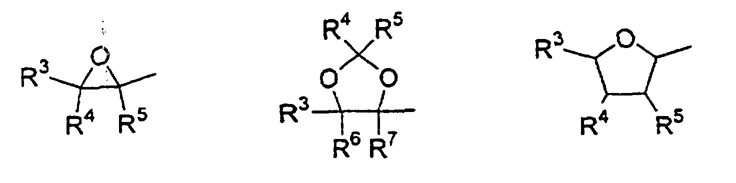 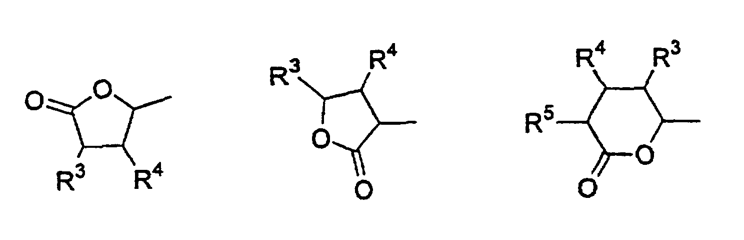 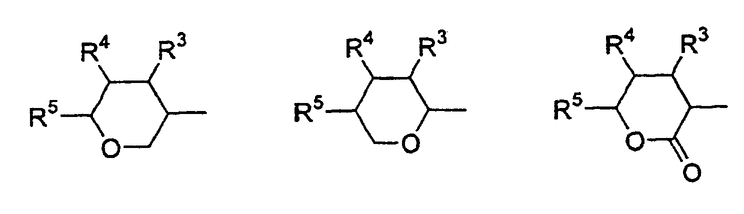 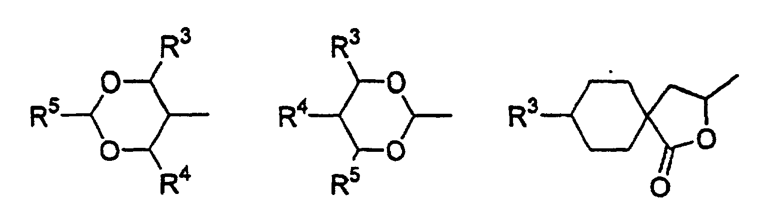 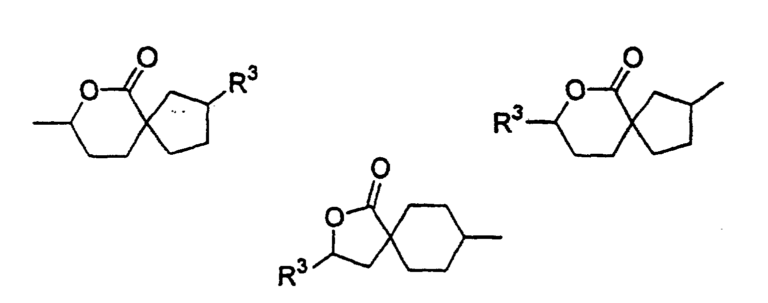 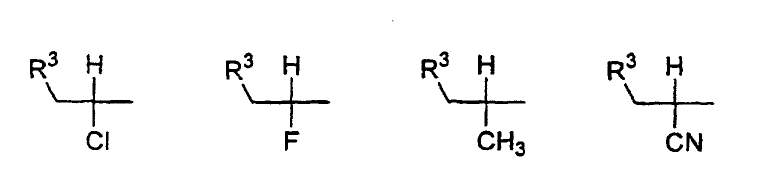 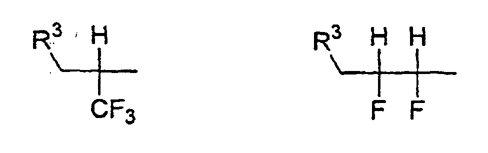
R³, R⁴, R⁵, R⁶, R⁷ sind gleich oder verschieden
a) Wasserstoff
b) ein geradkettiger oder verzweigter Alkylrest (mit oder ohne asymmetrischen Kohlenstoffatomen) mit 1 bis 16 C-Atomen, wobei
b1) eine oder mehrere nicht benachbarte und nicht terminale CH₂-Gruppen durch -O- ersetzt sein können und/oder
b2) eine oder zwei CH₂-Gruppen durch -CH=CH- ersetzt sein können,
c) R⁴ und R⁵ zusammen auch -(CH₂)₄- oder -(CH₂)₅-, wenn sie an ein Oxiran-, Dioxolan-, Tetrahydrofuran-, Tetrahydropyran-, Butyrolacton- oder Valerolacton-System gebunden sind;
R¹² Wasserstoff oder einen geradkettigen oder verzweigten Alkylrest (mit oder ohne asymmetrische C-Atome) mit 1 bis 16 C-Atomen, worin auch ein oder mehrere H durch F ersetzt sein können und worin auch eine oder zwei nicht benachbarte, nicht terminale -CH₂-Gruppen durch -O- ersetzt sein können
Z¹,Z²,Z³,Z⁴ ,Z⁵,Z⁶ unabhängig voneinander H oder F 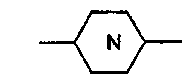 einen bivalenten Rest ausgewählt aus der Gruppe Pyridin-2,5-diyl, gegebenenfalls einfach durch F substituiert, Pyrimidin-2,5-diyl, gegebenenfalls einfach durch F substituiert, Pyrazin-2,5-diyl, gegebenenfalls einfach durch F substituiert, 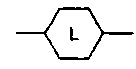 einen bivalenten Rest ausgewählt aus der Gruppe Cyclohexan-1,4-diyl, gegebenenfalls einfach substituiert durch CN, CH₃ oder zweifach durch F, Cyclohex-1-en-1,4-diyl , Perfluorcyclohexan-1,4-diyl, Cyclohex-2-en-1,4-diyl, 1-Alkyl-1-sila-cyclohexan-1,4-diyl, Bicyclo[2.2.2]octan-1,4-diyl.

7. Display nach einem der Ansprüche 1 bis 5, **dadurch gekennzeichnet, daß** die Flüssigkristallmischung aus 3 bis 30 Verbindungen besteht und mindestens eine Verbindung der Formel (I) und mindestens eine Verbindung der Formel (II) und mindestens eine weitere Verbindung, ausgewählt aus der Gruppe (III), (IV), (V), (VI), (VII) enthält, wobei die Verbindungen der Formeln (II) und (III) wie in Anspruch 6 definiert sind, 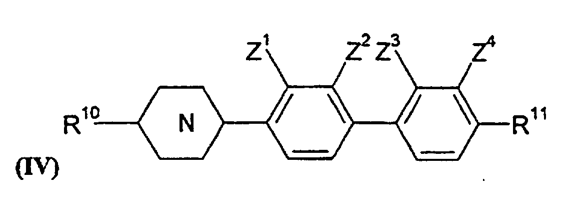 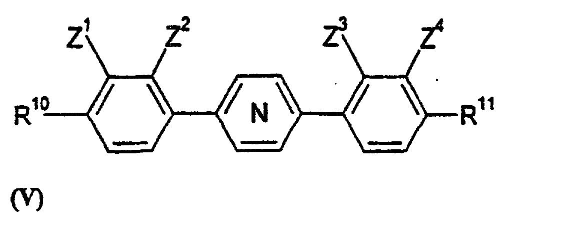 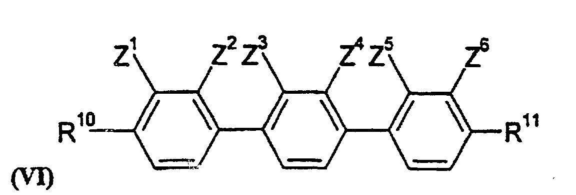 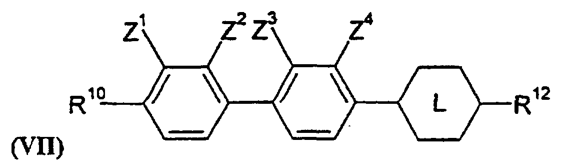 wobei die Symbole und Indices die in Anspruch 6 angegebene Bedeutung haben.

8. Display nach einem der Ansprüche 1 bis 7, **dadurch gekennzeichnet, daß** die Flüssigkristallmischung aus 3 bis 30 Verbindungen besteht und mindestens eine Verbindung der Formel (I) und mindestens eine Verbindung der Formel (II) und mindestens eine weitere Verbindung ausgewählt aus der Gruppe (VIII), (IX), (X), (XI), (XII), (XIII), (XIV), (XV), (XVI), (XVII) enthält, wobei die Verbindungen der Formeln (II) und (III) wie in Anspruch 6 definiert sind. 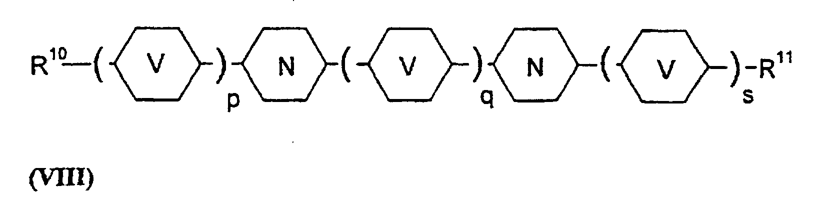 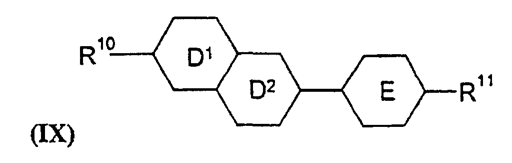 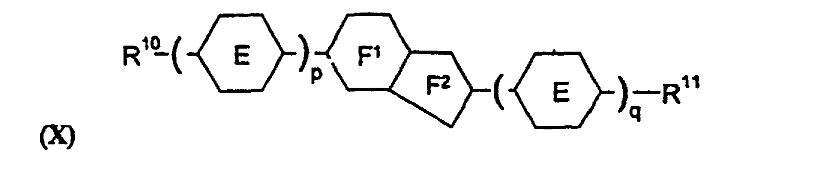 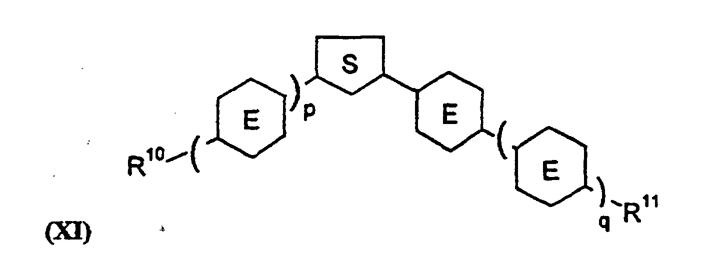 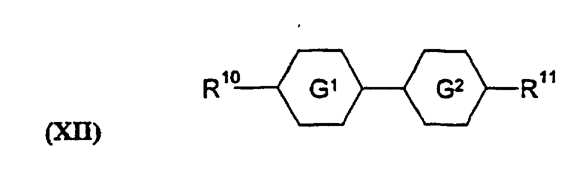 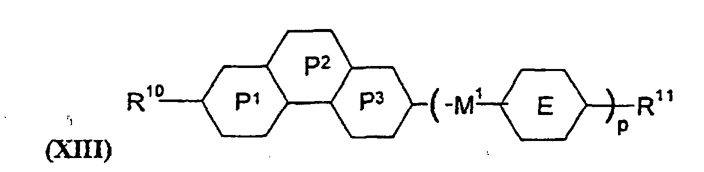 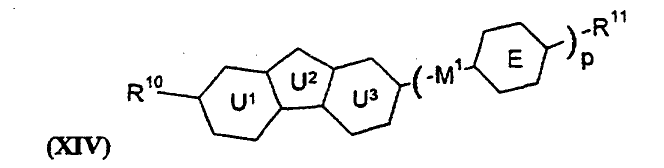 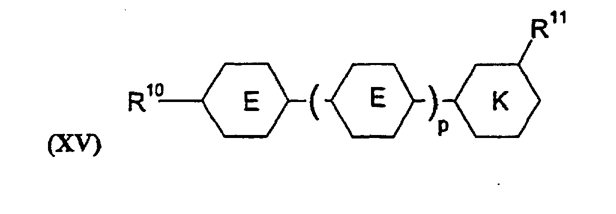 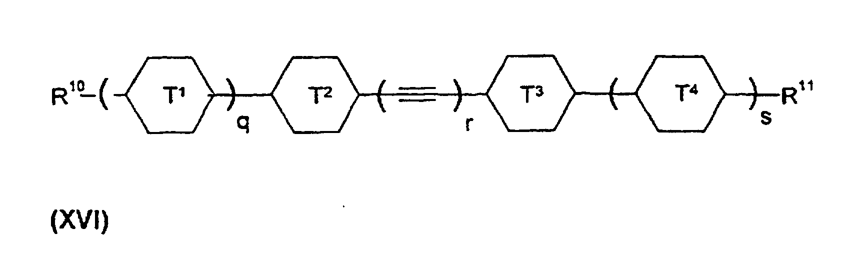 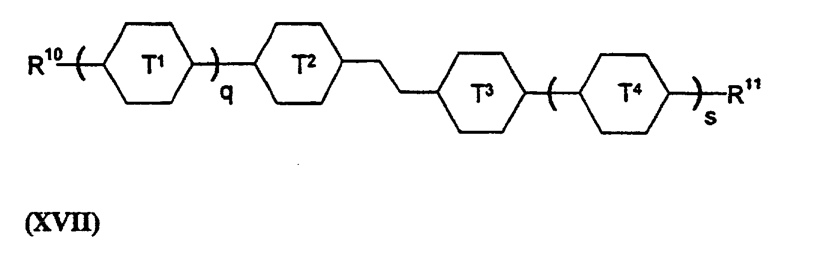 wobei die Symbole und Indices die in Anspruch 6 bzw. nachstehend angegebene Bedeutung haben: 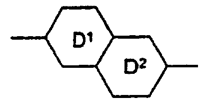 einen bivalenten Rest ausgewählt aus der Gruppe Naphthalin-2,6-diyl, worin auch ein oder zwei Ring-C-Atome durch N ersetzt sein können und das auch einfach oder zweifach durch F oder CN substituiert sein kann und worin auch D¹ oder D² einen (gesättigten ) Alicyclus bedeuten kann 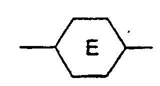 einen bivalenten Rest ausgewählt aus der Gruppe Phenylen-1,4-diyl, gegebenenfalls einfach oder zweifach substituiert durch CN oder einfach, zweifach, dreifach oder vierfach substituiert durch F, Pyridin-2,5-diyl, gegebenenfalls einfach substituiert durch F, Pyrimidin-2,5-diyl, gegebenenfalls einfach substituiert durch F, Cyclohexan-1,4-diyl 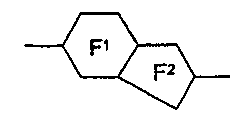 einen bivalenten Rest ausgewählt aus der Gruppe Indan-2,5-diyl, gegebenenfalls im aromatischen Ring einfach oder zweifach substituiert durch F, Indan-1-on-2,6-diyl, gegebenenfalls im aromatischen Ring einfach oder zweifach substituiert durch F, Benzothiazol-2,6-diyl, Benzothiazol-2,5-diyl, Benzo[b]thiophen-2,5-diyl, Benzo[b]thiophen-2,6-diyl 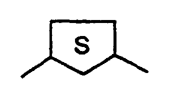 einen bivalenten Rest ausgewählt aus der Gruppe (1,3,4)-Thiadiazol-2,5-diyl, (1,3)-Thiazol-2,5-diyl, Thiophen-2,5-diyl, (1,3,4)-Oxadiazol-2,5-diyl, (1,3)-Oxazol-2,5-diyl, Isoxazol-2,5-diyl 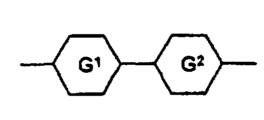 einen bivalenten Rest ausgewählt aus der Gruppe 1,1'-Biphenyl-4,4'-diyl, gegebenenfalls einfach oder zweifach substituiert durch CN oder einfach, zweifach, dreifach oder vierfach substituiert durch F , 1,1'-Phenylcyclohexyl-4,4'-diyl, 5,5'-Pyridylpyrimidin-2,2'-diyl, gegebenenfalls in einem oder beiden der Heterocyclen einfach durch F substituiert, 5,2'-Pyridylpyrimidin-2,5'diyl, gegebenenfalls in einem oder beiden der Heterocyclen einfach durch F substituiert, 1,2'-Phenyldioxan-4,5'-diyl, 1,2'-(2-Fluorphenyl)dioxan-4,5'-diyl, 1,2'-(3-Fluorphenyl)dioxan-4,5'-diyl, 1,2'- (2,3-Difluorphenyl)dioxan-4,5'-diyl 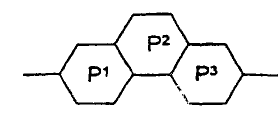 einen bivalenten Phenanthren-2,7-diyl-Rest, bei dem auch ein oder zwei Ring-C-Atome durch N ersetzt sein können und der auch einfach, zweifach, dreifach oder vierfach durch F substituiert sein kann und bei dem P² und / oder P³ auch einen (gesättigten) Alicyclus bedeuten können 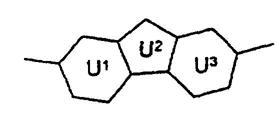 einen bivalenten Fluoren-2,7-diyl-Rest, bei dem auch die -CH₂-Gruppe in U² durch -C(=O)- , -CHF- oder -CF₂- ersetzt sein kann 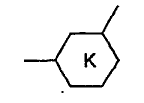 einen bivalenten Rest ausgewählt aus der Gruppe Phenylen-1,3-diyl, gegebenenfalls einfach oder zweifach substituiert durch F, Cyclohexan-1,3-diyl, gegebenenfalls einfach substituiert durch F oder CN, Pyridin-2,6-diyl, Pyridin-2,4-diyl, Pyridin-3,5-diyl, Pyridin-4,6-diyl, Pyrimidin-4,6-diyl, 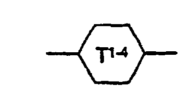 einen bivalenten Rest ausgewählt aus der Gruppe Phenylen-1,4-diyl, gegebenenfalls einfach oder zweifach substituiert durch CN oder F, Naphthalin-2,6-diyl, bei dem auch ein oder zwei Ring-C-Atome durch N ersetzt sein können und das gegebenenfalls einfach oder zweifach substituiert ist durch CN oder F, Cyclohexan-1,4-diyl, Cyclohex-1-en-1,4-diyl, Bicyclo[2.2.2]octan-1,4-diyl, (1,3)-Dioxan-2,5-diyl, Pyridin-2,5-diyl, gegebenenfalls einfach substituiert durch F, Pyrimidin-2,5-diyl, gegebenenfalls einfach substituiert durch F, (1,3,4)-Thiadiazol-2,5-diyl, Indan-2,5-diyl, gegebenenfalls im aromatischen Ring einfach oder zweifach substituiert durch F , Thiophen-2,5-diyl 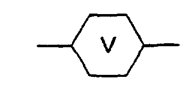 einen bivalenten Rest ausgewählt aus der Gruppe Phenylen-1,4-diyl, gegebenenfalls einfach oder zweifach substituiert durch CN oder F, Naphthalin-2-6-diyl, bei dem auch ein oder zwei Ring-C-Atome durch N ersetzt sein können und das gegebenenfalls einfach oder zweifach substituiert ist durch CN oder F, Cyclohexan-1,4-diyl, Cyclohex-1-en-1,4-diyl, Bicyclo[2.2.2]octan-1,4-diyl, (1,3)-Dioxan-2,5-diyl, Indan-2,5-diyl, gegebenenfalls im aromatischen Ring einfach oder zweifach substituiert durch F , Thiophen-2,5-diyl
p, q , s Null oder 1
r 1 oder 2.

9. Chiral smektische Flüssigkristallmischung nach einem der Ansprüche 1 bis 6, enthaltend 10 bis 60 % einer oder mehrerer Verbindungen der Formel (I).

10. Chiral smektische Flüssigkristallmischung nach Anspruch 6, **dadurch gekennzeichnet, daß** die Mischung 10 bis 60 % von 1 bis 15 Verbindungen der Formel (I) und 40 bis 90 % von 2 bis 15 Verbindungen der Formel (II) enthält.

11. Verbindungen der allgemeinen Formel (I) gemäß Anspruch 1, ausgewählt aus Verbindungen der Formel (XXIII), worin bedeuten: 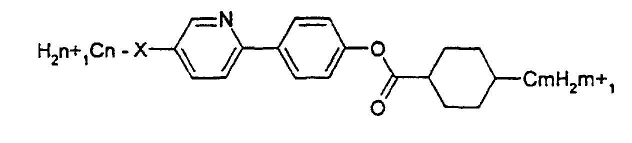 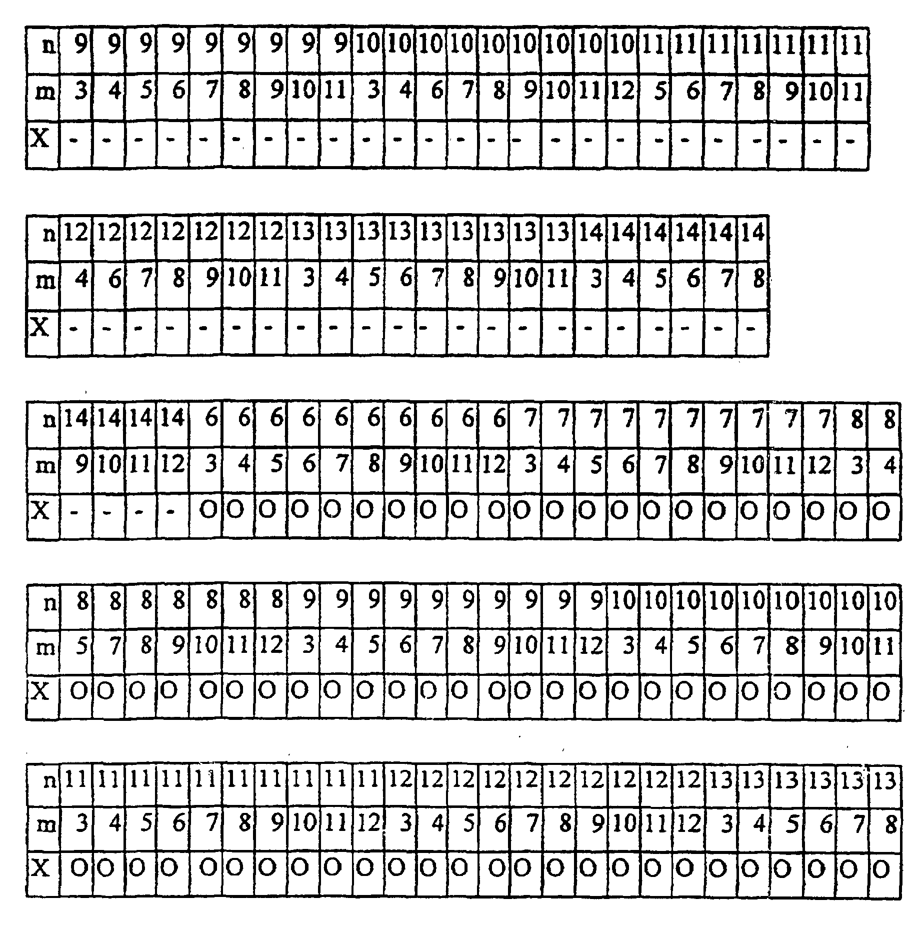 Verbindungen der Formel (XXIV), worin bedeuten: 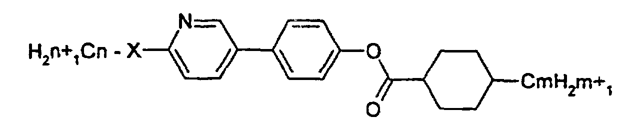
n ganze Zahl von 8 bis 14
m ganze Zahl von 3 bis 11
X Einfachbindung
ausgenommen n=11, m=3 oder 5, X Einfachbindung,
Verbindungen der Formel (XXV), worin bedeuten: 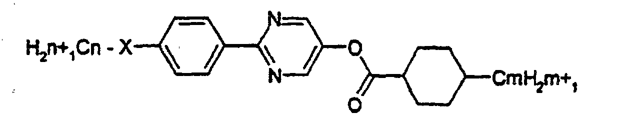
n ganze Zahl von 2 bis 13
m ganze Zahl von 3 bis 11
X O oder Einfachbindung
ausgenommen n=2, m=11, X=O; n=5, m=5, X=O,
Verbindungen der Formel (XXVI), worin bedeuten: 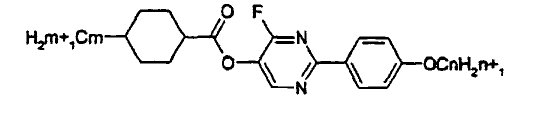
n ganze Zahl von 5 bis 13
m ganze Zahl von 3 bis 10
ausgenommen n=8, m=5,
Verbindungen der Formel (XXVII), worin bedeuten: 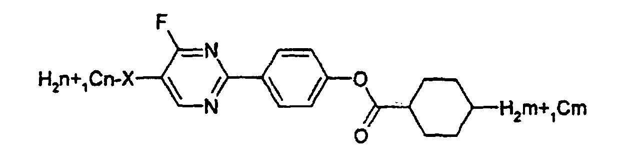 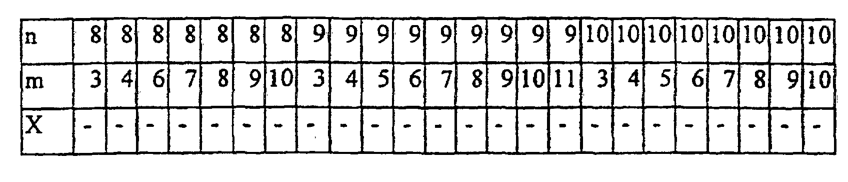 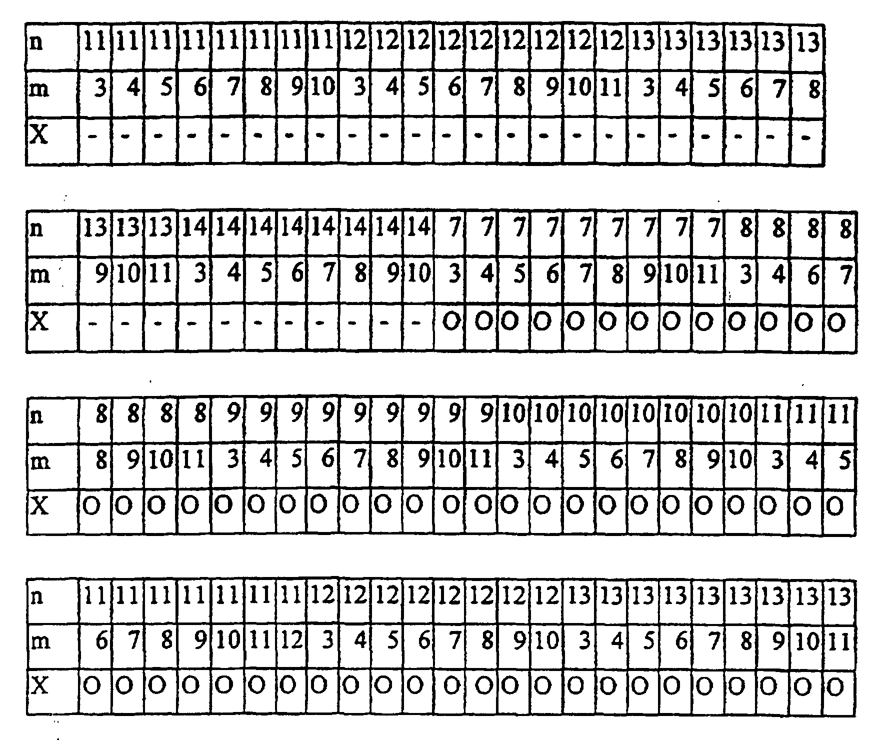 Verbindungen der Formel (XXIX), worin bedeuten: 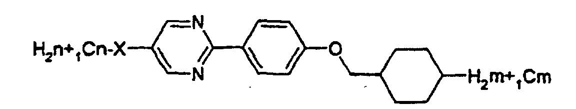 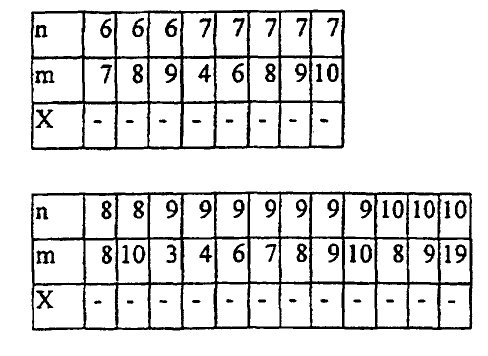 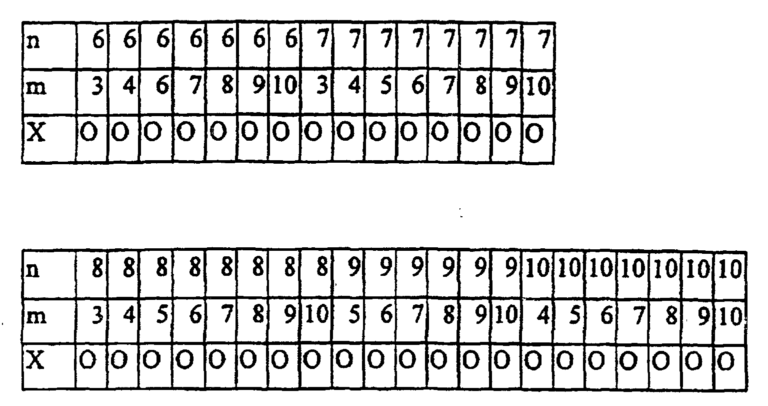 Verbindungen der Formel (XXX), worin bedeuten: 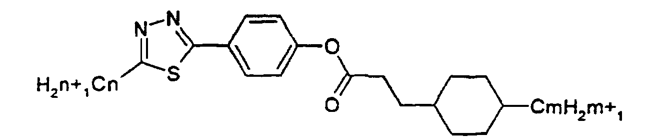
n ganze Zahl von 5 bis 13
m ganze Zahl von 3 bis 10
ausgenommen n=8, m=4; n=9, m=3,

12. Verbindungen der allgemeinen Formel (II) gemäß Anspruch 6, ausgewählt aus Verbindungen der Formel (XXXI), worin bedeuten: 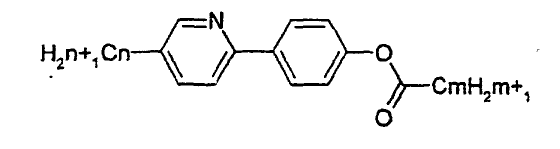 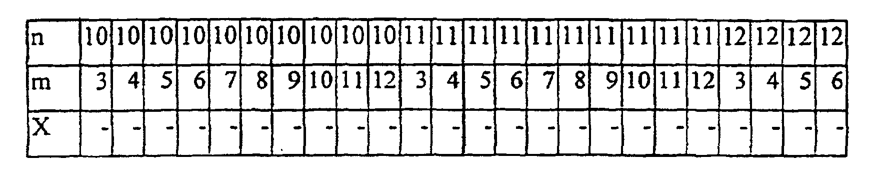 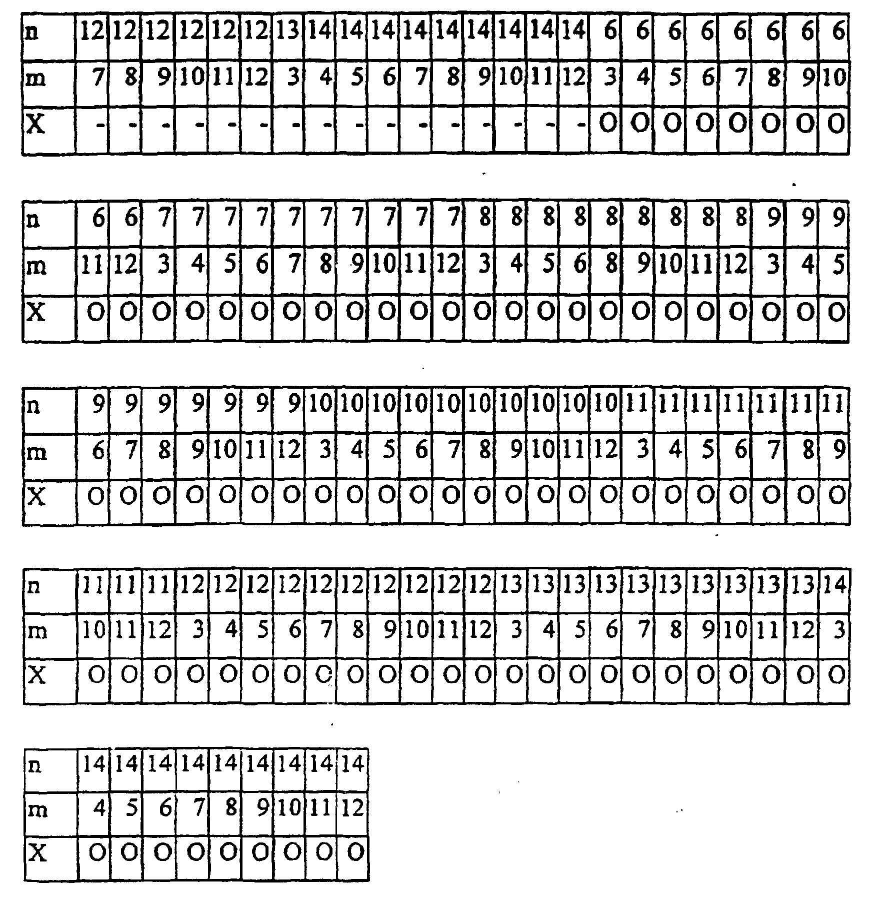 Verbindungen der Formel (XXVIII), worin bedeuten: 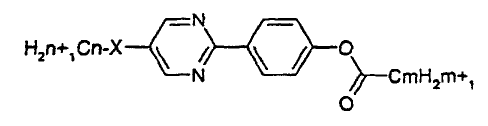 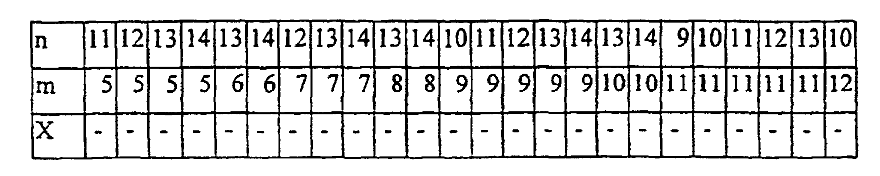 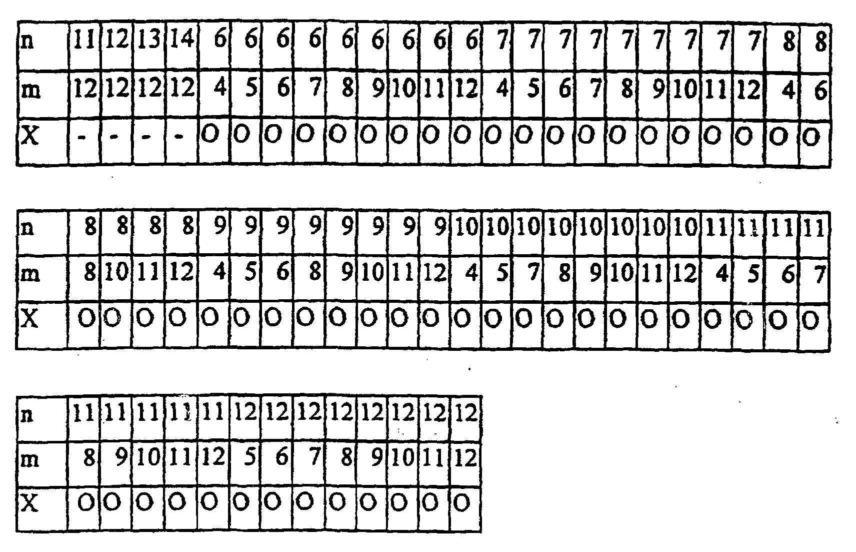 Verbindungen der Formel (XXXII), worin bedeuten: 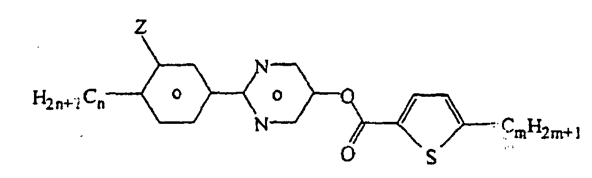 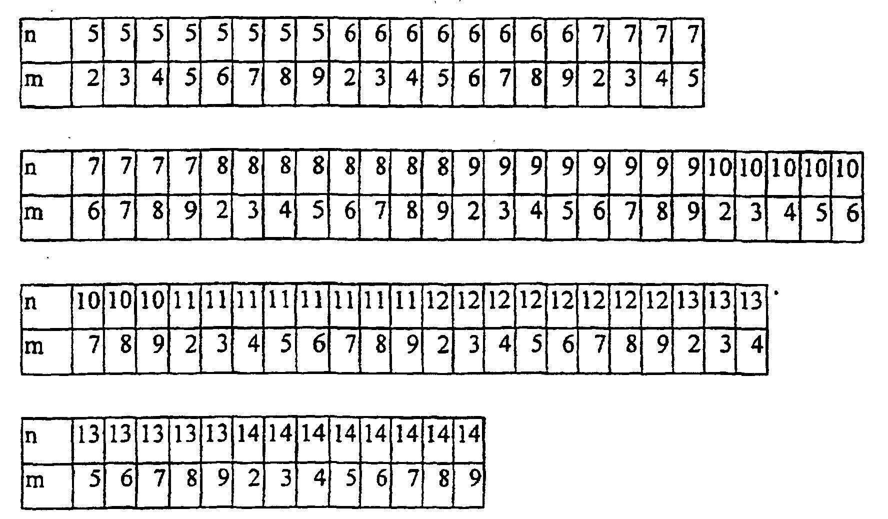 und Z in allen Fällen H oder F bedeutet.

## Claims

1. An active-matrix display containing a ferroelectric (chiral smectic) liquid-crystal mixture comprising at least one compound of the formula (I)
R¹-(A¹-M¹)ₐ-(A²-M²)_{b}-A³-X-B¹-(B²)_{c}-R² (I)
where the symbols are as defined below:
R¹, R² are, independently of one another, identical or different and are each
a) hydrogen, fluorine or CN
a straight-chain or branched alkenyl, alkenyloxy, alkyl or alkyloxy radical (with or without asymmetric carbon atoms) having 2 to 16 carbon atoms, where
b1) one or two nonterminal -CH₂- groups may be replaced by -O-, -OC(=O)-, -(C=O), -C(=O)O-, -Si(CH₃)₂-, - CH(CI)- and/or one or two -CH₂- groups may be replaced by -CH=CH- or -C≡C- and one or more H atoms may be replaced by F and/or
b2) one or more -CH₂- groups may be replaced by phenylene-1,4-diyl (unsubstituted, monosubstituted or disubstituted by F), phenylene-1,3-diyl (unsubstituted, monosubstituted or disubstituted by F), cyclohexane-1,4-diyl (unsubstituted or monosubstituted by F or CN) or cyclopropane-1,2-diyl
and one or more H atoms may be replaced by F
with the provisos that only one of the radicals R¹, R² can be hydrogen, F or CN and that two adjacent -CH₂- groups cannot be replaced by -O-
M¹, M² are, independently of one another, identical or different and are each
-C(=O)O-, -OC(=O)-, -CH₂O-, -OCH₂-, -CF₂O-, -OCF₂-, -CH₂CH₂-, -CF₂CF₂-, -CH=CH-, -CH=CF-, -CF=CF-, -C≡C-, - CH₂CH₂C(=O)O-, -OC(=O)CH₂CH₂-, -(CH₂)₄-, -OCH₂CH₂CH₂-, -CH₂CH₂CH₂O-, -OCH₂CF₂CH_{2,} -CH₂CF₂CH₂O- or a single bond
A¹, A², A³ are, independently of one another, identical or different and are each cyclohexane-1,4-diyl (unsubstituted or monosubstituted by F, CH₃, CN), cyclohex-1-ene-1,4-diyl, cyclo-hex-2-ene-1,4-diyl, 2-oxocyclohexane-1,4-diyl, 2-cyclohexen-1-one-3,6-diyl, 1-alkyl-1-silacyclohexane-1,4-diyl, bicyclo-[2.2.2]octane-1,4-diyl, spiro[4.5]decane-2,8-diyl, spiro[5.5]-undecane-3,9-diyl, phenylene-1,4-diyl (unsubstituted, monosubstituted or disubstituted by CN, CH₃, CF₃, OCH₃, unsubstituted, monosubstituted, disubstituted, trisubstituted or tetrasubstituted by F), phenylene-1,3-diyl (unsubstituted, monosubstituted or disubstituted by CN, CH₃, CF₃, OCF₃, unsubstituted, monosubstituted, disubstituted, trisubstituted or tetrasubstituted by F), thiophene-2,5-diyl, thiophene-2,4-diyl, (1,3,4)-oxadiazole-2,5-diyl, (1,3,4)-thiadiazole-2,5-diyl, 1,3-thiazole-2,5-diyl, 1,3-thiazole-2,4-diyl, (1,3)-oxazole-2,5-diyl, isoxazole-2,5-diyl, indane-2,6-diyl, naphthalene-2,6-diyl (unsubstituted, monosubstituted or disubstituted by F or CN), 1,2,3,4-tetrahydronaphthalene-2,6-diyl, decaline-2,6-diyl, pyrimidine-2,5-diyl (unsubstituted or monosubstituted by F), pyridine-2,5-diyl (unsubstituted, monosubstituted or disubstituted by F), pyrazine-2,5-diyl (unsubstituted or monosubstituted by F), pyridazine-3,6-diyl, quinoline-2,6-diyl, quinoline-3,7-diyl, isoquinoline-3,7-diyl, quinazoline-2,6-diyl, 5,6,7,8-tetrahydroquinazoline-2,6-diyl, quinoxaline-2,6-diyl, 1,3-dioxane-2,5-diyl (unsubstituted or monosubstituted by CN), benzothiazole-2,6-diyl, piperidine-2,4-diyl, piperazine-1,4-diyl
B¹ is cyclohexane-1,4-diyl (unsubstituted, monosubstituted or disubstituted by F, CH₃, CN), perfluorocyclohexane-1,4-diyl, cyclohex-1-ene-1,4-diyl, cyclohex-2-ene-1,4-diyl, 1-alkyl-1-silacyclohexane-1,4-diyl, bicyclo[2.2.2]octane-1,4-diyl, cyclopentane-1,3-diyl, cycloheptane-1,4-diyl, tetrahydrofuran-2,5-diyl, tetrahydrofuran-2,4-diyl, phenylene-1,4-diyl (unsubstituted, monosubstituted or disubstituted by CN, CH₃, CF₃, OCF₃, unsubstituted, monosubstituted, disubstituted, trisubstituted or tetrasubstituted by F), phenylene-1,3-diyl (unsubstituted, monosubstituted or disubstituted by CN, CH₃, CF₃, OCF₃, unsubstituted, monosubstituted, disubstituted or trisubstituted by F), thiophene-2,5-diyl (unsubstituted or monosubstituted by F), thiophene-2,4-diyl (unsubstituted or monosubstituted by F), 1,3-thiazol-2,5-diyl (unsubstituted or monosubstituted by F), 1,3-thiazol-2,4-diyl (unsubstituted or monosubstituted by F), (1,3,4)-thiadiazol-2,5-diyl, 1,3-dioxane-2,5-diyl (unsubstituted or monosubstituted by CN), tetrahydropyran-2,5-diyl, 6,6-difluorotetrahydro pyran-2,5- diyl, 6,6-difluoro-2,3-dihydro-6H-pyran-2,5-diyl, 6-fluoro-3,4-dihydro-2H-pyran-2,5-diyl, piperidine-1,4-diyl, piperazine-1,4-diyl, pyrimidine-2,5-diyl (unsubstituted or monosubstituted by F), pyridine-2,5-diyl (unsubstituted or monosubstituted by F), 1,2,3,4-tetrahydronaphthalene-2,6-diyl, decaline-2,6-diyl
B² is cyclohexane-1,4-diyl (unsubstituted, monosubstituted or disubstituted by F, CH₃, CN), cyclohex-1-ene-1,4-diyl (unsubstituted or monosubstituted by F), cyclohex-2-ene-1,4-diyl, 1-alkyl-1-silacyclohexane-1,4-diyl, bicyclo[2.2.2]-octane-1,4-diyl, phenylene-1,4-diyl (unsubstituted, monosubstituted or disubstituted by CN, CH₃, CF₃, OCF₃, unsubstituted, monosubstituted, disubstituted, trisubstituted or tetrasubstituted by F), phenylene-1,3-diyl (unsubstituted, monosubstituted or disubstituted by CN, CH₃, CF₃, OCF₃, unsubstituted, monosubstituted, disubstituted or trisubstituted by F), thiophene-2,5-diyl, thiophene-2,4-diyl, 1,3-thiazole-2,5-diyl, 1,3-thiazole-2,4-diyl, (1,3,4)-thiadiazole-2,5-diyl, 1,3-dioxane-2,5-diyl (unsubstituted or monosubstituted by CN), tetrahydrofuran-2,5-diyl, tetrahydropyran-2,5-diyl, 6,6-difluorotetrahydropyran-2,5-diyl, 6,6-difluoro-2,3-dihydro-6H-pyran-2,5-diyl, 6-fluoro-3,4-dihydro-2H-pyran-2,5-diyl, pyrimidine-2,5-diyl (unsubstituted or monosubstituted F), pyridine-2,5-diyl (unsubstituted or monosubstituted F), indane-2,6-diyl, piperidine-1,4-diyl, piperazine-1,4-diyl, pyrimidine-2,5-diyl (unsubstituted or monosubstituted by F)
X is -(CH₂)ₙ-, where
a) one or two -CH₂- groups may be replaced by -O- or - C(=O)- and/or
b) one -CH₂CH₂- group may be replaced by -CH=CH- and one or more H of the -CH₂- groups may be replaced by F
with the provisos that
1) n is 2, 3 or 4
2) two adjacent -CH₂- groups cannot be replaced by -O-
a, b, c are each zero, 1 or 2, with the provisos that
1) a must be 1 when R¹ is hydrogen, F or CN
2) the sum of a+b+c is at least 1
3) the radicals A and M, respectively, in the brackets may be identical or different when the corresponding index is 2,
in a liquid crystal layer in the form of a monodomain having an unambiguously defined direction of the layer normal z of the SmC* phase, where the layer normal z and the preferential direction n of the nematic or cholesteric phase (N* phase) form an angle of more than 5°.

2. A display as claimed in claim 1, wherein the liquid-crystal mixture has a spontaneous polarization of < 200 nC/cm² and DT (15,1) is > 20.

3. A display as claimed in one of claims 1 or 2, wherein, in (I),
X is -OC(=O)-, -OCH₂- or -OC(=O)CH₂CH₂-.

4. A display as claimed in one of claims 1 to 3, wherein, in (I),
B¹ is cyclohexane-1,4-diyl, cyclohex-1-ene-1,4-diyl, phenylene-1,4-diyl, unsubstituted, monosubstituted or disubstituted by F, or thiophene-2,5-diyl.

5. A display as claimed in one of claims 1 to 4, wherein, in (I),
A¹ is pyrimidine-2,5-diyl (unsubstituted or monosubstituted by F), pyridine-2,5-diyl (unsubstituted or monosubstituted by F), phenylene-1,4-diyl (unsubstituted, monosubstituted or disubstituted by F), or (1,3,4)-thiadiazol-2,5-diyl.

6. A display as claimed in one of claims 1 to 5, wherein the liquid-crystal mixture is composed of 3 to 30 compounds and comprises at least one compound of the formula (I) and at least one compound of the formula (II) below and, if desired, at least one compound of the formula (III) below 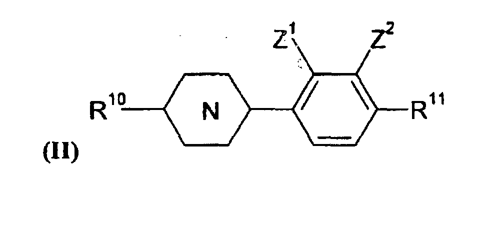 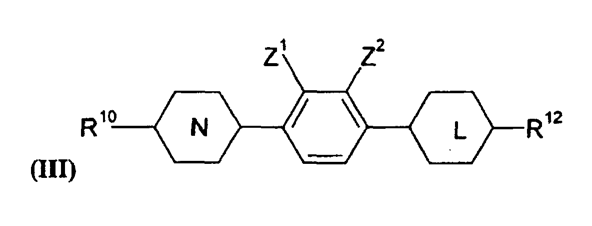 where:
R¹⁰, R¹¹ are as defined for R¹, R², where additionally the terminal -CH₃- group may in each case be replaced by one of the chiral groups (optically active or racemic) below: 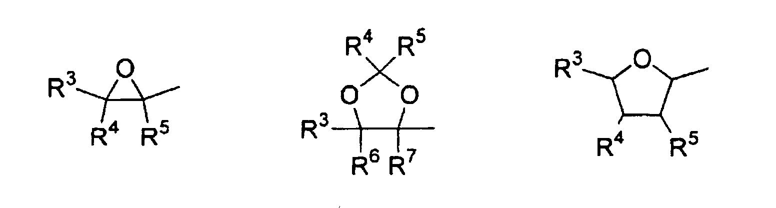 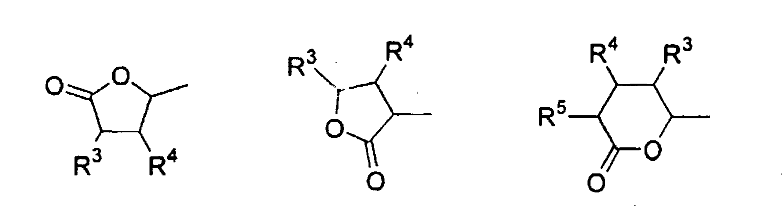 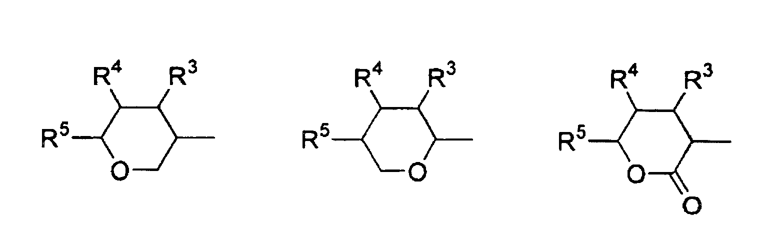 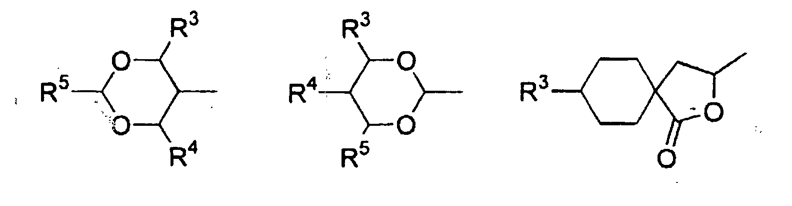 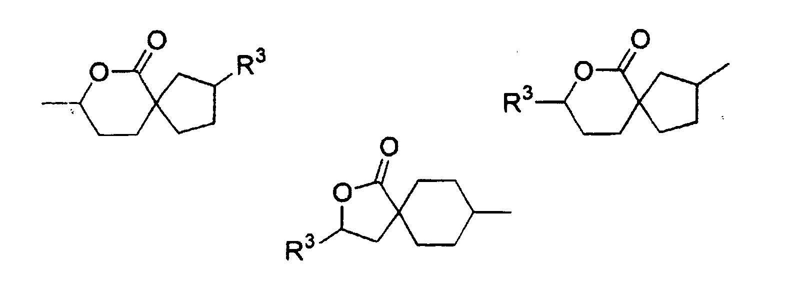 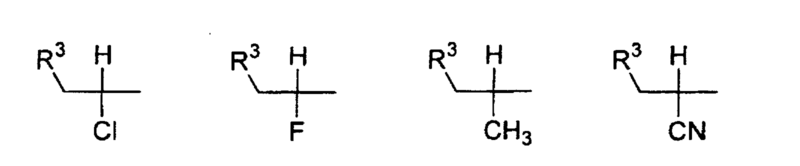 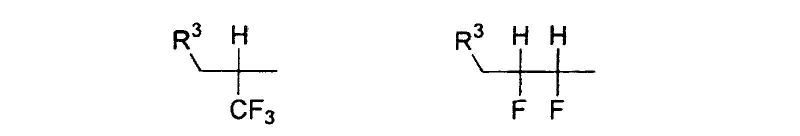
R³, R⁴, R⁵, R⁶, R⁷ are identical or different and are each
a) hydrogen
b) a straight-chain or branched alkyl radical (with or without asymmetric carbon atoms) having 1 to 16 carbon atoms, where
b1) one or more nonadjacent and nonterminal CH₂ groups may be replaced by -O- and/or
b2) one or two CH₂ groups may be replaced by -CH=CH-,
c) R⁴ and R⁵ together may alternatively be -(CH₂)₄- or -(CH₂)₅- if they are attached to an oxirane, dioxolane, tetrahydrofuran, tetrahydropyran, butyrolactone or valerolactone system;
R¹² is hydrogen or a straight-chain or branched alkyl radical (with or without asymmetric carbon atoms) having 1 to 16 carbon atoms, where one or more H may be replaced by F and one or two nonadjacent nonterminal -CH₂- groups may be replaced by -O-
Z¹, Z², Z³, Z⁴, Z⁵, Z⁶ are each, independently of one another, H or F 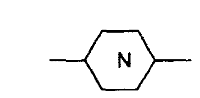 is a bivalent radical selected from the group consisting of pyridine-2,5-diyl, unsubstituted or monosubstituted by F, pyrimidine-2,5-diyl, unsubstituted or monosubstituted by F, pyrazine-2,5-diyl, unsubstituted or monosubstituted by F, 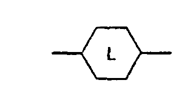 is a bivalent radical selected from the group consisting of cyclohexane-1,4-diyl, unsubstituted or monosubstituted by CN, CH₃, or disubstituted by F, cyclohex-1-ene-1,4-diyl, perfluorocyclohexane-1,4-diyl, cyclohex-2-ene-1,4-diyl, 1-alkyl-1-siiacyclohexane-1,4-diyl, bicyclo[2.2.2]octane-1,4-diyl.

7. A display as claimed in one of claims 1 to 5, wherein the liquid-crystal mixture is composed of 3 to 30 compounds and comprises at least one compound of the formula (I) and at least one compound of the formula (II) and at least one additional compound, selected from the group consisting of (III), (IV), (V), (VI), (VII), where the compounds of the formulae (II) and (III) are as defined in claim 6, 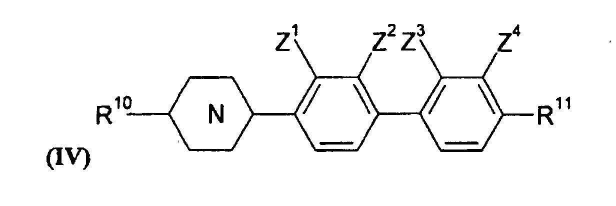 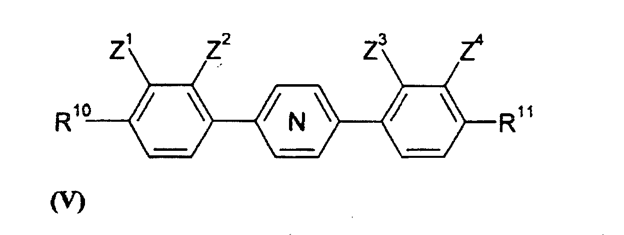 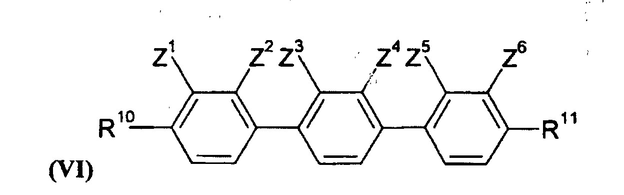 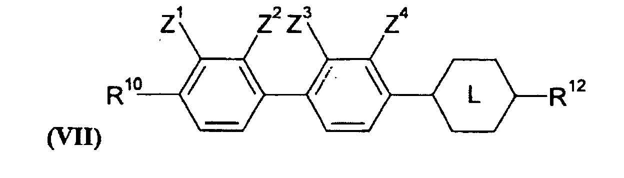 where the symbols and indices are as defined in claim 6.

8. A display as claimed in one of claims 1 to 7, wherein the liquid-crystal mixture is composed of 3 to 30 compounds and comprises at least one compound of the formula (I) and at least one compound of the formula (II) and at least one additional compound selected from the group consisting of (VIII), (IX), (X), (XI), (XII), (XIII), (XIV), (XV), (XVI), (XVII), where the compounds of the formulae (II) and (III) are as defined in claim 6, 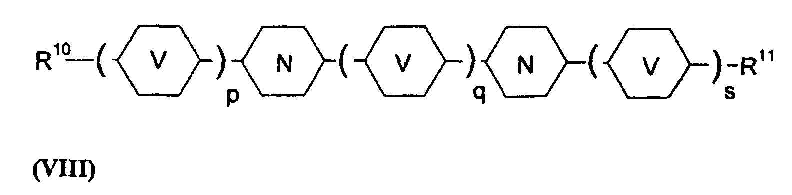 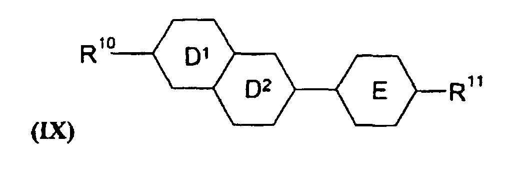 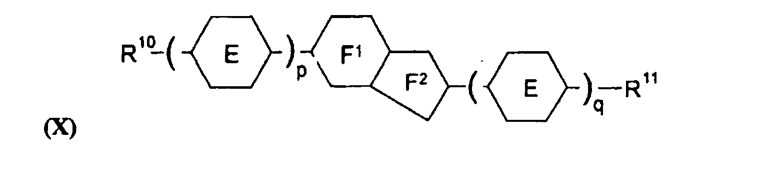 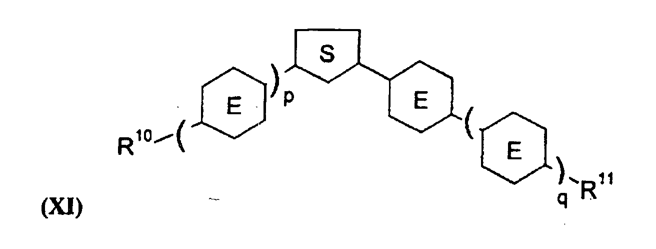 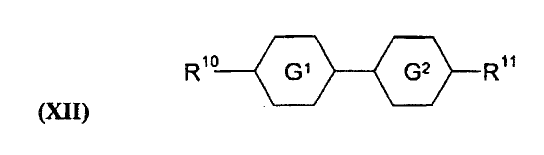 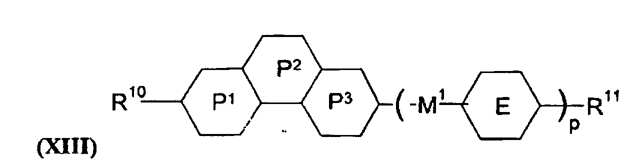 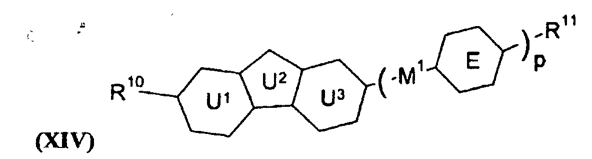 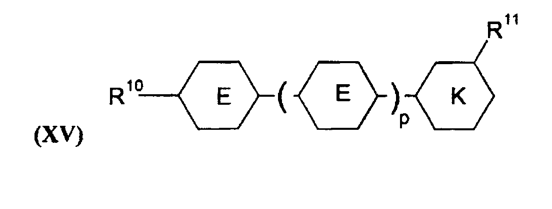 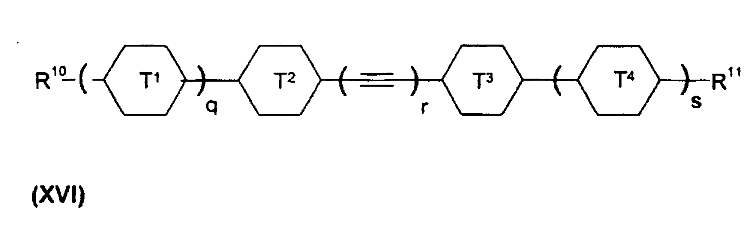 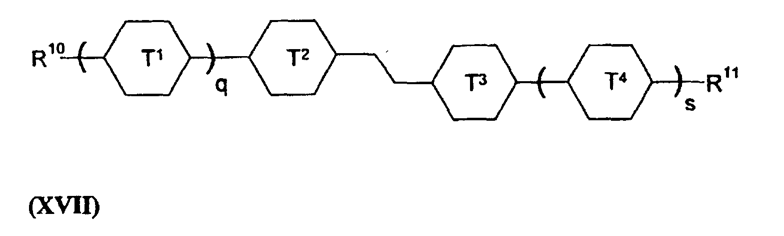 where the symbols and indices are as defined in claim 6 or as defined below: 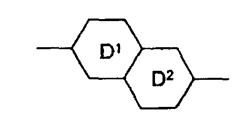 is a bivalent radical selected from the group consisting of naphthalene-2,6-diyl, in which one or two ring carbon atoms may be replaced by N and which can be monosubstituted or disubstituted by F or CN and in which D¹ or D² may also be a (saturated) alicycle 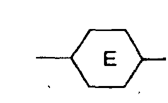 is a bivalent radical selected from the group consisting of phenylene-1,4-diyl, unsubstituted, monosubstituted or disubstituted by CN, or unsubstituted, monosubstituted, disubstituted, trisubstituted or tetrasubstituted by F, pyridine-2,5-diyl, unsubstituted or monosubstituted by F, pyrimidine-2,5-diyl, unsubstituted or monosubstituted by F, cyclohexane-1,4-diyl 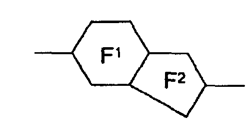 is a bivalent radical selected from the group consisting of indane-2,5-diyl, unsubstituted, monosubstituted or disubstituted by F in the aromatic ring, indan-1-one-2,6-diyl, unsubstituted, monosubstituted or disubstituted by F in the aromatic ring, benzothiazole-2,6-diyl, benzothiazole-2,5-diyl, benzo[b]-thiophene-2,5-diyl, benzo[b]thiophene-2,6-diyl 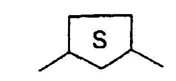 is a bivalent radical selected from the group consisting of (1,3,4)-thiadiazole-2,5-diyl, (1,3)-thiazole-2,5-diyl, thiophene-2,5-diyl, (1,3,4)-oxadiazole-2,5-diyl, (1,3)-oxazole-2,5-diyl, isoxazole-2,5-diyl 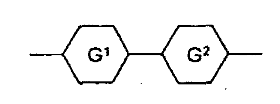 is a bivalent radical selected from the group consisting of 1,1'-biphenyl-4,4'-diyl, unsubstituted, monosubstituted or disubstituted by CN, or unsubstituted, monosubstituted, disubstituted, trisubstituted or tetrasubstituted by F, 1,1'-phenylcyclohexyl-4,4'-diyl, 5,5'-pyridylpyrimidine-2,2'-diyl, unsubstituted or monosubstituted by F in one or both of the heterocycles, 5,2'-pyridylpyrimidine-2,5'-diyl, unsubstituted or monosubstituted by F in one or both of the heterocycles, 1,2'-phenyldioxane-4,5'-diyl, 1,2'-(2-fiuorophenyl)dioxane-4,5'-diyl, 1,2'-(3-fluorophenyl)dioxane-4,5'-diyl, 1,2'-(2,3-difluorophenyl)dioxane-4,5'-diyl 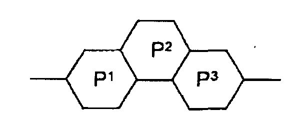 is a bivalent phenanthrene-2,7-diyl radical in which one or two ring carbon atoms may be replaced by N and which may be monosubstituted, disubstituted, trisubstituted or tetrasubstituted by F and in which P² and/or P³ may be a (saturated) alicycle 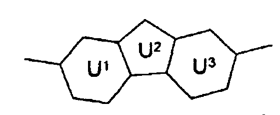 is a bivalent fluorene-2,7-diyl radical in which the -CH₂- group in U² may be replaced by -C(=O)-, -CHF- or -CF₂- 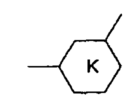 is a bivalent radical selected from the group consisting of phenylene-1,3-diyl, unsubstituted, monosubstituted or disubstituted by F, cyclohexane-1,3-diyl, unsubstituted or monosubstituted by F or CN, pyridine-2,6-diyl, pyridine-2,4-diyl, pyridine-3,5-diyl, pyridine-4,6-diyl, pyrimidine-4,6-diyl, 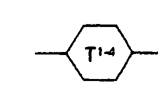 is a bivalent radical selected from the group consisting of phenylene-1,4-diyl, unsubstituted, monosubstituted or disubstituted by CN or F, naphthalene-2,6-diyl, in which one or two ring carbon atoms may be replaced by N and which may be monosubstituted or disubstituted by CN or F, cyclohexane-1,4-diyl, cyclohex-1-ene-1,4-diyl, bicyclo[2.2.2]octane-1,4-diyl, (1,3)-dioxane-2,5-diyl, pyridine-2,5-diyl, unsubstituted or monosubstituted by F, pyrimidine-2,5-diyl, unsubstituted or monosubstituted by F, (1,3,4)-thiadiazole-2,5-diyl, indane-2,5-diyl, unsubstituted, monosubstituted or disubstituted by F in the aromatic ring, thiophene-2,5-diyl 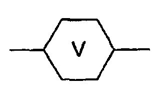 is a bivalent radical selected from the group consisting of phenylene-1,4-diyl, unsubstituted, monosubstituted or disubstituted by CN or F, naphthalene-2,6-diyl, in which one or two ring carbon atoms may be replaced by N and which may be monosubstituted or disubstituted by CN or F, cyclohexane-1,4-diyl, cyclohex-1-ene-1,4-diyl, bicyclo[2.2.2]octane-1,4-diyl, (1,3)-dioxane-2,5-diyl, indane-2,5-diyl, unsubstituted, monosubstituted or disubstituted by F in the aromatic ring, thiophene-2,5-diyl
p, q, s are each zero or 1
r is 1 or 2.

9. A chiral smectic liquid-crystal mixture as claimed in one of claims 1 to 6, comprising from 10 to 60% of one or more compounds of the formula (I).

10. A chiral smectic liquid-crystal mixture as claimed in claim 6, comprising from 10 to 60% of 1 to 15 compounds of the formula (I) and from 40 to 90% of 2 to 15 compounds of the formula (II).

11. A compound of the general formula (I) as claimed in claim 1, selected from compounds of the formula (XXIII), where: 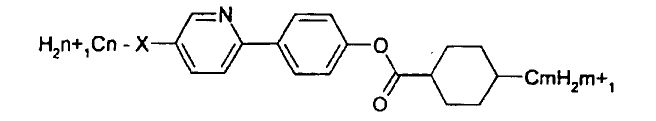 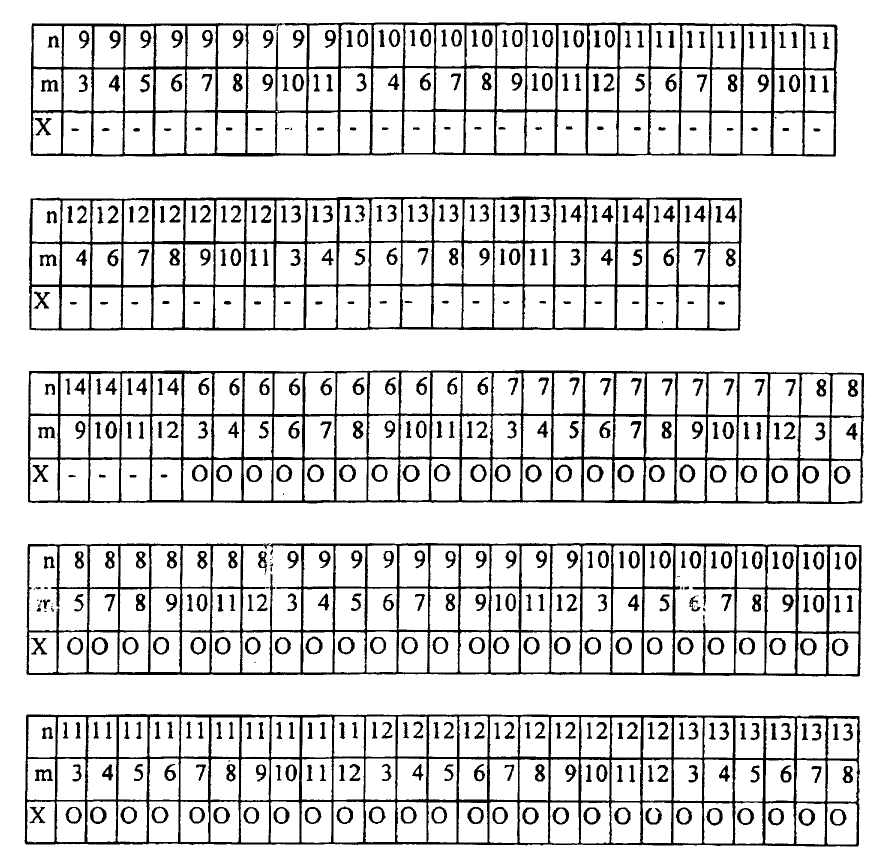 compounds of the formula (XXIV), where: 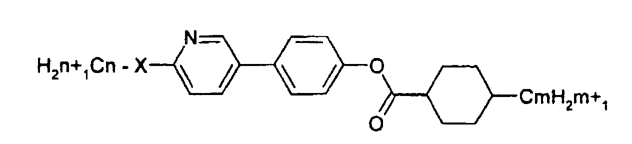
n is an integer from 8 to 14
m is an integer from 3 to 11
X is a single bond
with the exception of n=11, m=3 or 5, X is a single bond,
compounds of the formula (XXV), where: 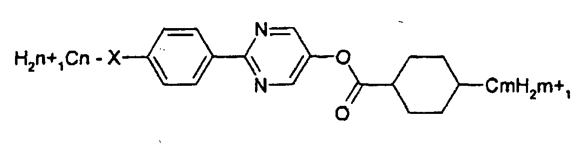
n is an integer from 2 to 13
m is an integer from 3 to 11
X is O or a single bond
with the exception of n=2, m=11, X=O; n=5, m=5, X=O,
compounds of the formula (XXVI), where: 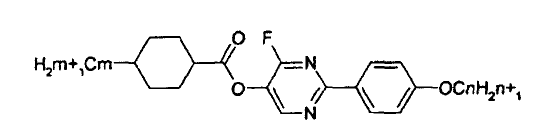
n is an integer from 5 to 13
m is an integer from 3 to 10
with the exception of n=8, m=5,
compounds of the formula (XXVII), where: 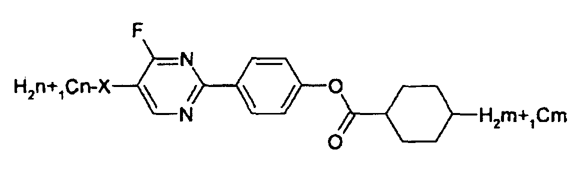 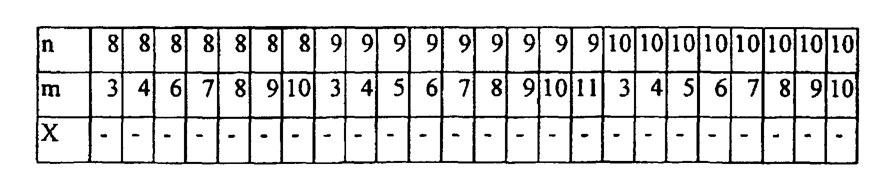 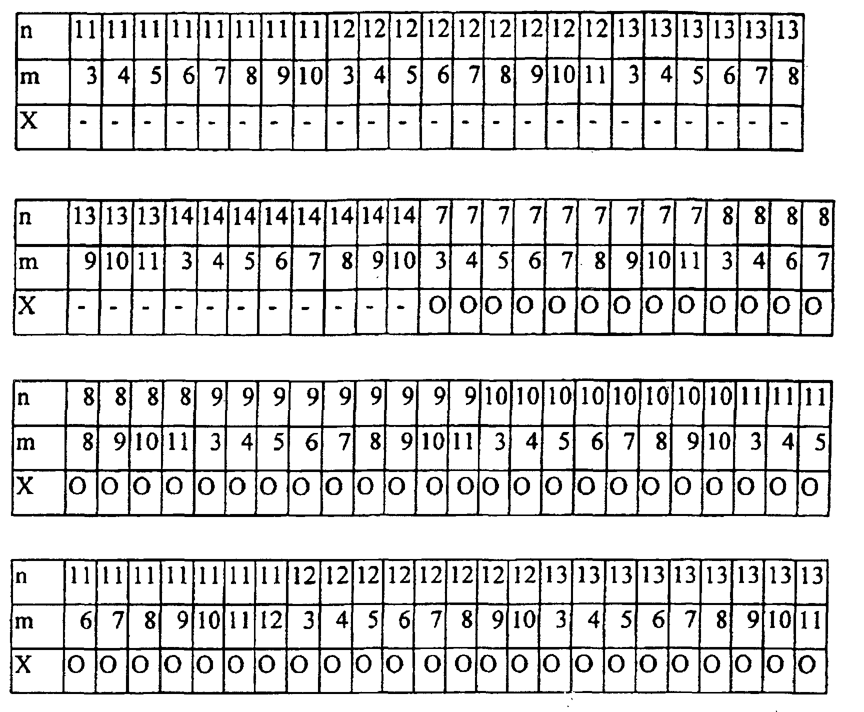 compounds of the formula (XXIX), where: 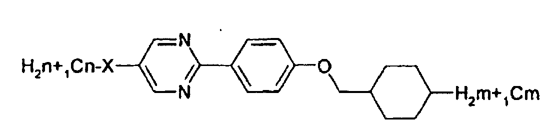 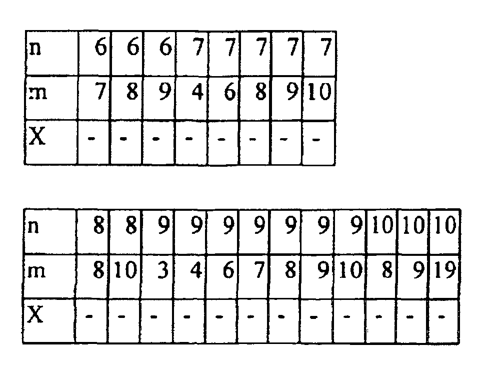 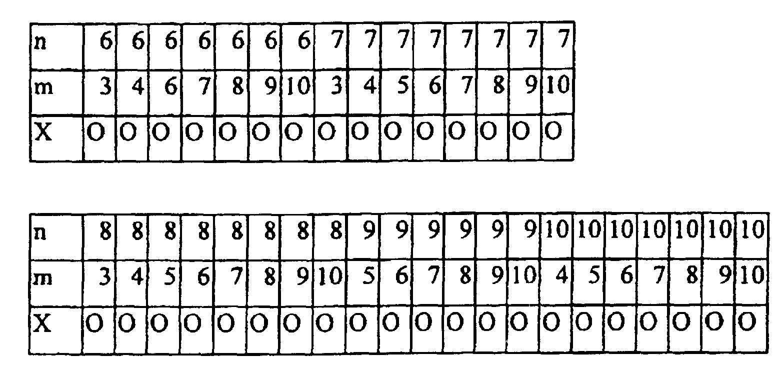 compounds of the formula (XXX), where: 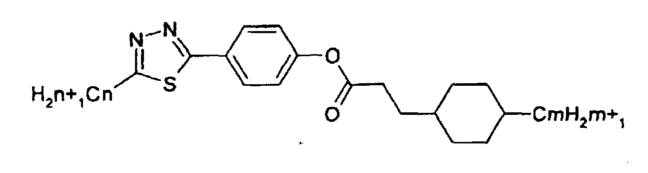
n is an integer from 5 to 13
m is an integer from 3 to 10
with the exception of n=8, m=4; n=9, m=3.

12. A compound of the general formula (II) as claimed in claim 6, selected from compounds of the formula (XXXI), where: 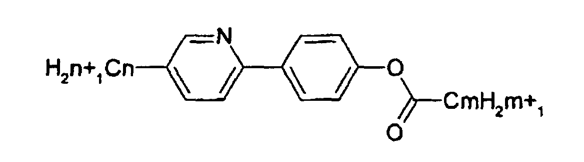 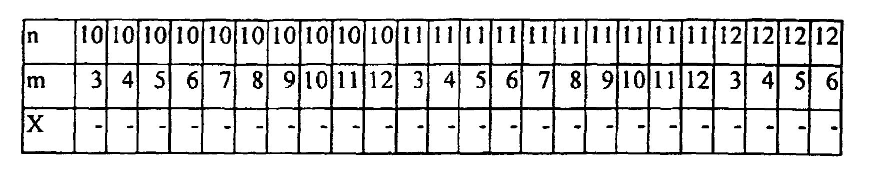 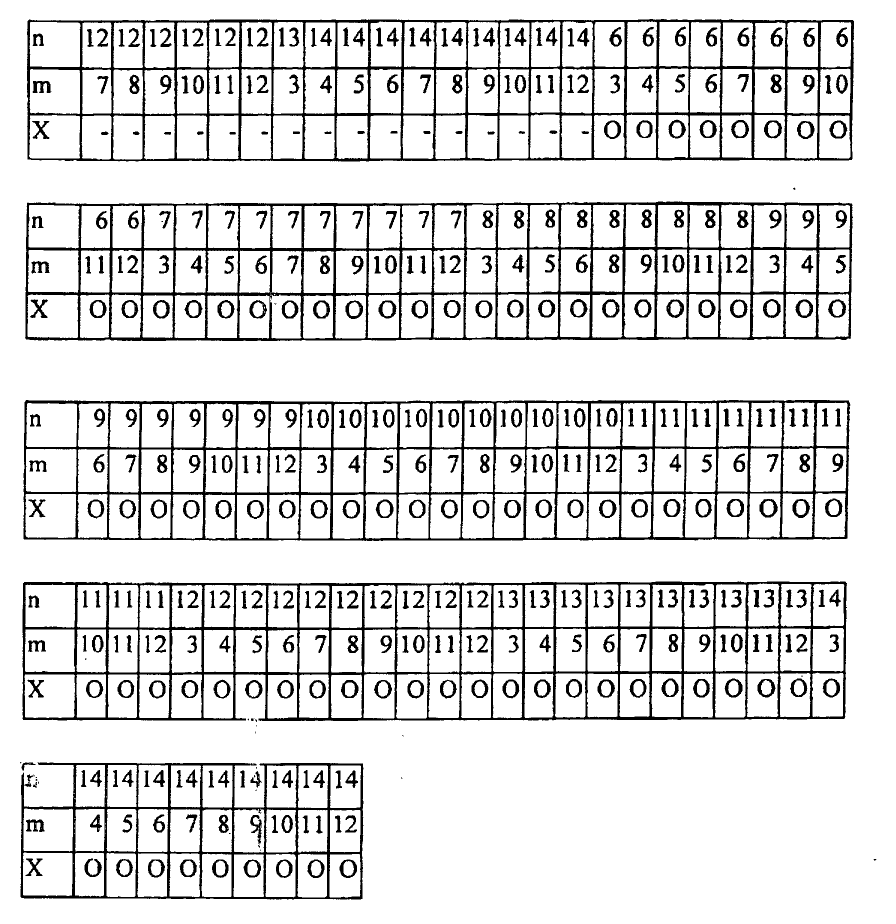 compounds of the formula (XXVIII), where: 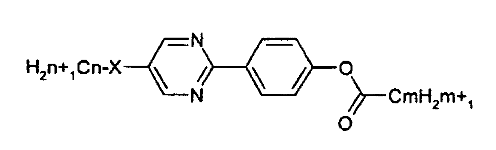 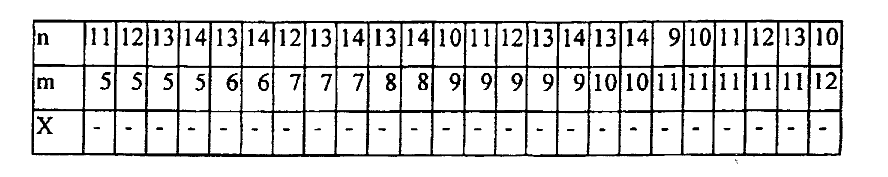 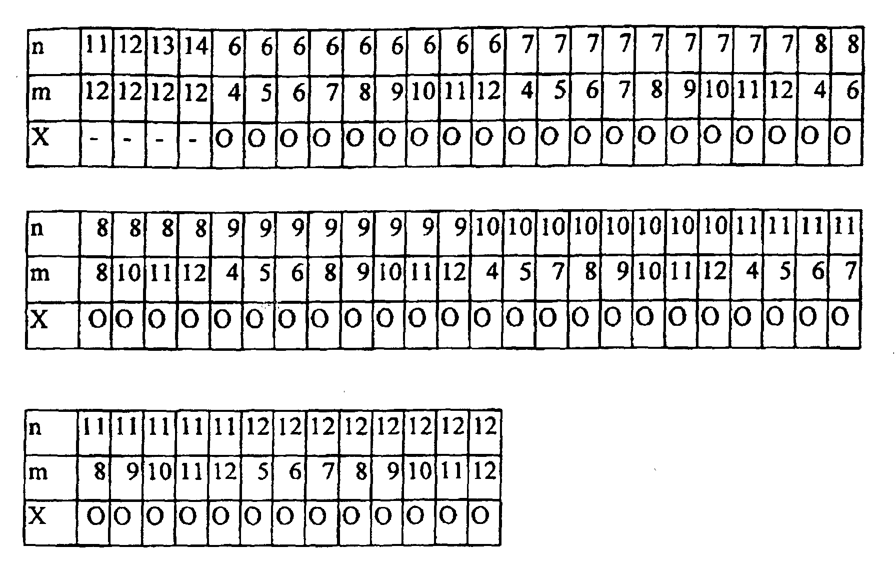 compounds of the formula (XXXII), where: 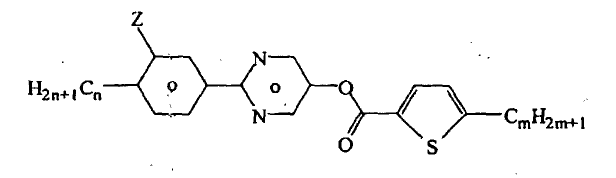 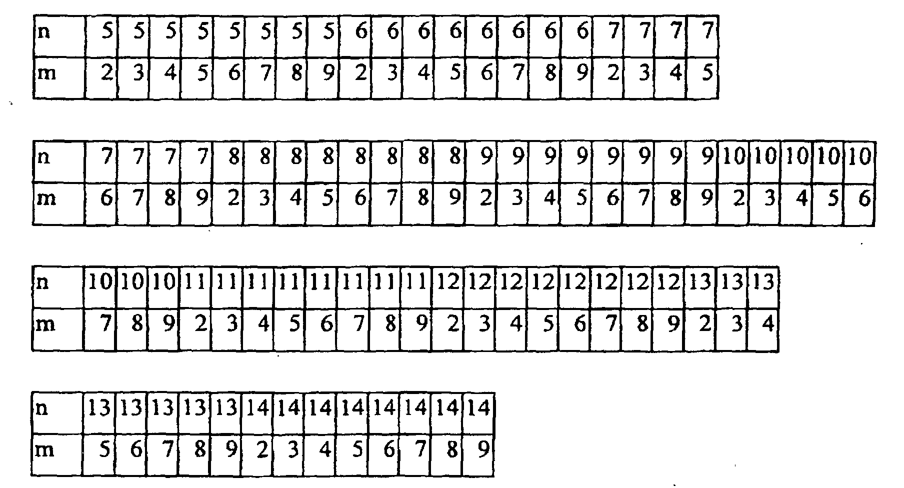 and where Z is H or F in all cases.

## Revendications

1. Afficheur à matrice active renfermant un mélange de cristaux liquides (chiral smectique) ferroélectrique qui contient au moins un composé de formule (I)
R¹-(A¹-M¹)ₐ-(A²-M²)_{b}-A³-X-B¹-(B²)_{c}-R² (I)
où les symboles possèdent les significations suivantes :
R¹, R², indépendamment l'un de l'autre, identiques ou différents, représentent
a) un atome d'hydrogène, un atome de fluor ou un groupe CN
un reste alcényle, alcényloxy, alkyle ou alkyloxy (avec ou sans atomes de carbone asymétriques) linéaire ou ramifié, présentant 2 à 16 atomes de carbone, où
b1) un ou deux groupes -CH₂- non terminaux peuvent être remplacés par des groupes -O-, -OC(=O)-, -(C=O), -C(=O)O-, -Si(CH₃)₂-, -CH(Cl)- et/ou un ou deux groupes -CH₂- peuvent être remplacés par un groupe -CH=CH- ou -C≡C-
et où aussi un ou plusieurs atomes d'hydrogène peuvent être remplacés par F, et/ou
b2) un ou plusieurs groupes -CH₂- peuvent être remplacés par un groupe phénylène-1,4-diyle (éventuellement substitué 1 ou 2 fois par F), phénylène-1,3-diyle (éventuellement substitué 1 ou 2 fois par F), cyclohexane-1,4-diyle (éventuellement substitué 1 fois par F ou CN) ou cyclopropane-1,2-diyle,
et où aussi un ou plusieurs atomes d'hydrogène peuvent être remplacés par F,
à condition que seulement un des restes R¹, R² puisse représenter un atome d'hydrogène, un atome de F ou un groupe CN et deux groupes -CH₂- voisins ne puissent pas être remplacés par -O-,
M¹, M², indépendamment l'un de l'autre, identiques ou différents, représentent un groupe
-C(=O)O, -OC(=O)-, -CH₂O-, -OCH₂, -CF₂O-, -OCF₂-, -(CH₂-CH₂-, -CF₂-CF₂-, -CH=CH-, -CH=CF-, -CF=CF-, -C≡C-, -CH₂-CH₂C(=O)O-, -OC(=O)CH₂-CH₂-, -(CH₂)₄-, - OCH₂CH₂CH₂-, -CH₂CH₂CH₂O-, -OCH₂CF₂CH₂-, - CH₂CF₂CH₂O- ou une liaison simple,
A1, A2, A3, indépendamment l'un de l'autre, identiques ou différents, représentent un groupe cyclohexane-1,4-diyle (éventuellement substitué 1 fois par F, CH₃, CN), cyclohex-1-ène-1,4-diyle, cyclohex-2-ène-1,4-diyle, 2-oxocyclohexane-1,4-diyle, 2-cyclohexène-1-one-3,6-diyle, 1-alkyl-1-sila-cyclohexane-1,4-diyle, bicyclo[2.2.2]octane-1,4-diyle, spiro[4.5]décane-2,8-diyle, spiro[5.5]undécane-3,9-diyle, phénylène-1,4-diyle (éventuellement substitué 1 ou 2 fois par un substituant CN, CH₃, CF₃, OCF₃, éventuellement substitué 1, 2, 3 ou 4 fois par F), phénylène-1,3-diyle (éventuellement substitué 1 ou 2 fois par un substituant CN, CH₃, CF₃, OCF₃, éventuellement substitué 1, 2, 3 ou 4 fois par F), thiophène-2,5-diyle, thiophène-2,4-diyle, (1,3,4)-oxadiazole-2,5-diyle, (1,3,4)-thiadiazole-2,5-diyle, 1,3-thiazol-2,5-diyle, 1,3-thiazole-2,4-diyle, (1,3)-oxazol-2,5-diyle, isoxazol-2,5-diyle, indane-2,6-diyle, naphtalène-2,6-diyle (éventuellement substitué 1 ou 2 fois par F ou CN), 1,2,3,4-tétrahydronaphtalène-2,6-diyle, décaline-2,6-diyle, pyrimidine-2,5-diyle (éventuellement substitué 1 fois par F), pyridine-2,5-diyle (éventuellement substitué 1 ou 2 fois par F), pyrazine-2,5-diyle (éventuellement substitué 1 fois par F), pyridazine-3,6-diyle, quinoléine-2,6-diyle, quinoléine-3,7-diyle, isoquinoléine-3,7-diyle, quinazoline-2,6-diyle, 5,6,7,8-tétrahydroquinazoline-2,6-diyle, quinoxaline-2,6-diyle, 1,3-dioxanne-2,5-diyle (éventuellement substitué 1 fois par un substituant CN), benzothiazole-2,6-diyle, pipéridine-1,4-diyle, pipérazine-1,4-diyle
B1 représente un groupe cyclohexane-1,4-diyle (éventuellement substitué 1 ou 2 fois par un substituant F, CH₃, CN), perfluorocyclohexane-1,4-diyle, cyclohex-1-ène-1,4-diyle, cyclohex-2-ène-1,4-diyle, 1-alkyl-1-sila-cyclohexane-1,4-diyle, bicyclo[2.2.2]octane-1,4-diyle, cyclopentane-1,3-diyle, cycloheptane-1,4-diyle, tétrahydrofuranne-2,5-diyle, tétrahydrofuranne-2,4-diyle, phénylène-1,4-diyle (éventuellement substitué 1 ou 2 fois par un substituant CN, CH₃, CF₃, OCF₃, éventuellement substitué 1, 2, 3 ou 4 fois par F), phénylène-1,3-diyle (éventuellement substitué 1 ou 2 fois par un substituant CN, CH₃, CF₃, OCF₃, éventuellement substitué 1, 2 ou 3 fois par F), thiophène-2,5-diyle (éventuellement substitué 1 fois par F), thiophène-2,4-diyle (éventuellement substitué 1 fois par F), 1,3-thiazole-2,5-diyle (éventuellement substitué 1 fois par F), 1,3-thiazole-2,4-diyle (éventuellement substitué 1 fois par F), (1,3,4)-thiadiazole-2,5-diyle, 1,3-dioxanne-2,5-diyle (éventuellement substitué 1 fois par un substituant CN), tétrahydropyrane-2,5-diyle, 6,6-difluoro-tétrahydropyrane-2,5-diyle, 6,6-difluoro-2,3-dihydro-6H-pyrane-2,5-diyle, 6-fluoro-3,4-dihydro-2H-pyrane-2,5-diyle, pipéridine-1,4-diyle, pipérazine-1,4-diyle, pyrimidine-2,5-diyle (éventuellement substitué 1 fois par F), pyridine-2,5-diyle (éventuellement substitué 1 fois par F), 1,2,3,4-tétrahydronaphtalène-2,6-diyle, décaline-2,6-diyle,
B² représente un groupe cyclohexane-1,4-diyle (éventuellement substitué 1 ou 2 fois par un substituant F, CH₃, ON), cyclohex-1-ène-1,4-diyle (éventuellement substitué 1 fois par F), cyclohex-2-ène-1,4-diyle, 1-alkyl-1-silacyclohexane-1,4-diyle, bicycle[2.2.2]octane-1,4-diyle, phénylène-1,4-diyle (éventuellement substitué 1 ou 2 fois par un substituant CN, CH₃, CF₃, OCF₃, éventuellement substitué 1, 2, 3 ou 4 fois, par F), phénylène-1,3-diyle (éventuellement substitué 1 ou 2 fois par un substituant CN, CH₃, CF₃, OCF₃, éventuellement substitué 1, 2 ou 3 fois par F), thiophène-2,5-diyle, thiophène-2,4-diyle, 1,3-thiazole-2,5-diyle, 1,3-thiazole-2,4-diyle, (1,3,4)-thiadiazole-2,5-diyle, 1,3-dioxanne-2,5-diyle (éventuellement substitué 1 fois par un substituant CN), tétrahydrofuranne-2,5-diyle, tétrahydropyrane-2,5-diyle, 6,6-difluorotétrahydropyrane-2,5-diyle, 6,6-difluoro-2,3-dihydro-6H-pyrane-2,5-diyle, 6-fluoro-3,4-dihydro-2H-pyrane-2,5-diyle, pyrimidine-2,5-diyle (éventuellement substitué 1 fois par F), pyridine-2,5-diyle (éventuellement substitué 1 fois par F), indane-2,6-diyle, pipéridine-1,4-diyle, pipérazine-1,4-diyle, pyrimidine-2,5-diyle (éventuellement substitué 1 fois par F)
X représente un groupe -(CH₂)ₙ-, où
a) un ou deux groupes -CH₂- peuvent être remplacés par -O- ou -C(=O)- et/ou
b) un groupe -CH₂-CH₂- peut être remplacé par -CH=CH- et un ou plusieurs H des groupes -CH₂- peuvent être remplacés par F,
à condition que
1) n soit 2, 3 ou 4
2) deux groupes -CH₂- voisins ne puissent pas être remplacés par -O-,
a, b, c valent zéro, 1 ou 2, à condition que
1) a soit 1, lorsque R¹ représente un atome d'hydrogène, F ou un groupe CN,
2) la somme a+b+c soit au moins 1,
3) les restes A ou M entre_parenthèses puissent avoir des significations différentes ou identiques, lorsque l'index correspondant est 2,
dans une couche de cristaux liquides sous forme d'un monodomaine ayant une direction exactement définie des normales à la couche z de la phase SmC*, les normales à la couche z et la direction préférentielle n de la phase nématique ou cholestérique (phase N*) forment un angle supérieur à 5°.

2. Afficheur selon la revendication 1, **caractérisé en ce que** le mélange de cristaux liquides présente une polarisation spontanée <200 nC/cm² et DT(15,1) > 20.

3. Afficheur selon l'une des revendications 1 et 2, **caractérisé en ce que** dans (I)
X représente -OC(=O)-, -OCH₂- ou -OC(=O)CH₂CH₂-.

4. Afficheur selon l'une des revendications 1 à 3, **caractérisé en ce que** dans (I)
B1 représente un groupe cyclohexane-1,4-diyle, cyclohex-1-ène-1,4-diyle, phénylène-1,4-diyle, éventuellement substitué une ou deux fois par F, ou thiophène-2,5-diyle.

5. Afficheur selon l'une des revendications 1 à 4, **caractérisé en ce que** dans (I)
A1 représente un groupe pyrimidine-2,5-diyle (éventuellement substitué une fois par F), pyridine-2,5-diyle (éventuellement substitué une fois par F), phénylène-1,4-diyle (éventuellement substitué une ou deux fois par F) ou (1,3,4)-thiadiazole-2,5-diyle.

6. Afficheur selon l'une des revendications 1 à 5, **caractérisé en ce que** le mélange de cristaux liquides est constitué par 3 à 30 composés et renferme au moins un composé de formule (I) et au moins un composé de formule (II) ci-après et éventuellement au moins un composé de formule (III) ci-après 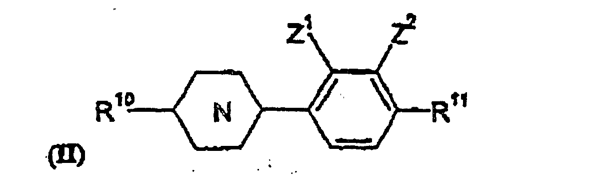 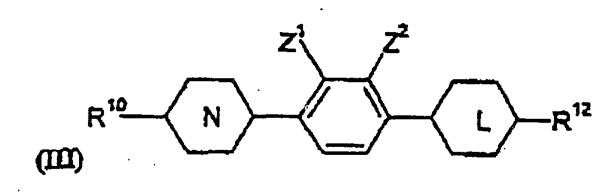 où
R¹⁰, R¹¹ sont comme R¹, R², de plus le groupes terminal -CH₃ peut être remplacé par un des groupes chiraux (optiquement actifs ou racémiques) suivants 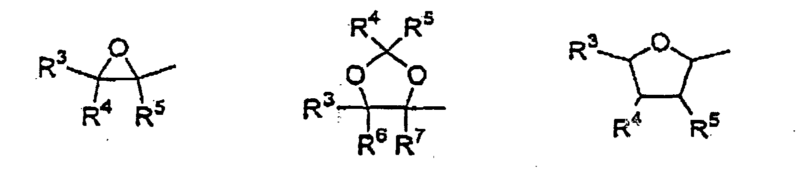 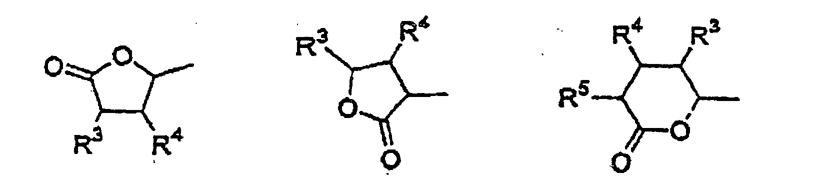 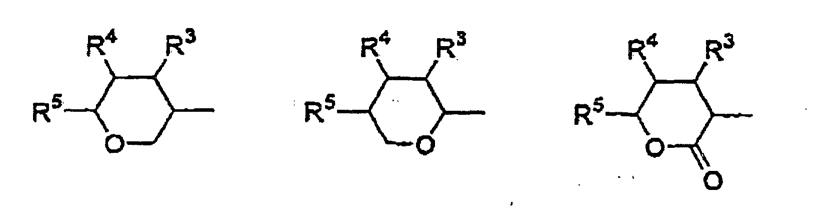 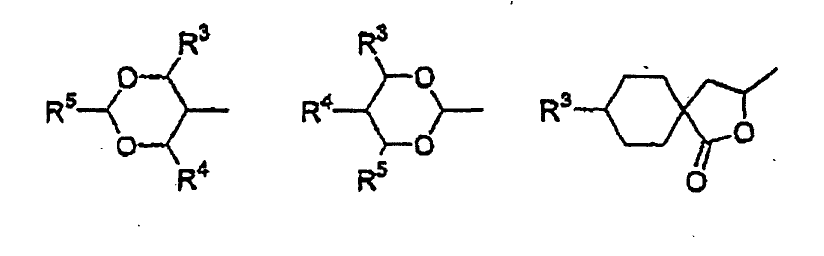 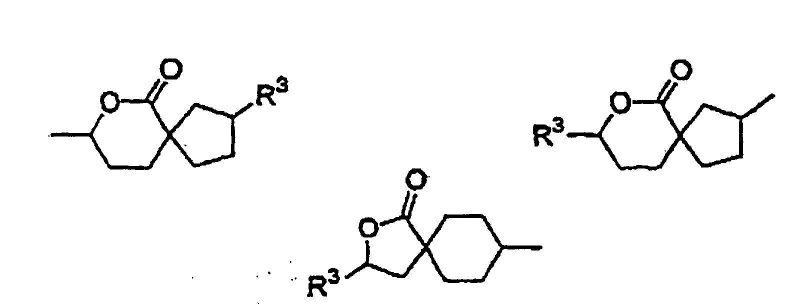 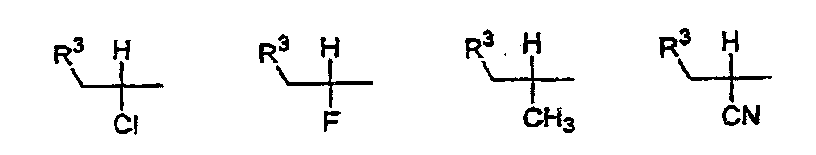 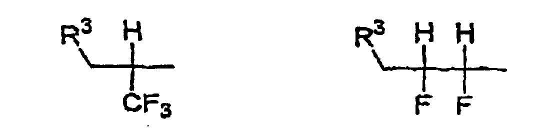 R³, R⁴, R⁵, R⁶, R⁷, identiques ou différents, représentent
a) un atome d'hydrogène,
b) un reste alkyle linéaire ou ramifié (avec ou sans atomes de carbone asymétriques) présentant 1 à 16 atomes de carbone, où
b1) un ou plusieurs groupes -CH₂- non voisins et non terminaux peuvent être remplacés par -O- et/ou
b2) un ou deux groupes -CH₂- peuvent être remplacés par -CH=CH-,
c) R⁴ et R⁵ conjointement forment aussi -(CH₂)₄- ou -(CH₂)₅-, lorsqu'ils sont liés à un système oxirane, dioxolane, tétrahydrofuranne, tétrahydropyrane, butyrolactone ou valérolactone ;
R¹² représente un atome d'hydrogène ou un reste alkyle linéaire ou ramifié (avec ou sans atomes de carbone asymétriques) présentant 1 à 16 atomes de carbone, dans lequel un ou plusieurs atomes de H peuvent aussi être remplacés par F et dans lequel un ou deux groupes -CH₂- non voisins, non terminaux, peuvent être remplacés par -O-,
Z¹, Z², Z³, Z⁴, Z⁵, Z⁶ indépendamment les uns des autres représentent H ou F 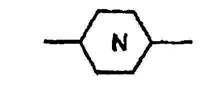 représente un reste bivalent pris dans le groupe comprenant pyridine-2,5-diyle, éventuellement substitué une fois par F, pyrimidine-2,5-diyle, éventuellement substitué une fois par F, pyrazine-2,5-diyle, éventuellement substitué une fois par F, 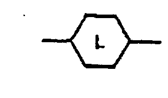 représente un reste bivalent pris dans le groupe comprenant cyclohexane-1,4-diyle, éventuellement substitué une fois par CN, CH₃ ou deux fois par F, cyclohex-1-ène-1,4-diyle, perfluorocyclohexane-1,4-diyle, cyclohex-2-ène-1,4-diyle, 1-alkyl-1-sila-cyclohexane-1,4-diyle, bicyclo[2.2.2]octane-1,4-diyle.

7. Afficheur selon l'une des revendications 1 à 5, **caractérisé en ce que** le mélange de cristaux liquides est constitué par 3 à 30 composés et renferme au moins un composé de formule (I) et au moins un composé de formule (II) et au moins un autre composé pris dans le groupe comprenant (III), (IV), (V), (VI), (VII), les composés de formules (II) et (III) sont définis comme à la revendication 6, 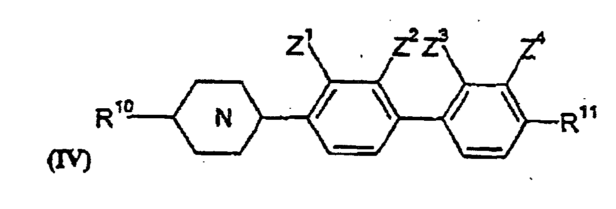 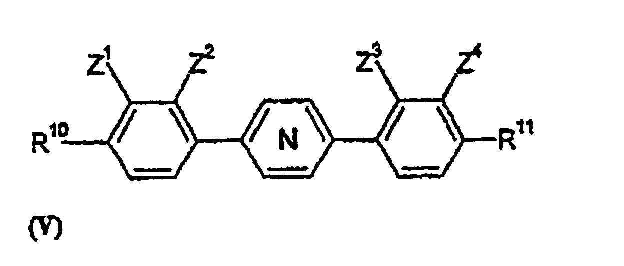 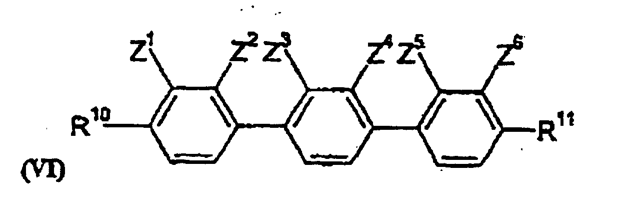 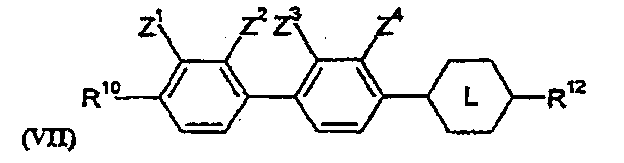 les symboles et les indices possèdent les significations données à la revendication 6.

8. Afficheur selon l'une des revendications 1 à 7, **caractérisé en ce que** le mélange de cristaux liquides est constitué par 3 à 30 composés et renferme au moins un composé de formule (I) et au moins un composé de formule (II) et au moins un autre composé pris dans le groupe comprenant (VIII), (IX), (X), (XI), (XII), (XIII), (XIV), (XV), (XVI), (XVII), les composés de formules (II) et (III) sont définis comme à la revendication 6, 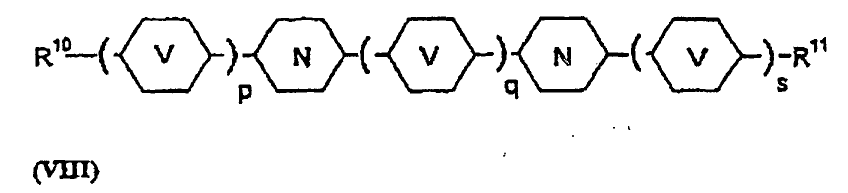 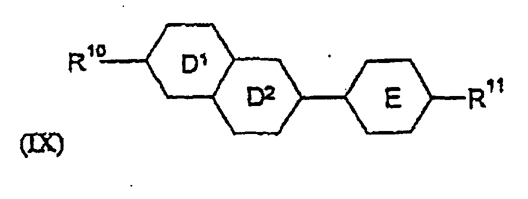 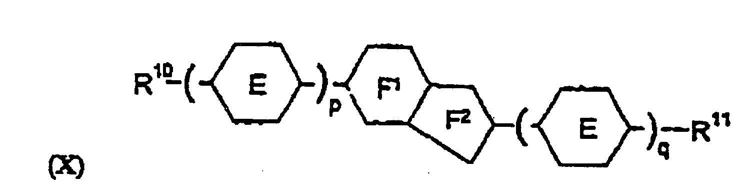 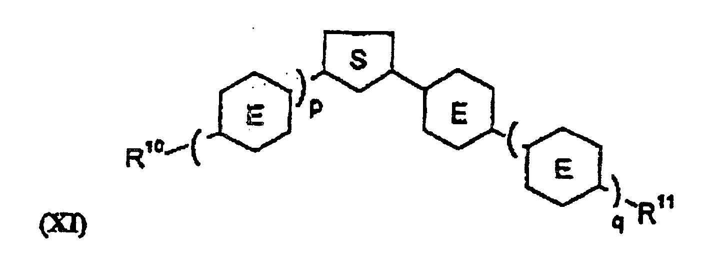 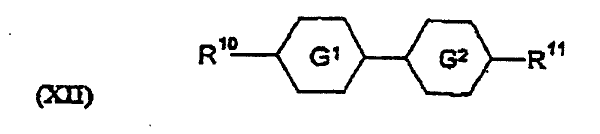 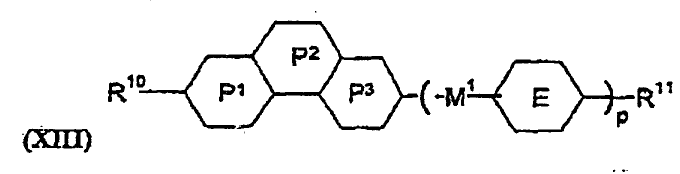 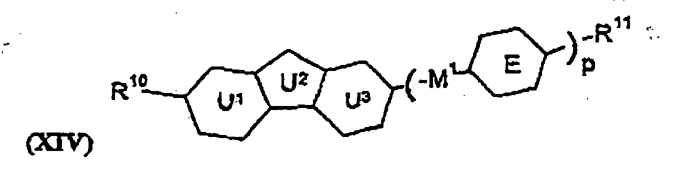 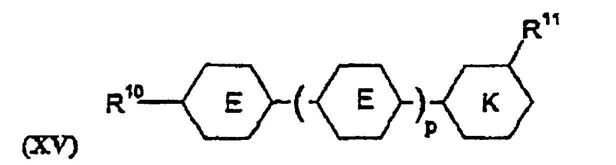 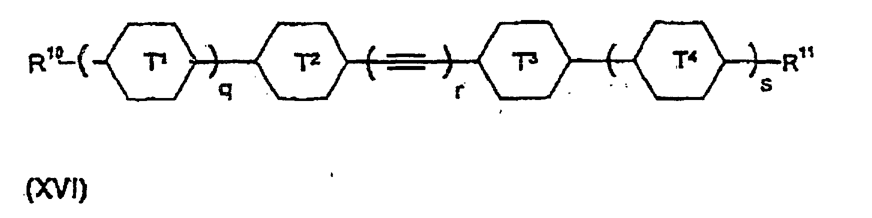 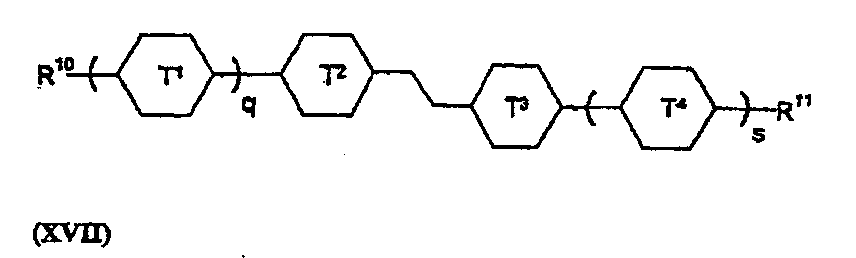 les symboles et les indices possèdent les significations données à la revendication 6 ou données ci-après : 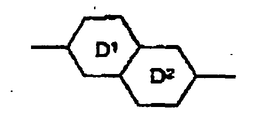 représente un reste bivalent pris dans le groupe comprenant naphtalène-2,6-diyle, où aussi un ou deux atomes de carbone nucléaires peuvent être remplacés par N et qui peut aussi être substitué une ou deux fois par F ou CN et où D¹ ou D² peuvent représenter un alicycle (saturé) 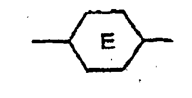 représente un reste bivalent pris dans le groupe comprenant phénylène-1,4-diyle, éventuellement substitué une ou deux fois par CN ou substitué une fois, deux fois, trois fois ou quatre fois par F, pyridine-2,5-diyle, éventuellement substitué une fois par F, pyrimidine-2,5-diyle, éventuellement substitué une fois par F, cyclohexane-1,4-diyle, 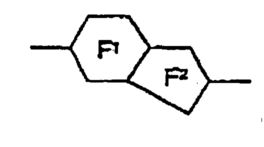 représente un reste bivalent pris dans le groupe comprenant indane-2,5-diyle, éventuellement substitué dans le cycle aromatique une ou deux fois par F, indan-1-one-2,6-diyle, éventuellement substitué dans le cycle aromatique une ou deux fois par F, benzothiazole-2,6-diyle, benzothiazole-2,5-diyle, benzo[b]thiophène-2,5-diyle, benzo[b]thiophène-2,6-diyle, 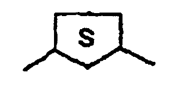 représente un reste bivalent pris dans le groupe comprenant (1,3,4)-thiadiazole-2,5-diyle, (1,3)-thiazole-2,5-diyle, thiophène-2,5-diyle, (1,3,4)-oxadiazole-2,5-diyle, (1,3)-oxazole-2,5-diyle, isoxazole-2,5-diyle, 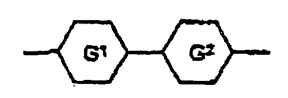 représente un reste bivalent pris dans le groupe comprenant 1,1'-biphényl-4,4'-diyle, éventuellement substitué une fois ou deux fois par CN ou substitué une fois, deux fois, trois fois ou quatre fois par F, 1,1'-phénylcyclohexyl-4,4'-diyle, 5,5'-pyridyl-pyrimidine-2,2'-diyle, éventuellement substitué une fois par F dans un ou les deux hétérocycles, 5,2'-pyridylpyrimidine-2,5'-diyle, éventuellement substitué une fois par F dans un ou les deux hétérocycles, 1,2'-phényldioxanne-4,5'-diyle, 1,2'-(2-fluorophényl)-dioxanne-4,5'-diyle, 1,2'-(3-fluorophényl)dioxanne-4,5'-diyle, 1,2'-(2,3-difluorophényl)dioxanne-4,5'-diyle, 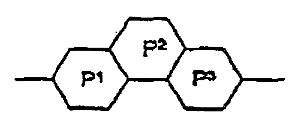 représente un reste bivalent phénanthrène-2,7-diyle, dans lequel un ou deux atomes de carbone nucléaires peuvent aussi être remplacés par N et qui peut être substitué une fois, deux fois, trois fois ou quatre fois par F, et P² et/ou P³ peuvent aussi représenter un alicycle (saturé), 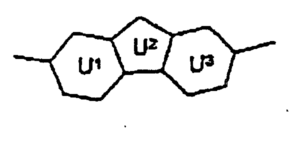 représente un reste bivalent fluorène-2,7-diyle, dans lequel le groupe -CH₂- dans U² peut aussi être remplacé par -C(=O)-, - CHF- ou -CF₂-, 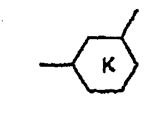 représente un reste bivalent pris dans le groupe comprenant phénylène-1,3-diyle, éventuellement substitué une fois ou deux fois par F, cyclohexane-1,3-diyle, éventuellement substitué une fois par un substituant F ou CN, pyridine-2,6-diyle, pyridine-2,4-diyle, pyridine-3,5-diyle, pyridine-4,6-diyle, pyrimidine-4,6-diyle, 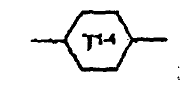 représente un reste bivalent pris dans le groupe comprenant phénylène-1,4-diyle, éventuellement substitué une fois ou deux fois par un substituant CN ou F, naphtalène-2,6-diyle, pour lequel un ou deux atomes de carbone nucléaires peuvent aussi être remplacés par N et qui est éventuellement substitué une ou deux fois par un substituant CN ou F, cyclohexane-1,4-diyle, cyclohex-1-ène-1,4-diyle, bicyclo[2.2.2]octane-1,4-diyle, (1,3)-dioxanne-2,5-diyle, pyridine-2,5-diyle, éventuellement substitué une fois par F, pyrimidine-2,5-diyle, éventuellement substitué une fois par F, (1,3,4)-thiadiazole-2,5-diyle, indane-2,5-diyle, éventuellement substitué dans le cycle aromatique une ou deux fois par F, thiophène-2,5-diyle, 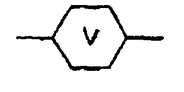 représente un reste bivalent pris dans le groupe comprenant phénylène-1,4-diyle, éventuellement substitué une fois ou deux fois par un substituant CN ou F, naphtalène-2,6-diyle, pour lequel un ou deux atomes de carbone nucléaires peuvent aussi être remplacés par N et qui est éventuellement substitué une ou deux fois par un substituant CN ou F, cyclohexane-1,4-diyle, cyclohex-1-ène-1,4-diyle, bicyclo[2.2.2]octane-1,4-diyle, (1,3)-dioxanne-2,5-diyle, indane-2,5-diyle, éventuellement substitué dans le cycle aromatique une ou deux fois par F, thiophène-2,5-diyle,
p, q, s valent zéro ou 1,
r vaut 1 ou 2.

9. Mélange de cristaux liquides smectique, chiral, selon l'une des revendications 1 à 6, renfermant 10 à 60 % d'un ou plusieurs composés de formule (I).

10. Mélange de cristaux liquides smectique, chiral, selon la revendication 6, **caractérisé en ce que** le mélange renferme 10 à 60 % de 1 à 15 composés de formule (I) et 40 à 90 % de 2 à 15 composés de formule (II).

11. Composés de formule générale (I) selon la revendication 1, pris parmi les composés de formule (XXIII) : 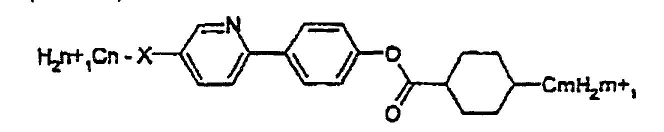 où : 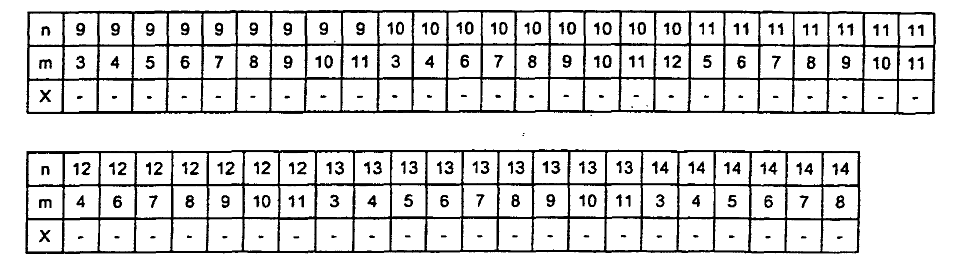 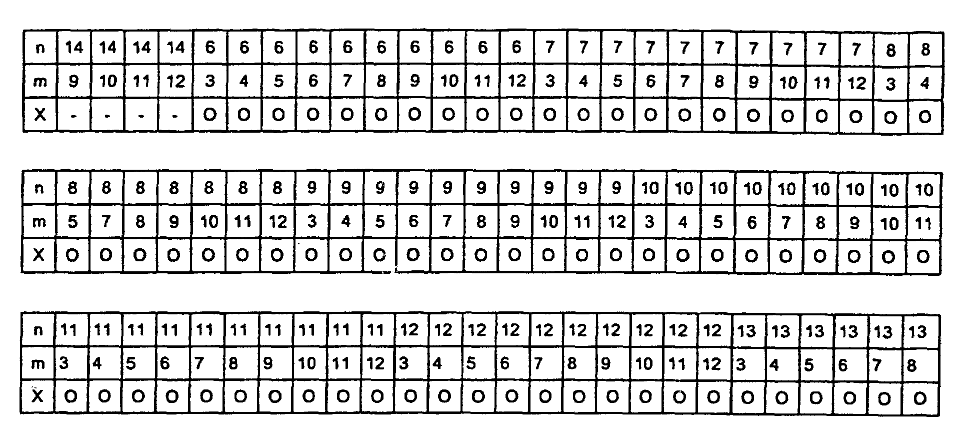 Composés de formule (XXIV) 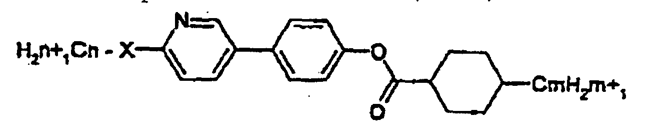 où:
n est un entier de 8 à 14,
m est un entier de 3 à 11,
X est une liaison simple
à l'exception de n = 11, m = 3 ou 5, X liaison simple,
Composés de formule (XXV) 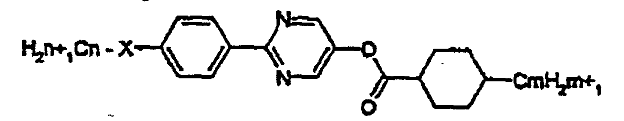 où :
n est un entier de 2 à 13,
m est un entier de 3 à 11,
X est une liaison simple
à l'exception de n = 2, m = 11, X = O ; n = 5, m = 5, X = O,
Composés de formule (XXVI) 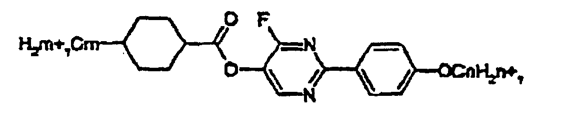 où :
n est un entier de 5 à 13,
m est un entier de 3 à 10,
à l'exception de n = 8, m = 5,
Composés de formule (XXVII) 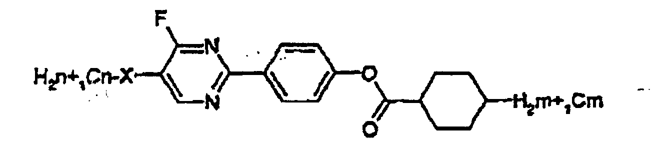 où : 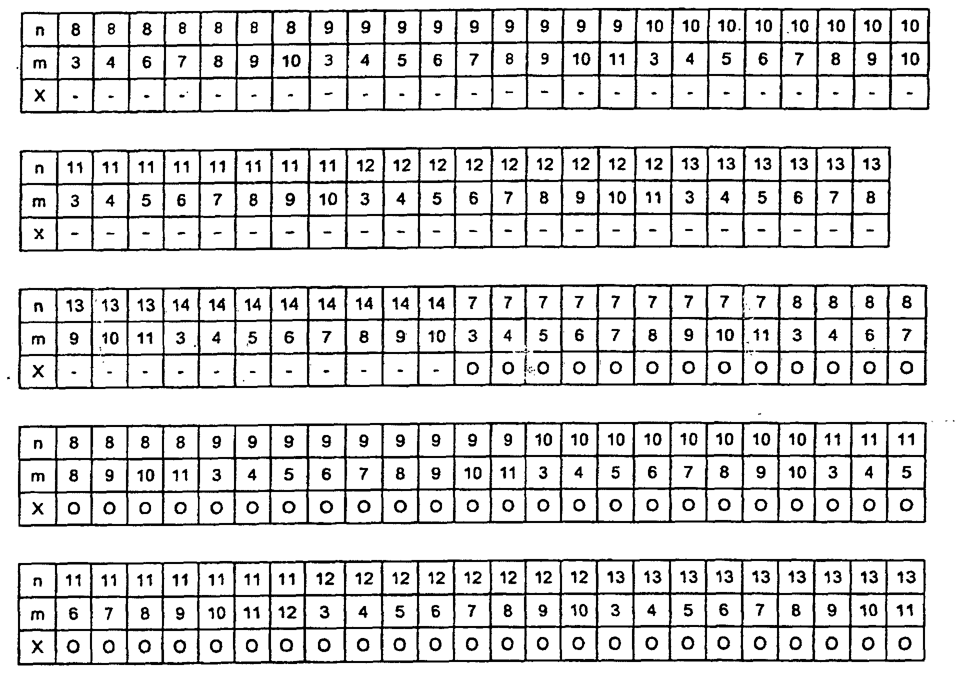 Composés de formule (XXIX) 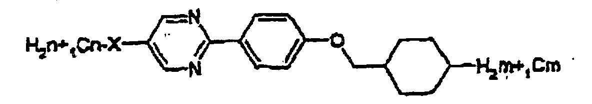 où : 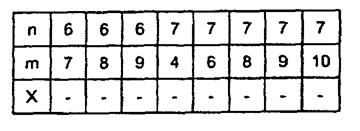 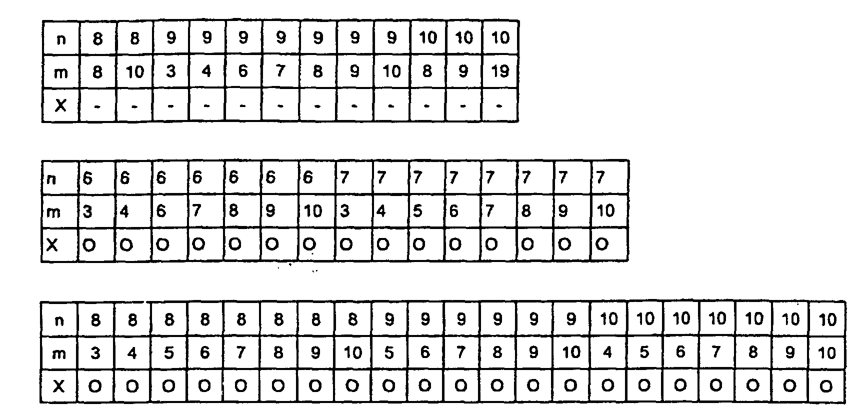 Composés de formule (XXX) 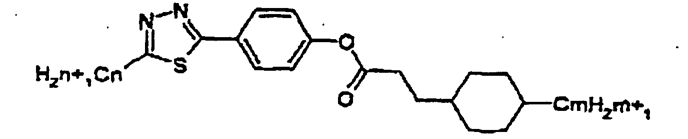 où :
n est un entier de 5 à 13,
m est un entier de 3 à 10,
à l'exception de n = 8, m = 4 ; n = 9, m = 3.

12. Composés de formule générale (II) selon la revendication 6, pris parmi les composés de formule 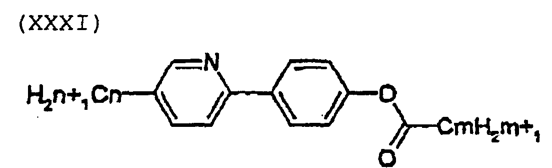 où : 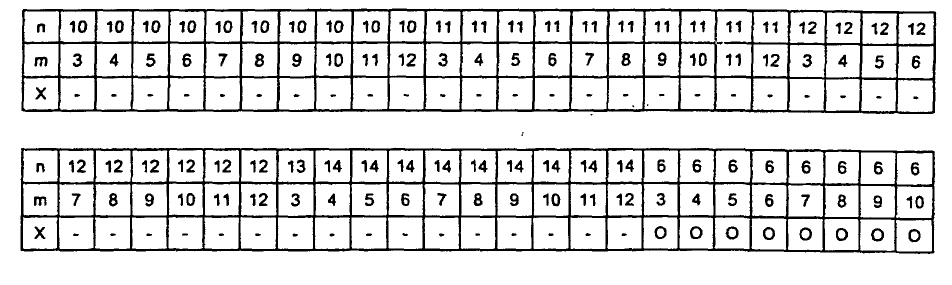 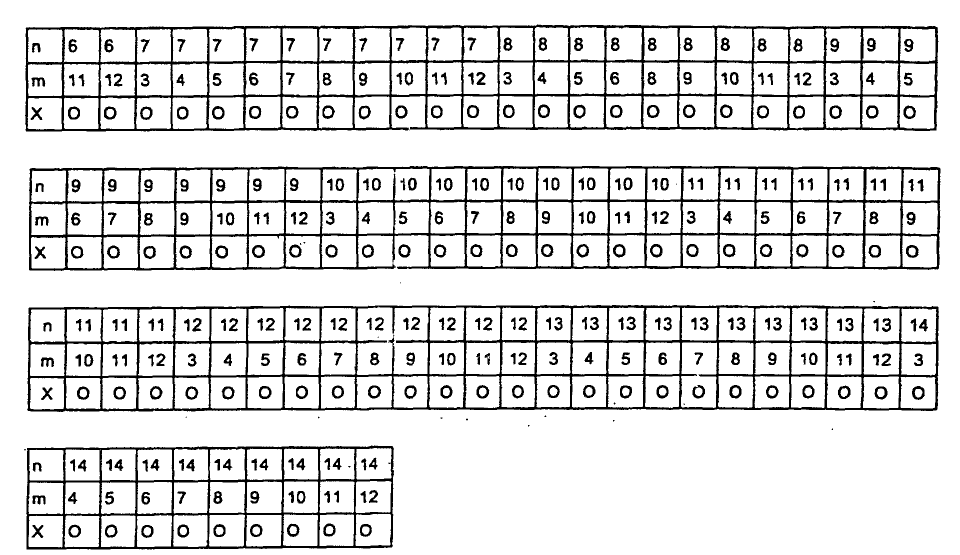 Composés de formule (XXVIII) 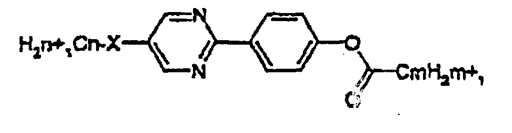 où : 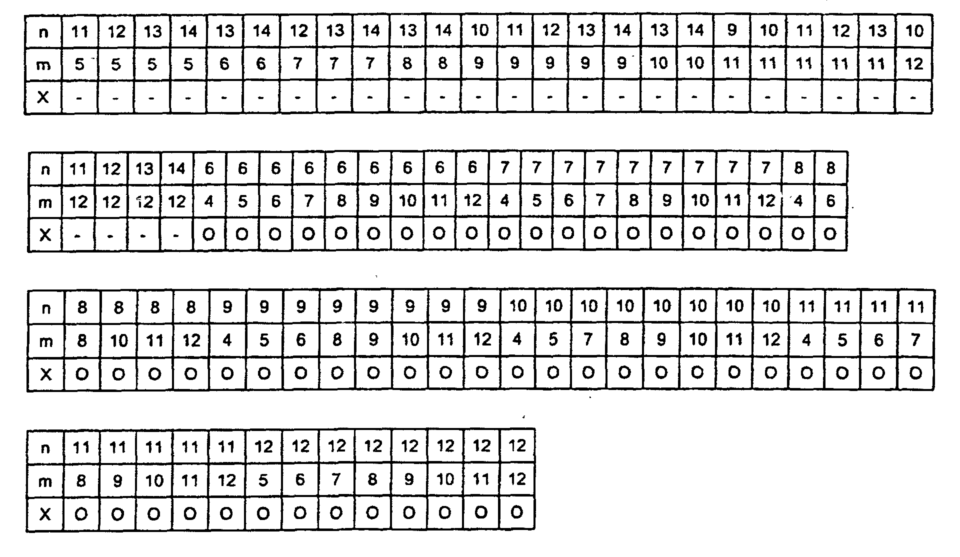 Composés de formule (XXXII) 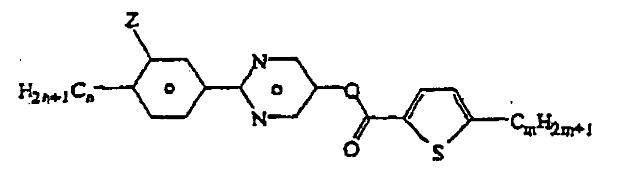 où : 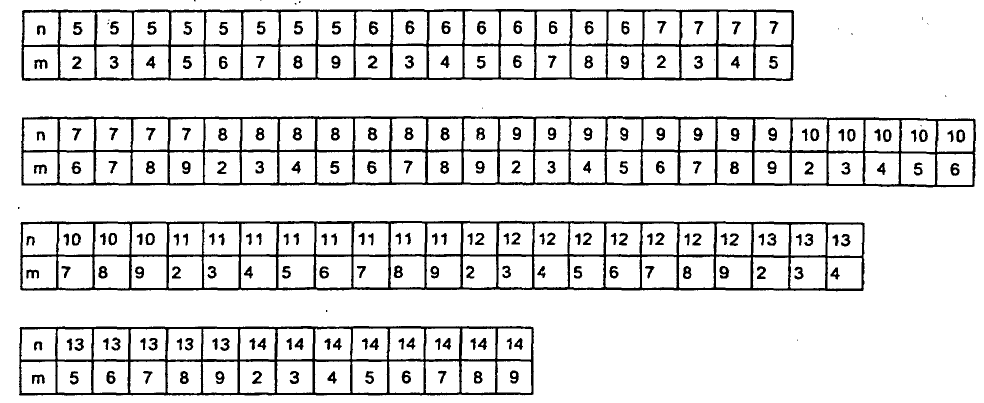 et Z représente dans tous les cas H ou F.
